# EUROPEAN PATENT APPLICATION

(11) **EP 4 624 584 A1**
(43) Date of publication of application: **01.10.2025**
(21) Application number: 23912207.0
(22) Date of filing: 26.12.2023
(51) Int. Cl.: C12N 15/864, C12N 5/10, C12N 7/01, C12N 15/34, C12N 15/35

(54) **HELPER GENE CONTROL**

(30) Priority: 27.12.2022 JP 2022210794
(71) Applicant: Synplogen Co., Ltd., Kobe-shi, Hyogo 650-0047 (JP)
(72) Inventor: NISHIMURA, Yuya, Kobe-shi, Hyogo 650-0047 (JP); SAITO, Shunsuke, Kobe-shi, Hyogo 650-0047 (JP)
(74) Representative: Engelhard, Markus
(86) International application number: PCT/JP2023/046813
(87) International publication number: WO 2024/143429

(57) **Abstract**

The present disclosure provides control of expression of a Rep gene by control of expression of a helper gene. In one aspect, the present disclosure provides a method for controlling the expression of a Rep gene, including a step of controlling the expression of a helper gene. In one embodiment, the helper gene includes a gene selected from adenovirus E1B, E2, E4 and VA. In one embodiment, the helper gene includes an adenovirus E1B gene. In one embodiment, the helper gene includes two or more helper genes related to the production of an adeno-associated virus (AAV) -derived construct, and optionally, the helper genes can be selected from E1A, E1B, E2, E4, and VA. In one embodiment, the helper gene includes a helper gene selected from E1A and E1B and a helper gene selected from E2, E4 and VA.

## Description

### Technical Field

The present disclosure provides control of expression of a Rep gene by control of expression of a helper gene. The present disclosure also provides a method for controlling the expression of a Rep gene, including steps of controlling the expression of a helper gene, means (such as compositions) for the same, and products thereof (such as virus-derived constructs).

### Background Art

With development of gene therapy drugs, various viral vectors such as an adeno-associated virus (AAV) vector are used (Patent Literature 1). For example, production of AAV vectors is dominated by a method in which producer cells are transfected with a plasmid containing an inverted terminal repeat (ITR) and a gene of interest (GOI), a packaging plasmid containing rep and cap derived from AAV, and a helper plasmid containing a helper gene derived from adenovirus (AdV) to allow transient expression of AAV vectors. However, with expansion of indication diseases in which AAV vectors are used, an amount of AAV vectors to be required is increasing, and accordingly, a demand for plasmid DNA required for each production batch when transient expression is used is also increasing. Although attempts have also been made to develop cell lines that can incorporate these genes used for transient expressions into chromosomes of producer cells and stably produce AAV vectors, toxicity of a Rep gene to host cells is often a problem. Therefore, it is beneficial to provide a method for controlling the expression of a Rep gene.

### Citation List

### Patent Literature

Patent Literature 1: WO 2001/090392 A

### Summary of Invention

### Solution to Problem

The inventors have found that the expression of a Rep gene can be efficiently controlled by controlling the expression of a helper gene. Based on this finding, the present disclosure provides a method for controlling the expression of a Rep gene, including steps of controlling the expression of a helper gene. Similarly, the present disclosure provides use of nucleic acids containing an expression control system for the helper gene in controlling the expression of a Rep gene. Further, the present disclosure also provides producer cells and virus-derived constructs produced therefrom.

Accordingly, the present disclosure provides:
(Item 1)
   A method for controlling expression of a Rep gene, including a step of controlling expression of a helper gene selected from E1B, E2, E4 and VA.
(Item 2)
   A method for controlling expression of a Rep gene, including a step of controlling expression of an E1B helper gene.
(Item 3)
   A method for controlling expression of a Rep gene, including a step of controlling expression of two or more helper genes related to production of an adeno-associated virus (AAV) -derived construct.
(Item 4)
   The method of any of the above items, wherein the helper gene includes a helper gene selected from E1A, E1B, E2, E4 and VA.
(Item 5)
   The method of any of the above items, wherein the helper gene includes a helper gene selected from E1A and E1B and a helper gene selected from E2, E4 and VA.
(Item 6)
   The method of any of the above items, wherein a decrease in expression of the helper gene results in a decrease in expression of the Rep gene.
(Item 7)
   The method of any of the above items, wherein the step of controlling expression of the helper gene uses an expression control system having at least one of the following functions of controlling activity of a promoter operably linked to the helper gene, degrading a nucleic acid transcribed from the helper gene, making the helper gene transcriptionally inactive, and degrading a protein encoded by the helper gene.
(Item 8)
   The method of any of the above items, wherein the expression control system is activated by addition or removal of a drug.
(Item 9)
   The method of any of the above items, wherein the expression control system includes a stimuli-responsive promoter operably linked to the helper gene, a Cre protein-responsive nucleic acid region that enables removal or inversion of at least a part of a nucleic acid region of the helper gene or a promoter operably linked thereto, a CRISPR-Cas9 system or an shRNA for a nucleic acid transcribed from the helper gene.
(Item 10)
   The method of any of the above items, further including a step of triggering an expression control system for the Rep other than the helper gene.
(Item 11)
   The method of any of the above items, wherein expression of the Rep gene is controlled in a cell containing the helper gene and the Rep gene.
(Item 12)
   The method of any of the above items, wherein expression of the Rep gene is controlled in a producer cell containing the helper gene and the Rep gene that produces an AAV-derived construct.
(Item 13)
   The method of any of the above items, wherein production of the AAV-derived construct is controlled by controlling expression of the helper gene.
(Item 14)
   The method of any of the above items, wherein the producer cell contains an inverted terminal repeat (ITR), a gene of interest (GOI), and a Cap gene.
(Item 15)
   The method of any of the above items, wherein the producer cell contains the helper gene, ITR, GOI, Cap gene and Rep gene in a chromosome.
(Item 16)
   The method of any of the above items, wherein the producer cell contains the helper gene, ITR, GOI, Cap gene, and Rep gene at one locus on a chromosome.
(Item 17)
   A combination for controlling expression of a Rep gene, including:
   a nucleic acid containing a Rep gene;
   a nucleic acid containing a helper gene selected from E1B, E2, E4 and VA; and
   a control system for the helper gene.
(Item 18) A combination for controlling expression of a Rep gene, including:
   a nucleic acid containing a Rep gene;
   a nucleic acid containing two or more helper genes required for production of an adeno-associated virus (AAV) -derived construct; and
   a control system for the helper gene.
(Item 19)
   The combination of any of the above items, wherein the helper gene includes a helper gene selected from E1A, E1B, E2, E4 and VA.
(Item 20)
   The combination of any of the above items, wherein the helper gene includes a helper gene selected from E1A and E1B and a helper gene selected from E2, E4 and VA.
(Item 21)
   The combination of any of the above items, wherein the nucleic acid containing the Rep gene and the nucleic acid containing the helper gene form separate molecules or separate double-stranded complexes.
(Item 22)
   The combination of any of the above items, wherein the nucleic acid containing the Rep gene and the nucleic acid containing the helper gene form the same molecule or the same double stranded complex.
(Item 23)
   The combination of any of the above items, wherein the control system includes a combination of a drug and the drug-responsive promoter operably linked to the helper gene, a combination of a Cre protein and the Cre protein-responsive nucleic acid region that enables removal of a poly A region linked to the helper gene, or an shRNA for a nucleic acid transcribed from the helper gene.
(Item 24)
   The combination of any of the above items, wherein the nucleic acid containing the Rep gene and/or the nucleic acid containing the helper gene is a plasmid.
(Item 25)
   The combination of any of the above items, wherein the nucleic acid containing the Rep gene and/or the nucleic acid containing the helper gene forms a complex with a cationic substance.
(Item 26)
   The combination of any of the above items, including a cell that contains a nucleic acid containing the Rep gene and a nucleic acid containing the helper gene.
(Item 27)
   The combination according to item 26, wherein the cell is a producer cells that produces an AAV-derived construct.
(Item 28)
   The combination of any of the above items, wherein the producer cell contains an inverted terminal repeat (ITR), a gene of interest (GOI), and a Cap gene.
(Item 29)
   The combination of any of the above items, wherein the producer cell contains the helper gene, ITR, GOI, Cap gene and Rep gene in a chromosome.
(Item 30)
   The combination of any of the above items, wherein the producer cell contains the helper gene, ITR, GOI, Cap gene, and Rep gene at one locus on a chromosome.
(Item 31)
   A method for producing an adeno-associated virus (AAV) -derived construct using the combination of any of the above items, the method including:
   a step of preparing a producer cell that contains a nucleic acid containing the Rep gene and a nucleic acid containing the helper gene; and
   a step of controlling expression of the Rep gene using a control system for the helper gene.
(Item 32) The method of any of the above items, wherein the producer cell contains the helper gene and the Rep gene in a chromosome.
(Item 33)
   An AAV-derived construct produced by the method of any of the above items.

In the present disclosure, it is intended that the one or more features described above can be provided in further combination in addition to the specified combination. Still further embodiments and advantages of the present disclosure will be appreciated by those skilled in the art upon reading and understanding the following detailed description as appropriate.

### Advantageous Effects of Invention

According to the present disclosure, it is possible to control expression of a Rep gene, thereby efficiently providing a virus-derived construct.

### Brief Description of Drawings

[FIG. 1] FIG. 1 shows an outline of establishment of tTA genome-introduced cells of a Tet-off system used in Examples.
[FIG. 2] FIG. 2 shows an outline of the structure of a plasmid used for incorporating an E1A/E1B gene into a genome, in which an expression control system of the Tet-off system in Example 1 is incorporated.
[FIG. 3] FIG. 3 shows an outline of an AAV vector production system in Example 1.
[FIG. 4] FIG. 4 shows an expression level of mRNA of E1 (E1A, E1B19K, E1B55K) under each condition. A relative expression level under each condition is shown based on the expression level in 293 cells of No. 1 using a wild-type gene.
[FIG. 5] FIG. 5 shows the expression level of mRNA of Rep under each condition. The relative expression level under each condition is shown based on the expression level in 293 cells of No. 1 using the wild-type gene.
[FIG. 6] FIG. 6 shows an AAV vector production amount under each condition. The genomic copy number per mL of cell culture supernatant is shown.
[FIG. 7] FIG. 7 shows an outline of a plasmid structure used for AAV vector production in Example 2.
[FIG. 8] FIG. 8 shows the expression levels of mRNA of E1 (E1A, E1B19K, E1B55K) and Rep and the AAV vector production amount under each condition in HeLa cells. Under each gene introduction condition, a corresponding relative value during addition of doxycycline is shown based on the value without doxycycline.
[FIG. 9] FIG. 9 shows the expression levels of mRNA of E1 (E1A, E1B19K, E1B55K) and Rep and the AAV vector production amount under each condition in CHO cells. Under each gene introduction condition, the corresponding relative value during addition of doxycycline is shown based on the value without doxycycline.
[FIG. 10] FIG. 10 shows an outline of an all-in-one structure plasmid containing an E1A/E1B gene, in which the expression control system of the Tet-off system used in Example 3 is incorporated.
[FIG. 11] FIG. 11 shows the expression levels of mRNA of E1 (E1A, E1B19K, E1B55K) and Rep and the AAV vector production amount under each condition. The expression level of mRNA is shown as a relative expression level under each condition based on the expression level in HeLa cells of No. 1 using the wild-type gene. The AAV vector production amount indicates the genome copy number per mL of cell culture supernatant.
[FIG. 12] FIG. 12 shows an outline a plasmid structure used for AAV vector production in Example 4.
[FIG. 13] FIG. 13 shows the expression levels of mRNA of E1 (E1A, E1B19K, E1B55K) and Rep under each condition in HeLa cells. The relative expression level under each condition is shown based on the expression level in HeLa cells of No. 1 using the wild-type gene.
[FIG. 14] FIG. 14 shows the expression levels of mRNA of E1 (E1A, E1B19K, E1B55K) and Rep under each condition in CHO cells. The relative expression level under each condition is shown based on the expression level in CHO cells of No. 1 using the wild-type gene.
[FIG. 15] FIG. 15 shows the AAV vector production amount under each condition in HeLa cells and CHO cells. The genomic copy number per mL of cell culture supernatant is shown.
[FIG. 16] FIG. 16 shows an outline of an exemplary plasmid structure in which an E1A/E1B expression control system by a drug control switch (Cumate) is incorporated.
[FIG. 17] FIG. 17 shows an outline of an exemplary plasmid structure in which an E1A/E1B expression control system by a drug control switch (mifepristone) is incorporated.
[FIG. 18] FIG. 18 shows an outline of an exemplary plasmid structure in which an E1A/E1B expression control system by a Cre protein control switch is incorporated.
[FIG. 19] FIG. 19 shows an outline of an exemplary plasmid structure in which the E1A/E1B expression control system by the Cre protein control switch is incorporated.
[FIG. 20] FIG. 20 shows an outline of an exemplary plasmid structure in which an E1A/E1B expression control system and expression control systems of other helper genes are incorporated.
[FIG. 21] FIG. 21 shows an outline of an exemplary plasmid structure in which an E1A/E1B expression control system utilizing RNAi through expression of shRNA by the Tet-on system and an expression control system for a Rep gene are incorporated.
[FIG. 22] FIG. 22 shows the expression levels of mRNA of E1 (E1A, E1B19K, E1B55K,) E4orf6 and Rep and the AAV vector production amount under each condition in HEK293 cells. The expression level of mRNA indicates the relative expression level under each condition based on the expression level in HEK293 cells of No. 1 using the wild-type gene. The AAV vector production amount indicates the genome copy number per mL of cell culture supernatant.

### Description of Embodiments

Hereinafter, the present disclosure will be described while showing the best mode. It is should be understood that throughout the present description, the singular forms of expression also include the concept of the plural forms thereof, unless otherwise stated. Thus, it is should be understood that the singular article (for example, in English, "a", "an", "the", and the like) also includes the concept of the plural thereof, unless otherwise stated. Further, it is should be understood that the terms used herein are used in the sense commonly used in the art, unless otherwise stated. Thus, unless otherwise defined, all technical and scientific terms used herein have the same meaning as commonly understood by one of ordinary skill in the art to which the present disclosure belongs. In the case of conflict, the present description (including definitions) will control.

Hereinafter, definitions and/or basic technical contents of terms particularly used herein will be described as appropriate.

As used herein, "polynucleotide" and "nucleic acid" are used interchangeably, and refer to a polymer of nucleotides of any length. As used herein, unless otherwise specified, a "nucleic acid" means both a single molecule nucleic acid and multiple molecules of nucleic acid, and both a single-stranded nucleic acid and a double-stranded nucleic acid. As used herein, unless otherwise specified, a statement that a nucleic acid contains a plurality of elements includes embodiments in which the plurality of elements are present on the same nucleic acid molecule and embodiments in which the plurality of elements are each present on different nucleic acid molecules, and includes embodiments in which the plurality of elements are present in the same double-stranded nucleic acid complex and embodiments in which the plurality of elements are each present in different double-stranded nucleic acid complexes. Examples of nucleic acids include DNA, RNA, cDNA, mRNA, rRNA, tRNA, microRNA (miRNA), and lncRNA. The term also includes "polynucleotide derivative". A "polynucleotide derivative" refers to a polynucleotide that contains a nucleotide derivative or have an unusual internucleotide linkage. A "nucleotide derivative" refers to a nucleotide having a structure different from a normal nucleotide used in natural DNA or RNA, and examples thereof include a locked nucleic acid (LNA), an ethylene nucleic acid such as a 2'-O,4'-C-ethylene bridged nucleic acid (ENA), other bridged nucleic acids (BNA), a hexitol nucleic acid (HNA), an amido-bridged nucleic acid (AmNA), a morpholino nucleic acid, tricyclo-DNA (tcDNA), a polyether nucleic acid (see, e.g., US 5,908,845), and a cyclohexene nucleic acid (CeNA). Examples of unusual internucleotide linkages include an interoligonucleotide linkage in which a phosphodiester bond is converted to a phosphorothioate bond, an interoligonucleotide linkage in which a phosphatediester bond is converted to an N3'-P5' phosphoramidate bond, and an interoligonucleotide linkage in which ribose and a phosphatediester bond are converted to a peptide nucleic acid bond.

Unless otherwise indicated, it is contemplated that a particular nucleic acid sequence also encompasses conservatively modified variants thereof (for example, degenerate codon substituents) and complementary sequences, as well as the sequence explicitly indicated. Specifically, degenerate codon substituents can be achieved by preparing sequences in which the third position of one or more selected (or all) codons is substituted with mixed-bases and/or deoxyinosine residues. For example, variants based on specific wild-type sequences, such as adenovirus serotypes 1 to 52 and adeno-associated virus serotypes 1 to 12, include, besides known variants (for example, rh10, DJ, DJ/8, PHP.eB, PHP.S, AAV2-retro, AAV2-QuadYF, AAV2.7m8, AAV6.2, rh.74, AAV2.5, AAV-TT, Anc80, etc.), also nucleic acids containing a sequence that has at least 70%, at least 80%, at least 85%, at least 90%, at least 95%, at least 96%, at least 97%, at least 98%, at least 99%, or at least 99.5% sequence identity to the parent sequence.

As used herein, a "gene" refers to a nucleic acid part that performs certain biological functions. The biological functions include encoding a polypeptides or a protein, encoding a protein-non-coding functional RNA (rRNA, tRNA, microRNA (miRNA), lncRNA, etc.), controlling the production of a polypeptide, a protein or a protein-non-coding functional RNA, specifically binding to a specific protein, controlling cleavage or replication of a nucleic acid. Thus, in addition to a nucleic acid part encoding a protein or a protein-non-coding functional RNA, the gene used herein includes a transcriptional and translational regulatory sequence such as a promoter, a terminator, an enhancer, an insulator, a silencer, an origin of replication, and an internal ribosome entry site, and a nucleic acid part required for packaging into a viral particle. As used herein, a "gene product" refers to mRNA transcribed from a gene, a polypeptide, a protein, or a protein-non-coding functional RNA encoded by a gene. As used herein, "expression of a gene" refers to the production of a gene product. In particular, viral genes often encode multiple types of proteins of different lengths in a single nucleic acid region. As used herein, a gene is referred to as expressed in a case where at least one type of protein that can be produced from the gene is produced.

As used herein, "Rep" refers to a Rep gene possessed by adeno-associated virus (AAV), a part thereof, or a modified form thereof. The wild-type AAV Rep encodes a regulatory protein (Rep78, Rep68, Rep52, Rep40) that controls replication and transcription, and the Rep of the present disclosure can encode a protein having a function equivalent to at least one of Rep78, Rep68, Rep52, and Rep40. For AAV, serotypes of type 1 to 12 based on a capsid have been reported, and other serotypes of rh10, DJ, DJ/8, PHP.eB, PHP.S, AAV2-retro, AAV2-QuadYF, AAV2.7m8, AAV6.2, rh.74, AAV2.5, AAV-TT, and Anc80 have also been reported. The Rep of the present disclosure can be derived from Rep of any serotype of AAV. The Rep of the present disclosure or a gene product thereof (such as a protein) can have a sequence (base sequence or amino acid sequence) with high identity (e.g., at least 75%, at least 80%, at least 85%, at least 90%, at least 91%, at least 92%, at least 93%, at least 94%, at least 95%, at least 96%, at least 97%, at least 98%, or at least 99% identity, etc.) to Rep of any serotype of AAV or a gene product thereof (such as a protein).

As used herein, a "helper gene" refers to any gene that increases an AAV production amount in producer cells. For example, pAAV-ZsGreen (TAKARA: 6231) and pRC1 (TAKARA: 6672) were introduced into HEK293 cells (ATCC: CRL-1573), and cells into which candidate genes were transferred and cells into which candidate genes were not transferred were further produced. These cells were cultured in a DMEM (Thermo fisher: 11965) (2% FBS (Termo fisher: 10270), 1% Penicillin-Streptomycin mixed solution (Nacalai Tesque: 09367-34)) medium at 37°C in a 5%CO₂ incubator. After 3 days, 5 µL of Nuclease-Free water (Promega: P1193), 1 µL of 10×React Buffer (MgCl₂ added) (Thermo fisher: ED0521), and 2 µL of DNaseI (Thermo fisher: ED0521) were added to 2 µL of the collected supernatant, and the mixture was reacted in a block incubator at 37°C for 30 minutes, and then, 90 µL of Nuclease-Free water (0.001% Pluronic (registered trademark) F-68 (100X) (Thermo fisher: 24040032), 5mM EDTA (TAKARA: T9191)) was added, the mixture was reacted in a block incubator at 95°C for 10 minutes, and the resulting solution was diluted 100 times with Nuclease-Free water (0.001% Pluronic (registered trademark) F-68 (100 X)) to prepare a solution of 2 µL, then the prepared solution was mixed with 2 µL of Nuclease-Free water, 5 µL of PowerUp (registered trademark) SYBR (registered trademark) Green Master Mix (Thermo fisher: A25780), 0.5 µL of 10 µM CMV-F primer (CATCAATGGGCGTGGATAGC: SEQ ID NO: 7), and 0.5 µL of 10 µM CMV-R primer (GGAGTTGTTACGACATTTTGGAAA: SEQ ID NO: 6), and qPCR was performed using a QuantStudio 3 real-time PCR system Fast96 well (Thermo fisher: QS3-96F) under conditions of 95°C for 10 minutes, followed by 40 cycles of 95°C for 15 seconds, 55°C for 5 seconds, and 72°C for 30 seconds. When an AAV particle amount was measured, if the AAV particle amount was increased by at least 50% in the cells into which the candidate gene has been transferred as compared with the cells into which the candidate gene has not been transferred, the candidate gene can be determined to be a helper gene. Typically, the helper gene is selected from adenovirus E1A, E1B, E2A, VA, E4, parts thereof, or modified forms thereof. To date, 52 human adenovirus antigenic types (serotypes) have been identified. The helper gene of the present disclosure can be derived from helper genes of any antigenic type of adenovirus. The helper gene or a gene product thereof (such as a protein) of the present disclosure can have a sequence (base sequence or amino acid sequence) with high identity (e.g., at least 75%, at least 80%, at least 85%, at least 90%, at least 91%, at least 92%, at least 93%, at least 94%, at least 95%, at least 96%, at least 97%, at least 98%, or at least 99% identity, etc.) to the helper gene or a gene product thereof (such as a protein) of any serotype of adenovirus.

As used herein, "control of expression of a Rep gene" refers to any manipulation that increases or decreases the amount of a Rep gene product (for example, a Rep gene transcript). For example, in the same cell, the same culture medium or the same isolation space (such as a well or a dish), expression of the Rep gene is controlled in a case where the amount of Rep gene product or a ratio of the amount of Rep gene product to Rep gene varies by at least 30% at different time points (for example, 6 hours, 12 hours, 24 hours, or 72 hours apart).

As used herein, "control of expression of a helper gene" refers to any manipulation that increases or decreases the amount of a helper gene product (for example, a gene transcript). For example, in the same cell, the same culture medium or the same isolation space (such as a well or a dish), expression of the helper gene is controlled in a case where the amount of helper gene product or the ratio of the amount of helper gene product to helper gene varies by at least 30% at different time points (for example, 6 hours, 12 hours, 24 hours, or 72 hours apart). The step of controlling expression of the helper gene can include, for example, providing a stimulus (a drug, light, etc.) corresponding to a stimulus-responsive promoter operably linked to the helper gene.

As used herein, an "expression control system" for a gene refers to a substance or a part of a substance, or a combination thereof, capable of causing transcription and/or translation of the gene to start or stop, or degradation of a nucleic acid containing the gene or a gene product thereof under specific conditions. Examples of the expression control system include a combination of a stimulus (a drug, light, etc.) and a promoter responsive to the stimulus (control of transcription of a gene), a combination of a Cre protein and a Cre protein-responsive nucleic acid region (LoxP) (control of transcription of a gene), a CRISPR-Cas9 system (degradation of a nucleic acid containing a gene), siRNA or shRNA (degradation of a gene product), and a ubiquitin ligase (degradation of a gene product). In a case where the expression control system includes nucleic acid substances such as a promoter and a nucleic acid region having a specific function, this can be referred to as nucleic acid elements of the expression control system. In a case where a cell, culture or isolation space contains nucleic acid elements of an expression control system for a helper gene, expression of the helper gene is considered to be controlled. Typically, the expression control system can be activated and/or stopped by adding a substance that is not naturally present in a target cells to the target cell.

As used herein, two genes being in cis refers to that the genes are present on the same nucleic acid molecule or a nucleic acid molecule of a complementary strand (in the case of a double-stranded nucleic acid). Further, As used herein, two genes being in trans refers to that in a cell (in an organism), the genes are not present on the same nucleic acid molecule or a nucleic acid molecule of a complementary strand (in the case of a double-stranded nucleic acid). For example, the gene present on the genome and the gene on the nucleic acid introduced with a virus-derived construct can be in trans. Optionally, whether two genes are in trans can be determined in a state at a point in time when the two genes become simultaneously present in the cell (for example, when the nucleic acid is introduced into the cell).

When referring to the number of genes herein, one gene refers to a gene that has a contiguous sequence in a form that is normally (most frequent or 50% or more probability) present on the genome of an organism. For example, two exons encoding a protein can be two genes. For example, in a case where a promoter sequence and a sequence encoding a protein form a continuous sequence, a nucleic acid part including the promoter sequence and the sequence encoding the protein can be one gene. For example, in a case where a protein that becomes functional upon cleavage is encoded by a contiguous sequence on the genome, the protein can be encoded by one gene. When referring to a gene in terms of function, the nucleic acid sequence does not need to be a continuous sequence, for example, multiple exons encoding a protein are collectively referred to as the gene for that protein.

As used herein, "deficient" of a gene refers to that the nucleic acid does not contain the gene or contains a gene that has been modified so as not to perform the normal function of the gene (for example, the function of producing a functional protein).

As used herein, "operably linked" refers to that expression (actuation) of a desired sequence is located under the control of a transcriptional and translational regulatory sequence (for example, a promoter, an enhancer, or the like) or a translational regulatory sequence. In order for a promoter to be operably linked to a gene, the promoter is usually located immediately upstream of the gene, but not necessarily adjacent.

As used herein, "plasmid" refers to circular DNA that is present separately from the chromosome in a cell, or that is present separately from the chromosome when introduced into a cell.

As used herein, a "cationic substance" refers to a substance that is charged with a positive charge when added to neutral water, and is typically provided in a complex with a nucleic acid (such as a plasmid).

herein, a "virus-derived construct" refers to a nucleic acid construct that at least partially has a structure derived from a virus or a construct containing a protein produced therefrom. Virus-derived components include viral vectors, virus-like particles (VLPs), oncolytic viruses (including those modified to proliferate only in cancer cells), and viral replicons (self-replicating viral genomes that has been deleted the ability to produce viral particles).

As used herein, a "viral vector" refers to a construct that at least partially has a structure derived from a virus and is capable of introducing a nucleic acid into a target cell. Typically, a viral vector is in the form of a viral particle that contains viral structural proteins and a nucleic acid containing a heterologous gene.

As used herein, a "producer cell" means a cell capable of producing a desired virus-derived component, and can be a cell in which a gene required for producing a virus-derived component is expressed from a chromosome and an introduced plasmid. The desired virus-derived component can be produced by introducing a plasmid into a producer cell. For example, in the case of an AAV viral vector, E1A and E1B are required for the production, but HEK293 has these, allowing them to be removed from a helper plasmid. Other cells also function as producer cells when modified to have such functional cofactors.

herein, an "animal cell" refers to a cell obtained from an animal (human, mouse, rat, monkey, dog, etc.) or a cell obtained by modifying a cell obtained from an animal.

As used herein, a "host cell" refers to a cell (including the progeny of such a cell) into which an exogenous nucleic acid or protein or viral or virus-derived construct has been introduced.

herein, "protein", "polypeptide" and "peptide" are used interchangeably, and refer to a polymer of an amino acid of any length. The polymer may be linear, branched, or cyclic. The amino acid may be natural or non-natural and may be a modified amino acid. The term also encompasses natural or artificially modified polymers. Such modifications include, for example, disulfide bond formation, glycosylation, lipidation, acetylation, phosphorylation, or any other manipulation or modification (for example, conjugation with a labeling component).

As used herein, a "transcriptional and translational regulatory sequence" collectively refers to a promoter sequence, polyadenylation signals, a transcription termination sequence, an upstream regulatory domain, an origin of replication, an enhancer, IRES, and the like, which act together to enable replication, transcription and translation of a coding sequence in a recipient cell. Not all of these transcriptional and translational regulatory sequences need to be present, so long as the replication, transcription and translation of the selected coding sequence is possible in a suitable host cell. A person skilled in the art can easily identify a regulatory nucleic acid sequence from public information. Furthermore, one skilled in the art can identify a transcriptional and translational regulatory sequence that can be applied for use purposes, e.g., in vivo, ex vivo, or in vitro.

As used herein, a "promoter" refers to a segment of a nucleic acid sequence that controls transcription of an operably linked nucleic acid sequence. A promoter contains specific sequences that are sufficient for recognition, binding, and transcription initiation by RNA polymerase. A promoter may contain sequences that regulate RNA polymerase recognition, binding, or transcription initiation.

As used herein, an "enhancer" refers to a segment of a nucleic acid sequence that has the function of increasing expression efficiency of a gene of interest.

As used herein, a "silencer" refers to a segment of a nucleic acid sequence that has the function of reducing the expression efficiency of a gene of interest, contrary to an enhancer.

As used herein, an "insulator" refers to a segment of a nucleic acid sequence that has the function of cis-regulatory that regulates the expression of a gene located at a distant location on the sequence of DNA.

As used herein, a "terminator" refers to a segment of a nucleic acid sequence that is located downstream of a protein-coding region and is involved in transcription termination when a nucleic acid is transcribed into mRNA.

As used herein, an "origin of replication" refers to a segment of a nucleic acid sequence to which a protein (for example, initiator DnaA protein, etc.) that recognizes the nucleic acid sequence binds or to which RNA is synthesized to partially unwind a DNA double helix, thereby initiating replication.

As used herein, an internal ribosome entry site ("IRES") refers to a nucleic acid segment that promotes entry or retention of ribosomes during the translation of a nucleic acid sequence downstream thereof.

herein, "homology" of nucleic acids refers to a degree of identity of two or more nucleic acid sequences to each other, and generally, having "homology" refers to a high degree of identity or similarity. Therefore, the higher the homology between two nucleic acids, the higher the identity or similarity of their sequences. "Similarity" is a numerical value calculated for a similar base in addition to identity, and here, the similar base refers to a case where a part of bases in a mixed base (for example, R=A+G, M=A+C, W=A+T, S=C+G, Y=C+T, K=G+T, H=A+T+C, B=G+T+C, D=G+A+T, V=A+C+G, N=A+C+G+T) is identical. Whether two types of nucleic acids have homology can be examined by direct comparison of sequences or by hybridization methods under stringent conditions. When two nucleic acid sequences are directly compared, the genes have homology in a case where there is typically at least 50% identity, preferably at least 70% identity, more preferably at least 80%, 90%, 95%, 96%, 97%, 98% or 99% identity, between the nucleic acid sequences.

Amino acids can be herein referred to either by the commonly known threeletter symbol thereof or by the one-letter symbol recommended by the IUPAC-IUB Biochemical Nomenclature Commission. Nucleotides can likewise be referred to by the commonly recognized one-letter codes. As used herein, comparisons of the similarity, identity, and homology of amino acid sequence and base sequence are calculated using a tool for sequence analysis, BLAST, with default parameters. A search for identity can be performed, for example, using NCBI's BLAST 2.10.1+ (released on June 18, 2020). The value of identity as used herein usually refers to a value obtained when aligned under default conditions using the above BLAST. However, in a case where a higher value is obtained by changing parameters, the highest value is set as the value of identity. **In** a case where the identity is evaluated in a plurality of regions, the highest value among the plurality of regions is set as the value of identity. Similarity is a numerical value calculated for similar amino acids in addition to identity.

As used herein, unless otherwise specified, it is understood that reference to a biological material (for example, a protein, a nucleic acids, a gene) is also reference to a variant of the biological material (for example, a variant having modifications in the base sequence) that performs a function similar to (but not necessarily to the same extent as) the biological function of the biological material. Such variants can include fragments of the original molecule, molecules that are at least 30%, 40%, 50%, 60%, 70%, 80%, 90%, 95%, 98%, or 99% identical over the amino acid sequence or nucleic acid sequence of the original biological material of the same size, or when compared to the sequence of the original molecule that is aligned by computer homology programs known in the art. Variants can include molecules with modified nucleotides (for example, modification by methylation).

As used herein, a "corresponding" amino acid or nucleic acid refers to an amino acid or nucleotide that has or is predicted to have an action similar to that of a predetermined amino acid or nucleotide in a polypeptide or polynucleotide that serves as a reference for comparison in a certain polypeptide or polynucleotide molecule, and particularly in an enzyme molecule, refers to an amino acid that is located at a similar position in an active site and contributes similarly to catalytic activity. For example, in the case of an antisense molecule, it can be a similar part in an ortholog corresponding to a specific part of the antisense molecule. The corresponding amino acid can be, for example, a specific amino acid to which cysteinylation, glutathionylation, S-S bond formation, oxidation (for example, methionine side chain oxidation), formylation, acetylation, phosphorylation, glycosylation, myristylation, or the like is applied. Alternatively, the corresponding amino acid can be an amino acid responsible for dimerization. Such a "corresponding" amino acid or nucleic acid may be a region or domain that spans a certain range. Accordingly, in such a case, it is herein referred to as a "corresponding" region or domain .

As used herein, a "corresponding" gene (for example, a polynucleotide sequence or molecule) refers to a gene (for example, a polynucleotide sequence or molecule) that has or is predicted to have an action similar to that of a predetermined gene in a species that serves as a reference for comparison in a certain species, and refers to a gene that has the same evolutionary origin in a case where there are a plurality of genes having such an action. Therefore, a gene corresponding to a certain gene can be an ortholog of the gene. For example, the cap of AAV of serotype 1 can correspond to the cap of AAV of serotype 2. For example, a corresponding gene in a certain virus can be found by searching a sequence database of the virus using a gene sequence of the virus serving as a reference of the corresponding gene as a query sequence.

According to the present disclosure, the term "activity" herein refers to the function of a molecule in its broadest sense . Activity generally includes, but is not intended to be limited to, a biological, biochemical, physical or chemical function of a molecule. Activity includes, for example, enzymatic activity, the ability to interact with other molecules, and the ability to activate, promote, stabilize, inhibit, suppress, or destabilize the function of other molecules, stability, the ability to localize to a specific subcellular location. When applicable, the term also relates to the function of a protein complex in its broadest sense.

As used herein, when referring to a certain gene or a nucleic acid molecule or polypeptide related thereto, the "biological function" refers to a specific function that the gene, nucleic acid molecule or polypeptide can have within a living organism, and examples of the biological function include, but are not limited to, specific cell surface structure recognition ability, enzyme activity, and binding ability to a specific protein. In the present disclosure, examples of the biological function include, but are not limited to, a function in which a certain promoter is recognized in a specific host cell. As used herein, the biological function can be exerted by "biological activity". As used herein, the "biological activity" refers to activity that a certain factor (for example, a polynucleotide, a protein, and the like) can have within a living organism, and includes activity that exerts various functions (for example, transactivation activity), and also includes, for example, activity that activates or inactivates another molecule by interaction with a certain molecule. For example, in a case where a factor is an enzyme, its biological activity includes that enzyme activity. **In** another example, in a case where a factor is a ligand, binding of the ligand to a corresponding receptor is included. Such biological activity can be measured by techniques well known in the art.

As used in the present disclosure, "infectivity" of a virus or a virus-derived construct refers to the ability to introduce a nucleic acid within the virus or the virus-derived construct into a cell by adhesion or membrane fusion of the virus or the virus-derived construct to the cell. "Replication capacity" of a virus or a virus-derived construct refers to the ability to produce infectious virus particles or virus-derived construct particles within an infected cell.

As used As used herein, the terms "transformation", "transduction" and "transfection" are used interchangeably, unless otherwise stated, and refer to the introduction of a nucleic acid into a host cell (optionally via a virus or a virus-derived construct). As a transformation method, any method can be used as long as it is a method of introducing a nucleic acid into a host cell, and examples thereof include various well-known techniques such as use of competent cells, an electroporation method, a method using a particle gun (gene gun), and a calcium phosphate method.

As used herein, a "purified" substance or biological factor (for example, a nucleic acids or a proteins, etc.) refers to a substance from which at least a part of the factor naturally associated with the biological factor has been removed. Therefore, usually, the purity of the biological factor in the purified biological factor is higher (i.e., more concentrated) than the state in which the biological factor is normally present. The term "purified" as used herein means that preferably at least 75 wt%, more preferably at least 85 wt%, more preferably at least 95 wt%, and most preferably at least 98 wt% of the same type of biological agent is present. The substances used in the present disclosure are preferably "purified" substances.

As used herein, a "pharmaceutical component" means any component that can constitute a medicine, and examples thereof include an active component (which itself exhibits a medicinal effect) and an additional component (which itself is not expected to exhibit a medicinal effect, but are expected to play a certain role (for example, excipients, lubricants, surfactants, and the like) when contained as a medicine). The pharmaceutical component may be a single substance or a combination of a plurality of substances and agents. Any combination can also be included, such as a combination of an active component and an additional component, and a combination of an adjuvant and an active component.

As used herein, the "active component" refers to a component that exerts an intended medicinal effect, and can correspond to one or more components.

As used herein, the "additional component" refers to any component that is not expected to have a medicinal effect but plays a certain role when contained as a medicine, and examples thereof include a pharmaceutically acceptable carrier, a stabilizer, an (auxiliary) auxiliary, a solubility improving agent, a solubilizing agent, a diluent, an excipient, a buffer, a binder, a diluent, a flavoring agent, and a lubricant.

As used herein, a "medical agent", "agent", or "factor" (all of which correspond to agent in English) is used interchangeably in a broad sense, and may be any substance or other element (for example, energy such as light, radioactivity, heat, and electricity) as long as it can achieve the intended purpose. Examples of such substances include, but are not limited to, proteins, polypeptides, oligopeptides, peptides, polynucleotides, oligonucleotides, nucleotides, nucleic acids (including, for example, cDNA, DNA such as genomic DNA, and RNA such as mRNA), polysaccharides, oligosaccharides, lipids, organic low molecules (for example, hormones, ligands, signaling substances, organic low molecules, molecules synthesized by combinatorial chemistry, low molecules that can be utilized as pharmaceuticals (for example, low molecule ligands or the like), and the like), complex molecules thereof, and mixtures thereof.

As used As used herein, a "complex" or "complex molecule" means any construct including two or more parts. For example, in a case where one part is a polypeptide, the other part may be a polypeptide or a substance other than the polypeptide (for example, a substrate, a sugar, a lipid, a nucleic acid, other hydrocarbons, and the like). As used herein, two or more parts constituting a complex may be bonded by a covalent bond or may be bonded by other bonds (for example, a hydrogen bond, an ionic bond, hydrophobic interaction, a van der Waals force, etc.).

As used herein, a "label" refers to an entity for distinguishing a target molecule or substance from others (for example, substances, energy, electromagnetic waves, and the like). Examples of such a labeling method include a radioisotope (RI) method, a fluorescence method, a biotin method, and a chemiluminescence method. In a case where a plurality of target proteins or factors or means for capturing the target proteins are labeled by the fluorescence method, labeling is performed with fluorescent substances having mutually different fluorescence emission maximum wavelengths. The difference in maximum fluorescence emission wavelength is preferably 10 nm or more. Any label that does not affect the function can be used, but an example of a fluorescent substance is Alexa^{™}Fluor. Alexa^{™}Fluor is a water-soluble fluorescent dye obtained by modifying coumarin, rhodamine, fluorescein, cyanine, and the like, and is a series corresponding to a wide range of fluorescence wavelengths, and is very stable, bright, and has low pH sensitivity as compared with other fluorescent dyes with corresponding wavelengths. Examples of a combination of fluorescent dyes with a maximum fluorescence wavelength of 10 nm or more include a combination of Alexa^{™}555 and Alexa^{™}633, and a combination of Alexa^{™}488 and Alexa^{™}555. Examples of other fluorescent labels include a cyanine dye (for example, Cy3, Cy5 of CyDye^{™} series, and the like), a rhodamine 6G reagent, N-acetoxy-N2-acetylaminofluorene (AAF), and AAIF (iodine derivative of AAF). In the present disclosure, such a label can be utilized to modify a target of interest so that it can be detected by detection means used. Such modifications are known in the art, and those skilled in the art can carry out such methods as appropriate for the label and depending on the object of interest.

As used herein, a "kit" refers to a unit in which a part to be provided (for example, a virus-derived construct, instructions, etc.) is provided, usually divided into two or more compartments. The form of this kit is preferred when it is intended to provide a composition that should not be provided in admixture for reasons of stability or the like, but is preferably used in admixture immediately before use. It is advantageous that such a kit preferably includes instructions or instructions describing how to use the provided parts or how to treat the reagents. In a case where the kit is used herein as a reagent kit, the kit usually includes instructions or the like describing how to use a plasmid or the like.

As used herein, "instructions" describe to a physician or other user a method of using the present disclosure. In the instructions, words instructing administration of the medicine or the like of the present disclosure are described. Further, the instructions may include wording instructing the form of administration. It is specified that the instructions are prepared in accordance with a format prescribed by the supervisory authority of the country in which the present disclosure is implemented (for example, the Ministry of Health, Labour and Welfare in Japan, the Food and Drug Administration (FDA) in the United States, and the like) and approved by the supervisory authority. The instructions are so-called package inserts, and are usually provided in paper form, but is not limited thereto, and may also be provided, for example, in a form such as electronic media (for example, a website provided on the Internet, e-mail).

The term "about" refers to the indicated value plus or minus 10%. When used for temperature, "about" refers to the indicated temperature plus or minus 5°C, and when used for pH, "about" refers to the indicated pH plus or minus 0.5.

### (Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure by taking into account the description herein. It is also understood that the following embodiments may be used alone or in combination.

### (Control of expression of Rep gene)

In one aspect, the present disclosure provides control of the expression of a Rep gene by control of the expression of a helper gene. It is contemplated that any means for achieving this are within the scope of the present disclosure. For example, even if not explicitly stated, a description of a method of using a component contemplates embodiments that reflect other means, such as compositions comprising the component, the use of the component, and the component for use in the method.

Providing control of the expression of a Rep gene can control the production of virus-derived constructs, and doing so can reduce damage to producer cells. Without wishing to be bound by any particular theory, it is believed that the gene product of the helper gene (such as a protein) acts on the promoter of Rep (p5, p19, p40, etc.) to affect control of Rep expression.

In one aspect, the present disclosure provides a method for controlling the expression of a Rep gene, including a step of controlling the expression of a helper gene. In one embodiment, the helper gene includes a gene selected from adenovirus E1B, E2, E4 and VA. In one embodiment, the helper gene includes an adenovirus E1B gene. In one embodiment, the helper gene includes two or more helper genes related to the production of an adeno-associated virus (AAV) -derived construct, and optionally, the helper genes can be selected from E1A, E1B, E2, E4, and VA. In one embodiment, the helper gene includes a helper gene selected from E1A and E1B and a helper gene selected from E2, E4 and VA.

In a case where a gene encodes a plurality of types of proteins, it is contemplated that expression of any encoded protein is expression of the gene. For example, while the adenovirus E1B gene can produce two types of proteins, E1B19K and E1B55K, expression of the E1B gene includes expression of only E1B19K, expression of only E1B55K, and expression of both E1B19K and E1B55K. It is understood that the same applies in the context of expression control.

In one embodiment, the Rep gene or a gene product thereof (such as a protein) can have a sequence (base sequence or amino acid sequence) that has at least 75%, at least 80%, at least 85%, at least 90%, at least 91%, at least 92%, at least 93%, at least 94%, at least 95%, at least 96%, at least 97%, at least 98%, or at least 99% identity to Rep or a gene product thereof (such as a protein) of serotype AAV selected from serotypes of type 1 to 12,and serotypes of rh10, DJ, DJ/8, PHP.eB, PHP.S, AAV2-retro, AAV2-QuadYF, AAV2.7m8, AAV6.2, rh.74, AAV2.5, AAV-TT, and Anc80. In one embodiment, the helper gene or a gene product thereof (such as a protein) can have a sequence (base sequence or amino acid sequence) that has at least 75%, at least 80%, at least 85%, at least 90%, at least 91%, at least 92%, at least 93%, at least 94%, at least 95%, at least 96%, at least 97%, at least 98%, or at least 99% identity to a helper gene or a gene product thereof (such as a protein) of a prototype adenovirus selected from 1 to 52 serotypes.

In one embodiment, a decrease in the expression of the helper gene results in a decrease in the expression of the Rep gene. In one embodiment, an increase in the expression of the helper gene results in an increase in the expression of the Rep gene. In one embodiment, the step of controlling the expression of the helper gene reduces the amount of RNA transcribed from the Rep gene and/or the amount of protein encoded by the Rep gene by, for example, about 10% or more, about 20% or more, about 30% or more, about 40% or more, about 50% or more, about 60% or more, about 70% or more, about 80% or more, about 90% or more, or about 95% or more. In one embodiment, the step of controlling the expression of the helper gene increases the amount of RNA transcribed from the Rep gene and/or the amount of protein encoded by the Rep gene by, for example, about 10% or more, about 20% or more, about 50% or more, about 70% or more, about 100% or more, about 150% or more, about 200% or more, about 300% or more, about 500% or more, or about 1000% or more.

In one embodiment, the step of controlling the expression of the helper gene uses an expression control system having at least one of the following functions of controlling the activity of a promoter operably linked to the helper gene, degrading a nucleic acid transcribed from the helper gene, making the helper gene transcriptionally inactive, and degrading a protein encoded by the helper gene. In one embodiment, the expression control system for the helper gene includes a combination of a stimulus and a promoter responsive to the stimulus, a combination of a Cre protein and a Cre protein-responsive nucleic acid region (LoxP), a CRISPR-Cas9 system, siRNA or shRNA, or a ubiquitin ligase. In one embodiment, the expression control system for the helper gene includes a stimulus-responsive promoter operably linked to the helper gene, a Cre protein-responsive nucleic acid region that enables removal or inversion of at least a part of a nucleic acid region of the helper gene or a promoter operably linked thereto, a CRISPR-Cas9 system or an shRNA for a nucleic acid transcribed from the helper gene. In one embodiment, the expression control system is activated by addition or removal of a drug.

In one embodiment, the method for controlling the expression of a Rep gene of the present disclosure may further include a step of triggering an expression control system for Rep other than the helper gene. As this expression control system, the same one as the expression control system for the helper gene can be used. For example, the expression control system for Rep can be an expression control system having at least one of the following functions of controlling the activity of a promoter operably linked to the Rep, degrading a nucleic acid transcribed from the Rep, making the Rep transcriptionally inactive, and degrading a protein encoded by the Rep.

In one embodiment, the method for controlling the expression of a Rep gene of the present disclosure is carried out in a cell. In one embodiment, the method for controlling the expression of a Rep gene of the present disclosure is carried out in a producer cell producing an AAV-derived construct. In one embodiment, the method for controlling the expression of a Rep gene controls the production of an AAV-derived construct, wherein a decrease in the expression of the helper gene results in a decrease in the production of the AAV-derived construct, and/or an increase in the expression of the helper gene results in an increase in the production of the AAV-derived construct. Nucleic acid elements and producer cells for the production of AAV-derived constructs are known to those of skill in the art and are also described elsewhere herein.

In one embodiment, the method for controlling the expression of a Rep gene inhibits death and/or functional decline of producer cells. Since helper genes such as E1A are associated with the control of not only Rep but also other helper genes (E2A, E4 and the like may also damage cells), the control of Rep via helper genes can also control damage to producer cells via the control on other helper genes. For example, the death and/or functional decline of producer cells can be suppressed by the step of decreasing the expression of the helper gene. For example, the death and/or functional decline of producer cells can be evaluated in terms of the production amount of an AAV-derived construct per producer cell, and in a case where the production amount of an AAV-derived construct decreases over time under conditions in which the AAV-derived construct can be produced, it is considered that the death and/or functional decline of producer cells has occurred. Alternatively, the death and/or functional decline of producer cells can also be determined by detecting dead cells of the producer cells, and methods for detecting dead cells are known in the art. In one embodiment, the step of controlling the expression of the helper gene is carried out based on measurement results for the death and/or functional decline of producer cells. For example, a timing at which dead cells start to appear when producer cells are continuously cultured under conditions for producing the AAV-derived construct can be recorded, and based on the recording, the timing at which the expression of the helper gene is decreased (such as before dead cells start to appear) can be determined.

### (Combination)

**In** one aspect, the control of expression of a Rep gene by the control of expression of a helper gene of the present disclosure including the above method can use a nucleic acid containing the helper gene and the Rep gene. **In** one aspect, the present disclosure provides a combination for controlling the expression of the Rep gene, including a nucleic acid containing the Rep gene, a nucleic acid containing the helper gene, and an expression control system for the helper gene (particularly, nucleic acid elements thereof). The nucleic acid containing the Rep gene and the nucleic acid containing the helper gene may be the same nucleic acid molecule or different nucleic acid molecules, and in a case where the nucleic acids form a double strand, they may be contained in the same double-stranded nucleic acid complex or may be contained in different double-stranded nucleic acid complexes. **In** a case where the expression control system for the helper gene includes nucleic acid elements, the expression control system for the helper gene, the nucleic acid containing the Rep gene, and the nucleic acid containing the helper gene may be present on the same nucleic acid molecule or on different nucleic acid molecules, and in a case where the nucleic acids form a double strand, they may be contained in the same double-stranded nucleic acid complex or in different double-stranded nucleic acid complexes. It is not precluded that each element of the combination is provided in the same nucleic acid molecule or in the same double-stranded nucleic acid complex.

**In** one embodiment, the helper gene includes a gene selected from adenovirus E1B, E2, E4 and VA. **In** one embodiment, the helper gene includes an adenovirus E1B gene. **In** one embodiment, the helper gene includes two or more helper genes related to the production of an adeno-associated virus (AAV) -derived construct, and optionally, the helper genes can be selected from E1A, E1B, E2, E4, and VA. In one embodiment, the helper gene includes a helper gene selected from E1A and E1B and a helper gene selected from E2, E4 and VA. In a case where multiple genes (such as helper genes and Rep genes) are arranged in the same nucleic acid molecule or in the same double-stranded nucleic acid complex, the order and position can be arbitrary. In a preferred embodiment, the helper gene and the nucleic acid elements of the expression control system are provided in the same nucleic acid molecule or the same double-stranded nucleic acid complex, and the arrangement thereof can be appropriately determined by a person skilled in the art depending on characteristics of the expression control system. For example, the stimuli-responsive promoter may be positioned upstream of the helper gene, and the Cre protein-responsive nucleic acid region may be arranged to sandwich the helper gene or a part thereof (such as a poly A region) or a nucleic acid region of the promoter operably linked to the helper gene.

**In** a case where a gene encodes multiple types of proteins, nucleic acid elements of an expression control system may be arranged for a part encoding each protein. For example, while the adenovirus E1B gene can produce two types of proteins, E1B19K and E1B55K, the nucleic acid elements of the expression control system arranged relative to the E1B gene include nucleic acid elements of the expression control system arranged relative to the part encoding E1B19K, nucleic acid elements of the expression control system arranged relative to the part encoding E1B55K, combinations thereof, and nucleic acid elements of the expression control system arranged relative to the part encoding both E1B19K and E1B55K.

In one embodiment, the Rep gene has a sequence (base sequence or amino acid sequence) that has at least 75%, at least 80%, at least 85%, at least 90%, at least 91%, at least 92%, at least 93%, at least 94%, at least 95%, at least 96%, at least 97%, at least 98%, or at least 99% identity to Rep or a gene product thereof (such as a protein) of serotype AAV selected from serotypes of type 1 to 12, and serotypes of rh10, DJ, DJ/8, PHP.eB, PHP.S, AAV2-retro, AAV2-QuadYF, AAV2.7m8, AAV6.2, rh.74, AAV2.5, AAV-TT, Anc80, or can encode a protein having the sequence. In one embodiment, the helper gene has a sequence (base sequence or amino acid sequence) that has at least 75%, at least 80%, at least 85%, at least 90%, at least 91%, at least 92%, at least 93%, at least 94%, at least 95%, at least 96%, at least 97%, at least 98%, or at least 99% identity to a helper gene or a gene product thereof (such as a protein) of a prototype adenovirus selected from 1 to 52 serotypes, or can encode a protein having the sequence.

In one embodiment, the expression control system has at least one of the following functions of controlling the activity of a promoter operably linked to the helper gene, degrading a nucleic acid transcribed from the helper gene, making the helper gene transcriptionally inactive, and degrading a protein encoded by the helper gene. In one embodiment, the expression control system for the helper gene includes a stimulus-responsive promoter operably linked to the helper gene, a Cre protein-responsive nucleic acid region that enables removal or inversion of at least a part of a nucleic acid region of the helper gene or a promoter operably linked thereto, a CRISPR-Cas9 system or an shRNA for a nucleic acid transcribed from the helper gene. In one embodiment, the expression control system is activated by addition or removal of a drug.

In one embodiment, the combination for controlling the expression of the Rep gene of the present disclosure may include an expression control system for Rep. As this expression control system, the same one as the expression control system for the helper gene can be used.

Any additional elements for producing an adeno-associated virus-derived construct (a nucleic acid encoding a capsid, ITR, etc.) can be further combined. Additional nucleic acid elements may be provided in the same nucleic acid molecule or the same double-stranded nucleic acid complex as the nucleic acid containing the Rep gene and/or the nucleic acid containing the helper gene, or may be provided separately.

Adeno-associated virus (AAV) is a linear single-stranded DNA virus belonging to the genus Dependovirus in the family Parvoviridae, with virus particles measuring 20 to 26 nm in diameter. Adenovirus elements are required for AAV proliferation. At the quantitative end of the AAV genome, there is a T-shaped hairpin structure called an inverted terminal repeat (ITR). This part of the ITR serves as a start point of replication and acts as a primer. Further, the ITR is required for packaging into viral particles and integration into chromosomal DNA of host cells. The left half of the genome contains a rep gene encoding a non-structural protein, that is, a regulatory protein (Rep78, Rep68, Rep52, Rep40) that controls replication and transcription, and the right half of the genome contains a cap gene encoding three capsid proteins of a structural protein (VP1, VP2, VP3).

The life cycle of AAV is divided into latent infection and lytic infection. The former is a case where infection occurs alone, and is characterized by being incorporated into the AAVS1 region (19q13.3-qter) of the long arm of chromosome 19 of the host cell. This incorporation is due to non-homologous recombination and involves Rep. It has been reported that Rep78/Rep68 binds to a base sequence (GAGC repeat sequence) commonly present in the AAVS1 region and the Rep binding region of ITR. Therefore, it is believed that when wild-type AAV infects target cells, Rep binds to the ITR and AAVS1 of the AAV, and site-specific integration of the AAV genome into chromosome 19 occurs through the mediation of Rep. In a case where a helper virus such as adenovirus is simultaneously infected, when a cell latently infected with AAV is further superinfected with the helper virus, replication of AAV occurs, and a large amount of virus is released due to cell destruction (lytic infection).

In one embodiment, the combination of the present disclosure includes a nucleic acid containing a gene of interest between the 5'ITR and the 3'ITR. In one embodiment, the combination of the present disclosure includes a nucleic acid containing a gene of interest, a promoter and a terminator between the 5'ITR and the 3'ITR. In one embodiment, the nucleic acid of the combination of the present disclosure contains the 5'ITR, rep, cap, AAP (assembly activating protein), MAAP (membrane-associated accessory protein) and 3'ITR of AAV, and adenovirus E1A, E1B, E2A, VA and E4. Any gene may be provided in a form that is present in a cell (such as a producer cell) or may be provided in a form that is incorporated into the chromosome of the cell.

In one embodiment, any nucleic acid of the combination of the present disclosure may be in the form of a complex with a cationic substance. Examples of the cationic substance include PEI pro, FectoVIR-AAV, Lipofectamine2000, Lipofectamin3000, FuGENE6, FuGENE HD, and FuGENE 4K.

HEK293 and HEK293T, which are usually used as producer cells for AAV vectors, have E1 genes in the genomes, and therefore, introduction and evaluation of an E1 gene expression control system as in examples of the present description have not been often performed. However, HEK293 or HEK293T in which a helper gene expression control system disclosed herein is incorporated can be novel, since the introduction of the E1 gene expression control system can be useful for the control of Rep, as shown in the examples of the present description.

**In** one embodiment, any nucleic acid of the present disclosure can optionally contain a promoter upstream of the gene. **In** one embodiment, any nucleic acid of the present disclosure can contain a drug selective marker nucleic acid sequence.

The virus-derived construct produced has a structural protein (such as a capsid), and the structural protein can be involved in binding to a tissue or cell. Therefore, targeting of the virus-derived construct to a tissue or cell can be adjusted by modifying the structural protein to modify its binding to the tissue or cell (or a surface structure thereof). **In** one embodiment, the combination of the present disclosure may include a nucleic acid containing a gene encoding a structural protein, and the structural protein may be modified. For example, modifications of the structural protein that adjusts the targeting to a tissue or cell include substitution or fusion with other proteins (other viral capsids (for example, a capsid of a virus of another serotype, VSV-G), an antigen-binding region of an antibody, a ligand protein of a subject organism (a ligand for cancer antigens), etc.). Further, a virus-derived construct with an envelope can contain cell membrane components of a producer cell in the envelope. Therefore, the targeting of the virus-derived construct with an envelope to a tissue or cell can be adjusted by modifying the cell membrane components of the producer cell. **In** one embodiment, the virus-derived construct may be designed to target neural cells (cells of the peripheral or central nervous system, brain cells such as neurons and oligodendrocytes, and the like), lung cells, ocular cells (retinal cells, retinal pigment epithelium, corneal cells, etc.), epithelial cells (for example, intestinal or respiratory epithelial cells), muscle cells (for example, skeletal muscle cells, cardiomyocytes, smooth muscle cells, diaphragm muscle cells), dendritic cells, pancreatic cells (such as islet cells), hepatocytes, cardiomyocytes, bone cells (for example, bone marrow stem cells), hematopoietic stem cells, spleen cells, keratinocytes, fibroblasts, endothelial cells, prostate cells, germ cells, cancer cells, and the like. A surface structure (such as a receptor) specific to each cell is known, and a person skilled in the art can appropriately select a protein that strongly or specifically binds to the surface structure.

In one embodiment, the combination of the present disclosure may include a nucleic acid containing a gene encoding a protein that has attenuated the protein of an origin virus of the virus-derived construct to be produced. A gene encoding any known attenuated viral protein can be included.

In one embodiment, the combination of the present disclosure includes a nucleic acid containing a gene of interest. In one embodiment, the gene of interest can be located between two terminal repeats. The gene of interest can ultimately be incorporated into a virus-derived construct. Such a gene of interest may be encoded by a therapeutic protein, may be encoded by a gene for gene therapy, may be encoded by a gene for gene cell therapy such as CART therapy, may be encoded by a protein-non-coding functional RNA (rRNA, tRNA, microRNA (miRNA), lncRNA, etc.), or may include transcriptional and translational regulatory sequences such as a promoter, a terminator, an insulator, an enhancer, a silencer, and an origin of replication in combination or independently. In one embodiment, the gene of interest includes a viral promoter, such as a cytomegalovirus promoter, a CAG promoter, an SV40 promoter, an RSV promoter (optionally, upstream of a protein-coding gene). In one embodiment, the gene of interest includes an IRES (if necessary, upstream of the protein-coding gene). In one embodiment, the gene of interest can be incorporated into the chromosome of the subject to which the virus-derived construct is administered. In this embodiment, the gene of interest may have a function of controlling the expression of the gene originally possessed by the subject, or may result in long-term protein expression. For example, a virus-derived construct based on adeno-associated virus can be incorporated into the chromosome of the subject. In one embodiment, the gene of interest can be incorporated into a therapeutic cell (for example, chromosome) (for example, via treatment of the cells with the virus-derived construct ex vivo).

**In** one embodiment, the gene of interest can include a plurality of genes. **In** one embodiment, the gene of interest can include 2 to 100 genes, e.g., 2 or more, 3 or more, 4 or more, 5 or more, 7 or more, 10 or more, 12 or more, 15 or more, 20 or more, 25 or more, 30 or more, 40 or more, or 50 or more and 100 or less, 90 or less, 80 or less, 70 or less, 60 or less, 50 or less, 40 or less, 35 or less, 30 or more, 25 or less, 20 or less, 17 or less, 15 or less, 12 or less, or 10 or less genes. In one embodiment, the gene of interest can include a base length of about 0.1 to 1000kbp, e.g., about 0.1kbp or more, about 0.3kbp or more, about 1kbp or more, about 2kbp or more, about 5kbp or more, about 7kbp or more, about 10kbp or more, about 20kbp or more, about 50kbp or more, or about 100kbp or more, and about 1000kbp or less, about 700kbp or less, about 500kbp or less, about 200kbp or less, about 100kbp or less, about 70kbp or less, about 50kbp or less, about 20kbp or less, or about 10kbp or less. In one embodiment, the gene of interest includes a plurality of genes, such that a nucleic acid with high functionality can be delivered to the subject, such as a combination of a tissue (for example, cancer tissue) -specific or time-specific promoter of the subject and a therapeutic gene operably linked thereto (a therapeutic protein coding sequence, a gene for gene therapy, a gene for gene cell therapy, etc.), or a sequence encoding a set of enzymes that enables coordinated expression of a set of enzymes that control a metabolic cascade.

In one embodiment, the protein encoded by the gene of interest includes therapeutic polypeptides (for example, for replacement therapy), immunogenic polypeptides (for example, pathogen polypeptides, cancer antigen polypeptides), and the like. The therapeutic polypeptides include cystic fibrosis transmembrane regulatory protein (CFTR), dystrophin (mini-dystrophin and micro-dystrophin, myostatin propeptide, follistatin, activin type II soluble receptor, IGF-1, anti-inflammatory polypeptides, sarcospan, utrophin, mini-utrophin, coagulation factors (for example, factor VIII, factor IX, factor X, and the like), erythropoietin, angiostatin, endostatin, catalase, tyrosine hydroxylase, superoxide dismutase, leptin, LDL receptor, lipoprotein lipase, ornithine transcarbamylase, β-globin, α-globin, spectrin, α1-antitrypsin, adenosine deaminase, hypoxanthine phosphoribosyltransferase, β-glucocerebrosidase, sphingomyelinase, lysosomal hexosaminidase A, branched chain keto acid dehydrogenase, RP65 protein, cytokine (for example, α-interferon, β-interferon, interferon-γ, interleukin-2, interleukin-4, granulocyte macrophage colony stimulating factor, lymphotoxin, and the like), peptide growth factors, neurotrophic factors and hormone (for example, somatotropin, insulin, insulin-like growth factor 1 and 2, platelet-derived growth factors, epidermal growth factors, fibroblast growth factors, nerve growth factors, neurotrophic factor-3 and -4, brainderived neurotrophic factors, bone morphogenic proteins, glial-derived growth factors, transforming growth factor-α and -β, and the like), lysosomal acid alpha-glucosidase, alphagalactosidase A, tumor necrosis growth factor alpha soluble receptor, S100A1, parvalbumin, adenylyl cyclase type 6, anti-inflammatory factors, anti-myostatin protein, aspartoacylase, suicide gene products (for example, thymidine kinase, cytosine deaminase, diphtheria toxin, tumor necrosis factors), tumor suppressor gene products (for example, p53, Rb, Wt-1), TRAIL, and FAS-ligand.

The pathogen polypeptides include cell surface proteins of pathogenic organisms such as bacteria, fungi, and parasites, and proteins expressed on the surface of viruses (for example, spike proteins, envelope proteins, capsid proteins, etc.). Specific examples of the pathogen polypeptides include orthomyxovirus immunogen (for example, influenza virus hemagglutinin (HA), nucleoprotein), lentivirus immunogen (for example, HIV or SIV envelope GP160 protein, matrix/capsid protein, gag, pol, env gene products), arenavirus immunogen (for example, Lassa fever virus nuclear capsid protein, envelope glycoprotein), poxvirus immunogen (for example, vaccinia L1 or L8 gene products), flavivirus immunogen (for example, yellow fever virus or Japanese encephalitis virus immunogen), filovirus immunogen (for example, Ebola virus or Marburg virus immunogen, such as NP and GP gene products), bunyavirus immunogen (for example, RVFV, CCHF, SFS virus immunogen), coronavirus immunogen (for example, human coronavirus immunogen, such as human coronavirus envelope glycoprotein), poliovirus immunogen, herpesvirus immunogen (for example, CMV, EBV, HSV immunogen), mumps virus immunogen, measles virus immunogen, rubella virus immunogen, diphtheria toxin or other diphtheria immunogen, pertussis antigen, and hepatitis (for example, hepatitis A, hepatitis B, hepatitis C, or the like) immunogen.

The cancer antigen polypeptides include BRCA1 gene products, BRCA2 gene products, gp100, tyrosinase, GAGE-1/2, BAGE, RAGE, LAGE, NY-ESO-1, CDK-4, β-catenin, MUM-1, caspase-8, KIAA0205, HPVE, SART-1, PRAME, p15, melanoma tumor antigen, MART-1, gp100 MAGE-1, MAGE-2, MAGE-3, CEA, TRP-1, TRP-2, P-15, tyrosinase, HER-2/neu gene products, CA125, LK26, FB5 (endosialin), TAG72, AFP, CA19-9, NSE, DU-PAN-2, CA50, SPan-1, CA72-4, HCG, STN (sialyl Tn antigen), c-erbB-2 protein, PSA, L-CanAg, estrogen receptor, milk fat globulin, p53 tumor suppressor protein, mucin antigen, telomerase, nuclear matrix protein, prostatic acid phosphatase, and papillomavirus antigen.

In one embodiment, the gene of interest can be a gene for treating a specific disease (for example, a gene for gene therapy). The gene to be selected for a specific disease can be appropriately selected by those skilled in the art. Examples of such diseases include infections caused by various pathogens, cystic fibrosis, hemophilia A, hemophilia B, thalassemia, anemia, Alzheimer's disease, multiple sclerosis, Parkinson's disease, Huntington's disease, amyotrophic lateral sclerosis, spinal muscular atrophy, epilepsy, cancer (Melanoma, adenocarcinoma, thymoma, lymphoma, sarcoma, lung cancer, liver cancer, colon cancer, leukemia, uterine cancer, breast cancer, prostate cancer, ovarian cancer, cervical cancer, bladder cancer, kidney cancer, pancreatic cancer, brain cancer, etc.), diabetes, muscular dystrophy, Gaucher's disease, Hurler's disease, adenosine deaminase deficiency, glycogen storage disease, congenital pulmonary emphysema, Lesch-Nyhan syndrome, Nieman-Pick's disease, Tay Sachs disease, Angelman syndrome, maple syrup urine disease, age-related macular degeneration, amaurosis, diabetic retinopathy, retinal degenerative disease, astrocytoma, glioblastoma, heart failure, peripheral arterial disease, arthritis, arthropathy, intimal hyperplasia, AIDS, muscle wasting, kidney deficiency, hepatitis, LDL receptor deficiency, hyperammonemia, Krabbe's disease, Batten disease, spinal cerebral ataxia, phenylketonuria, autoimmune disease, amino acid metabolism disorder, organic acid metabolism disorder, fatty acid metabolism disorder, mitochondrial disease, carbohydrate metabolism disorder, lysosome disease, peroxisome disease, metal metabolism disorder, purine pyrimidine metabolism disorder, vitamin metabolism disorder, neurotransmitter disorder, lipid metabolism disorder, connective tissue disorder, congenital porphyria, α1-antitrypsin deficiency, lysosomal storage disease, mucopolysaccharidosis disorder, Fabry disease, Canavan disease, Leigh disease, Refsum's disease, Tourette syndrome, primary lateral sclerosis, progressive muscular atrophy, Pick's disease, muscular dystrophy, myasthenia gravis, Binswanger's disease, cerebral infarction, mood disorder, depression, bipolar affective disorder, persistent affective disorder, secondary mood disorder, schizophrenia, drug dependence disorder, anxiety, obsessive-compulsive disorder, body expressive disorder, dissociative disorder, grief, postpartum depression, hallucinations, delusions, dementia, paranoia, autism spectrum disorder, attention deficit disorder, mental disorder, sleep disorder, pain disorder, eating disorder, weight disorder, obesity, cachexia, anorexia nervosa, and binge eating disorder. In one embodiment, the gene of interest can be incorporated into a therapeutic cell (for example, chromosome) for use in CART therapy and the like (for example, via treatment of the cells with the virus-derived constructs ex vivo).

### (Cell)

In one aspect, the present disclosure provides control of the expression of a Rep gene using a cell. In one embodiment, the method for controlling the expression of a Rep gene described herein can be performed using a cell. Typically, the cell is a producer cell (for example, for producing an AAV-derived construct). In one embodiment, the present disclosure provides a cell containing a nucleic acid containing a Rep gene and a nucleic acid containing a helper gene. In one embodiment, the present disclosure provides a cell containing nucleic acid elements of a combination for controlling the expression of a Rep gene described herein. In one embodiment, the present disclosure provides a method for producing a cell including a step of introducing at least one element of a combination for controlling the expression of a Rep gene described herein.

Examples of producer cells include, for example, human embryonic kidney cells (established by transfecting human embryonic kidney cells with DNA obtained by cleaving adenovirus 5), 911 cells, PER.C6 cells, E1 transformed amniotic membrane cells, E1 transformed A549 cells, GH329:HeLa cells, HEK293 cells, IT293 SF cells, HEK293T, HEK293F, Vero cells, CHO cells, Sf9 cells, Freestyle (trademark) 293-F, Expi293 F (trademark), Expi293 inducible, Expi293 NGT-Viral Production Cells 1.0, Viral Production Cells 2.0 (VPC2.0 cells), AAVpro (registered trademark) 293 T Cell Line, Lenti-X (trademark) 293 T Cell Line, FreeStyle (trademark) CHO-S cells, ExpiCHO-S (trademark), VirusExpress (trademark) 293 AAV producing cells, and VirusExpress (trademark) 293 T lentivirus-producing cells, but not limited to these. Producer cells for producing an AAV-derived construct include cells such as HEK293, HEK293T, HEK293F, Hela, Sf9, among others.

In one embodiment, the cell contains one or more of a Rep gene, a helper gene, and nucleic acid elements of an expression control system for the helper gene in a chromosome. In one embodiment, the cell contains an inverted terminal repeat (ITR), a gene of interest (GOI), and a Cap gene (optionally in a chromosome). In one embodiment, the cell contains a helper gene, an ITR, a GOI, a Cap gene, and a Rep gene (optionally in a chromosome). In one embodiment, the cell contains a helper gene, an ITR, a GOI, a Cap gene, and a Rep gene at one locus on a chromosome.

### (Virus-derived construct)

In one aspect, the present disclosure provides a method for producing a virus-derived construct using the method for controlling the expression of a Rep gene described herein, and also provides the produced virus-derived construct. Typically, the virus-derived construct is an AAV-derived construct. The virus-derived construct can be a viral vector, a virus-like particle (VLP), an oncolytic virus (including one modified to proliferate only in cancer cells), or a viral replicon (a self-replicating viral genome that has been deleted for the ability to produce viral particles).

The AAV-derived construct of the present disclosure can be produced based on AAV of a suitable serotype depending on the target tissue. For example, the relationship of (serotype):(target tissue) may be selected as follows: (AAV1):(muscle, liver, respiratory tract, nerve cells), (AAV2):(muscle, liver, nerve cells), (AAV3):(muscle, liver, nerve cells), (AAV4):(muscle, ependymal cells), (AAV5):(muscle, liver, nerve cells, glial cells, respiratory tract), (AAV6):(muscle, liver, respiratory tract, nerve cells), (AAV7):(muscle, liver), (AAV8):(muscle, liver), (AAV9):(muscle, liver, respiratory tract). AAV capsids include wild-type, as well as capsids with targeted mutations (AAV2i8, AAV2.5, AAV-TT, AAV9.HR, etc.), capsids with random mutations (AAV-PHP.B, etc.), capsids designed in silico (such as Anc80), and in one embodiment, the AAV-derived construct of the present disclosure may include these modified capsids.

In one embodiment, the present disclosure provides a virus-derived construct-containing composition including a population of virus-derived constructs of the present disclosure. The virus-derived construct of the present disclosure and the virus-derived construct-containing composition of the present disclosure produced by the methods of the present disclosure can have structural features that cannot be fully analyzed or are very difficult to analyze, such as in the three-dimensional structure or surface modification of the virus-derived construct. Therefore, the feature of being produced by the method of the present disclosure is one of the features that appropriately describe the virus-derived construct of the present disclosure and the virus-derived construct-containing composition of the present disclosure.

### (Composition)

In one embodiment, the present disclosure provides a composition including at least one of the nucleic acid, elements of the expression control system, cells, and a virus-derived construct described herein.

### (Dosage form, etc.)

The nucleic acid, elements of the expression control system, cells, and the virus-derived construct described herein can be provided in a variety of forms (compositions). The form of the composition may be, for example, an injection, a capsule, a tablet, a granule, an inhalant, or the like. An aqueous solution for injection may be stored, for example, in a vial or in a stainless container. The aqueous solution for injection may also contain, for example, physiological saline, sugar (for example, trehalose), NaCl, NaOH, or the like.

In one embodiment, the composition of the present disclosure includes a pharmaceutically acceptable carrier or excipient. Such carriers can also be sterile liquids, such as water and oils, including those of petroleum, animal, plant, or synthetic origin, including but not limited to peanut oil, soybean oil, mineral oil, sesame oil, and the like. In a case where a medicine is administered orally, water is a preferred carrier. In a case where a pharmaceutical composition is administered intravenously, saline and aqueous dextrose are preferred carriers. Preferably, saline solutions and aqueous dextrose and glycerol solutions are used as liquid carriers for injectable solutions. Suitable excipients include light anhydrous silicic acid, crystalline cellulose, mannitol, starch, glucose, lactose, sucrose, gelatin, malt, rice, flour, chalk, silica gel, sodium stearate, glycerol monostearate, talc, sodium chloride, skimmed milk powder, glycerol, propylene, glycol, water, ethanol, carmellose calcium, carmellose sodium, hydroxypropylcellulose, hydroxypropylmethylcellulose, polyvinylacetal diethylaminoacetate, polyvinylpyrrolidone, gelatin, medium chain fatty acid triglyceride, polyoxyethylene hydrogenated castor oil 60, poloxamer, sucrose, carboxymethylcellulose, corn starch, and inorganic salts. The composition can also contain minor amounts of wetting or emulsifying agents, or pH buffering agents, if desired. These compositions can also take the form of solutions, suspensions, emulsions, tablets, pills, capsules, powders, sustained release formulations, and the like. It is also possible to formulate the composition as a suppository using traditional binders and carriers such as triglycerides. Oral formulations can also include standard carriers such as pharmaceutical grades of mannitol, lactose, starch, magnesium stearate, saccharin sodium, cellulose, magnesium carbonate, and the like. Examples of suitable carriers are described in E.W.Martin, Remington's Pharmaceutical Sciences (Mark Publishing Company, Easton, U.S.A.). In addition to these, for example, a surfactant, an excipient, a colorant, a flavoring agent, a preservative, a stabilizer, a buffer, a suspending agent, an isotonizing agent, a binder, a disintegrating agent, a lubricant, a fluidity accelerator, a flavoring agent, and the like may be contained. In one embodiment, the pH of any liquid composition of the present disclosure can be about 3, about 3.5, about 4, about 4.5, about 5, about 5.5, about 6, about 6.5, about 7, about 7.5, about 8, about 8.5, about 9, about 9.5, about 10, about 10.5, about 11, or a range between any two of these values.

Any component of the composition of the present disclosure can be provided as a pharmaceutically acceptable salt, for example, salts formed with free carboxyl groups derived from hydrochloric acid, phosphoric acid, acetic acid, oxalic acid, tartaric acid, and the like, salts formed with free amine groups such as those derived from isopropylamine, triethylamine, 2-ethylaminoethanol, histidine, procaine, and the like, and salts formed with sodium, potassium, ammonium, calcium, and ferric hydroxide.

In a preferred embodiment, the composition can be formulated as a pharmaceutical composition adapted for administration to humans according to known methods. Such compositions can be administered by injection. Typically, the composition for injectable administration is a solution in a sterile isotonic aqueous buffer. When necessary, the compositions can also include a solubilizing agent and a local anesthetic, such as lidocaine that relieve pain at the site of injection. Generally, the components can be supplied separately or mixed together in a unit dosage form and supplied as a lyophilized powder or water free concentrate, for example in a sealed container such as an ampoule or sachet indicating the amount of active agent. In a case where the composition is to be administered by infusion, it is also possible to dispense it using an infusion bottle containing sterile pharmaceutical grade water or saline. In a case where the composition is to be administered by injection, it is also possible to provide ampoules of sterile water for injection or saline so that the components can be mixed prior to administration.

### (Use/application)

The nucleic acids, elements of the expression control system, cells, or the virus-derived construct described As used herein, or a composition containing the same, can be used in a variety of applications, such as gene therapy, functional genomics, cancer vaccination, and/or antiviral vaccination.

In a case where the nucleic acid, elements of the expression control system, cells, or the virus-derived construct of the present disclosure, or a composition containing the same is applied to a subject, the subject is not particularly limited, and can be a mammal (for example, mouse, rat, hamster, rabbit, cat, dog, cow, sheep, pig, monkey, humans, etc.), birds, reptiles, an amphibian, an arthropod, fishes, or the like.

The amount of the nucleic acid, elements of the expression control system, cells, or the virus-derived construct of the present disclosure, or a composition containing the same, will vary depending on the nature of a disorder or condition to be treated or prevented, but can be determined by one of skill in the art using standard clinical techniques based on the description herein. In some cases, in vitro assays can also be used to help identify optimal dosage ranges. The exact dose to be used in the formulation should also be determined according to the judgment of the attending physician and the circumstances of each patient, as it will vary with the route of administration and the severity of the disease or disorder. The dosage of the virus-derived construct or the virus-derived construct-containing composition of the present disclosure is not particularly limited, and may be, for example, 1 x 10⁵, 1 x 10⁶, 1 x 10⁷, 1 x 10⁸, 1 x 10⁹, 1 x 10¹⁰, 1 x 10¹¹, 1 x 10¹², 1 x 10¹³, 1 x 10¹⁴, or 1 x 10¹⁵ virus-derived constructs per dose, and may be within a range of any two of these values. The administration interval is not particularly limited, but for example, it may be administered once or twice per 1, 7, 14, 21, or 28 days, or may be administered once or twice per a range of any two of these values. The dosage, administration interval, and administration method may be appropriately selected according to the age, body weight, symptom, target organ, and the like of the patient.

The route of administration of the nucleic acids, elements of expression control systems, cells, or the virus-derived construct described herein, or a composition containing the same, may be, for example, intravenous, intradermal, subcutaneous, intramuscular, intraperitoneal, intrathecal, intraventricular, intraparenchymal, pulmonary, intranasal, epidural, oral, or the like. In one embodiment, the composition of the present disclosure, can be used with various delivery systems. Such systems include, for example, encapsulation in liposomes, microparticles, and microcapsules; the use of receptormediated endocytosis. It is also possible to administer the medicine by a suitable route, for example by infusion, by bolus injection, by absorption through epithelial or mucocutaneous linings (for example, oral cavity, rectum, intestinal mucosa, and the like), to use an inhaler or nebulizer with an aerosolizing agent as appropriate, and to administer it together with other agents. Administration can also be systemic or local.

The nucleic acid, elements of the expression control system, cells, or the virus-derived construct of the present disclosure, or a composition containing the same can be provided as a kit. **In** one embodiment, the present disclosure provides a kit for controlling the expression of a Rep gene, the kit including a nucleic acid containing a Rep gene, a nucleic acid containing a helper gene, and an expression control system for the helper gene (particularly, nucleic acid elements thereof). **In** one embodiment, the present disclosure provides a drug pack or kit including one or more containers filled with one or more components that can be added to the composition of the present disclosure. **In** some cases, it is also possible to indicate, in association with such a container, information indicating authorization of manufacture, use or sale for human administration by a governmental agency in the form prescribed by the governmental agency regulating the manufacture, use or sale of pharmaceuticals or biological products.

The formulation procedure of the composition of the present disclosure as a medicine or the like is known in the art, and is described in, for example, the Japanese Pharmacopoeia, the United States Pharmacopeia, and pharmacopoeia of other countries. Thus, those skilled in the art, given the description herein, can determine embodiments, such as the amount to be used, without undue experimentation.

As used herein, "or" is used when "at least one or more" of the items listed in the text can be employed. The same applies to "or". In a case where "within a range" of "two values" is specified herein, the range includes the two values themselves.

References cited herein, such as scientific literature, patents, patent applications, and the like, are herein incorporated by reference in their entirety to the same extent as each was specifically described.

The present disclosure has been described above with reference to preferred embodiments for ease of understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described herein, but is limited only by the scope of the claims.

### Examples

As the reagents, specifically, products described in examples were used, but equivalent products of other manufacturers (Sigma-Aldrich, FUJIFILM Wako Pure Chemical Corporation, Nacalai Tesque, Inc., R & D Systems, USCN Life Science INC, etc.) can be substituted.

### (Example 1: Control of expression of Rep by helper gene control system incorporated into producer cell genome)

Producer cells in which various genes were incorporated into the genome were established by lentivirus. Note that, incorporation of various genes described herein into the chromosome (genome) has been confirmed (data not shown).

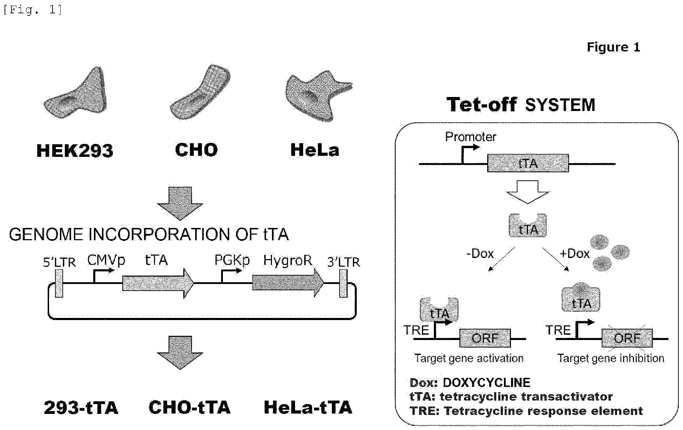

### • Establishement of CHO-tTA cells and HeLa-tTA cells (FIG. 1)

Tetracycline transactivator (tTA) suppresses the expression of downstream genes by binding to a tetracycline response element (TRE) in the presence of doxycycline (dox). For a test by the expression control system (Tet-off system) by tTA-dox-TRE, cells in which tTA was incorporated into the genome were established.

5 x 10⁶ 293T cells were seeded in a T75 flask on the day before transfection. After 24 hours of culture at 37°C and 5% CO₂, a transfection solution was added in which 500 µL of Opti-MEM medium containing three types of lentivirus packaging plasmids (pDNA# 1 to 3) shown in the table below and a plasmid (pDNA# 8) containing tTA that is a gene of interest (GOI) and 500 µL of Opti-MEM medium containing 10 µL of PEIpro (registered trademark) (PolyPlus-transfection SAS) were mixed. After 72 hours of culture, the supernatant was collected and filtered through a 0.45 µm filter to prepare a virus fluid.

**Table 1**

| Plasmid DNA used in establishment of CHO-tTA and HeLa-tTA cells | | | |
|---|---|---|---|
| pDNA# | Transfected pDNA | Length [bp] | Amount of transfected pDNA [µg] |
| 1 | pVSV-G | 5900 | 1.65 |
| 2 | pPACKH1-GAG | 11100 | 3.10 |
| 3 | pPACKH1-REV | 5500 | 3.08 |
| 8 | SYNp72_pLV-CMV-tTA-PGK-HygR | 7760 | 2.17 |

CHO cells and HeLa cells were each seeded at 5 × 10⁵ cells/well in a 6well plate on the day before transfection. After 24 hours of culture at 37°C and 5% CO₂, a 1/1000 volume of a polybrene solution (10 mg/mL) was mixed with the virus fluid and added to the culture. After 24 hours, the medium was replaced, and after another 24 hours, hygromycin was added to the medium to a concentration of 200 µg/mL. Thereafter, medium replacement and drug addition were repeated until all control cells to which the virus fluid was not added were killed by the drug.

In order to confirm the expression of GOI in the obtained cells, RNA extracted from the cells was reverse-transcribed into cDNA, and qPCR was performed. As primer sequences, 5'-CCTACGGCGCTCTGGATATG-3' (SEQ ID NO: 1) and 5'-TACCCACCGTACTCGTCAATTC-3' (SEQ ID NO: 2) were used. As PCR conditions, after 2 minutes at 95°C, 40 cycles of 95°C/15 seconds, 54°C/5 seconds, and 72°C/30 seconds were repeated.

### • Establishment of CHO-tTA-TRE-E1B cells and HeLa-tTA-TRE-E1B cells

Next, E1B control system cells in which helper genes E1B (regions encoding E1B19K and E1B55K) were arranged downstream of the TRE were established.

5 x 10⁶ 293T cells were seeded in a T75 flask on the day before transfection. After 24 hours of culture at 37°C and 5% CO₂, a transfection solution was added in which 500 µL of Opti-MEM medium containing three types of lentivirus packaging plasmids (pDNA# 1 to 3) shown in the table below and a plasmid (pDNA# 10) containing TRE-E1B as a GOI and 500 µL of Opti-MEM medium containing 10 µL of PEIpro (registered trademark) (PolyPlus-transfection SAS) were mixed. After 72 hours of culture, the supernatant was collected and filtered through a 0.45 µm filter to prepare a virus fluid.

**Table 2**

| Plasmid DNA used in establishement of CHO-tTA-TRE-E1B and HeLa-tTA-TRE-E1B expression cells | | | |
|---|---|---|---|
| pDNA# | Transfected pDNA | Length [bp] | Amount of transfected pDNA [µg] |
| 1 | pVSV-G | 5900 | 1.64 |
| 2 | pPACKH1-GAG | 11100 | 3.09 |
| 3 | pPACKH1-REV | 5500 | 3.06 |
| 10 | SYNp141_pLV-TRE-E1B-PuroR | 7969 | 2.22 |

CHO-tTA cells and HeLa-tTA cells were each seeded at 5 × 10⁵ cells/well in a 6well plate on the day before transfection. After 24 hours of culture at 37°C and 5% CO₂, a 1/1000 volume of a polybrene solution (10 mg/mL) was mixed with the virus fluid and added to the culture. After 24 hours, the medium was replaced, and after another 24 hours, puromycin was added to the medium to a concentration of 0.5 µg/mL. Thereafter, medium replacement and drug addition were repeated until all control cells to which the virus fluid was not added were killed by the drug.

In order to confirm the expression of GOI in the obtained cells, RNA extracted from the cells was reverse-transcribed into cDNA, and qPCR was performed. As primer sequences, 5'-ATGGAGGCTTGGGAGTGTTTG-3' (SEQ ID NO: 3) and 5'-ACTAACTTTGCCTGGGATGAGC-3' (SEQ ID NO: 4) were used. As PCR conditions, after 2 minutes at 95°C, 40 cycles of 95°C/15 seconds, 54°C/5 seconds, and 72°C/30 seconds were repeated.

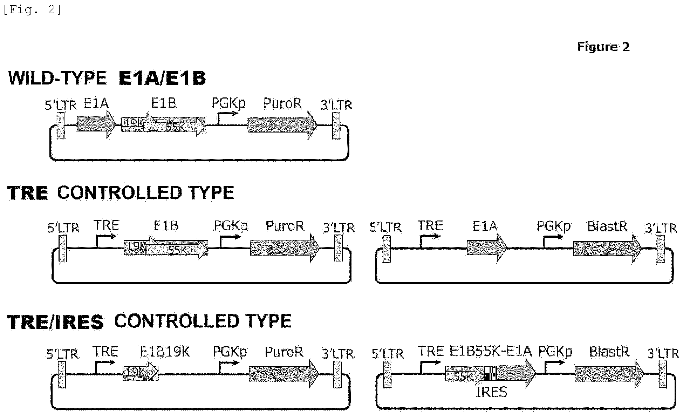

### • Establishment of CHO-tTA-TRE-E1B-TRE-E1A cells and HeLa-tTA-TRE-E1B-TREE1A cells (FIG. 2: TRE controlled type)

An E1A control system in which the helper gene E1A was arranged downstream of the TRE was incorporated into the E1B control system cells established above to establish E1A-E1B control system cells.

5 x 10⁶ 293T cells were seeded in a T75 flask on the day before transfection. After 24 hours of culture at 37°C and 5% CO₂, a transfection solution was added in which 500 µL of Opti-MEM medium containing three types of lentivirus packaging plasmids (pDNA# 1 to 3) shown in the table below and a plasmid (pDNA# 12) containing TRE-E1A as a GOI and 500 µL of Opti-MEM medium containing 10 µL of PEIpro (registered trademark) (PolyPlus-transfection SAS) were mixed. After 72 hours of culture, the supernatant was collected and filtered through a 0.45 µm filter to prepare a virus fluid.

**Table 3**

| Plasmid DNA used in establishment of CHO-tTA-TRE-E1B-TRE-E1A and HeLa-tTA-TREE1B-TRE-E1A expression cells | | | |
|---|---|---|---|
| pDNA# | Transfected pDNA | Length [bp] | Amount of transfected pDNA [µg] |
| 1 | pVSV-G | 5900 | 1.69 |
| 2 | pPACKH1-GAG | 11100 | 3.18 |
| 3 | pPACKH1-REV | 5500 | 3.15 |
| 12 | SYNp144_pLV-TRE-E1A-BlastR | 7760 | 1.98 |

CHO-tTA-TRE-E1B cells and HeLa-tTA-TRE-E1B cells were each seeded at 5 × 10⁵ cells/well in a 6well plate on the day before transfection. After 24 hours of culture at 37°C and 5% CO₂, a 1/1000 volume of a polybrene solution (10 mg/mL) was mixed with the virus fluid and added to the culture. After 24 hours, the medium was replaced, and after another 24 hours, blasticidin was added to the medium to a concentration of 20 µg/mL. Thereafter, medium replacement and drug addition were repeated until all control cells to which the virus fluid was not added were killed by the drug.

In order to confirm the expression of GOI in the obtained cells, RNA extracted from the cells was reverse-transcribed into cDNA, and qPCR was performed. As primer sequences, 5'-tctgccacggaggtgttattac-3' (SEQ ID NO: 5) and 5'-cagccagtacctcttcgatcag-3' (SEQ ID NO: 6) were used. As PCR conditions, after 2 minutes at 95°C, 40 cycles of 95°C/15 seconds, 54°C/5 seconds, and 72°C/30 seconds were repeated.

### • Establishment of CHO-tTA-TRE-E1B19K cells and HeLa-tTA-TRE-E1B19K cells

E1B19K control system cells in which only the region encoding E1B19K in the helper gene E1B was arranged downstream of TRE were established.

5 x 10⁶ 293T cells were seeded in a T75 flask on the day before transfection. After 24 hours of culture at 37°C and 5% CO₂, a transfection solution was added in which 500 µL of Opti-MEM medium containing three types of lentivirus packaging plasmids (pDNA# 1 to 3) shown in the table below and a plasmid (pDNA# 11) containing TREE1B19K as a GOI and 500 µL of Opti-MEM medium containing 10 µL of PEIpro (registered trademark) (PolyPlus-transfection SAS) were mixed. After 72 hours of culture, the supernatant was collected and filtered through a 0.45 µm filter to prepare a virus fluid.

**Table 4**

| Plasmid DNA used in establishment of CHO-tTA-TRE-E1B19K and HeLa-tTA-TRE-E1B19K expression cells | | | |
|---|---|---|---|
| pDNA# | Transfected pDNA | Length [bp] | Amount of transfected pDNA [µg] |
| 1 | pVSV-G | 5900 | 1.70 |
| 2 | pPACKH1-GAG | 11100 | 3.20 |
| 3 | pPACKH1-REV | 5500 | 3.17 |
| 11 | SYNp142_pLV-TRE-E1B19K-PuroR | 6704 | 1.93 |

CHO-tTA cells and HeLa-tTA cells were seeded at 5 × 10⁵ cells/well in a 6well plate on the day before transfection. After 24 hours of culture at 37°C and 5% CO₂, a 1/1000 volume of a polybrene solution (10 mg/mL) was mixed with the virus fluid and added to the culture. After 24 hours, the medium was replaced, and after another 24 hours, puromycin was added to the medium to a concentration of 0.5 µg/mL. Thereafter, medium replacement and drug addition were repeated until all control cells to which the virus fluid was not added were killed by the drug.

In order to confirm the expression of GOI in the obtained cells, RNA extracted from the cells was reverse-transcribed into cDNA, and qPCR was performed. As primer sequences, 5'-atggaggcttgggagtgtttg-3' (SEQ ID NO: 3) and 5'-ACTAACTTTGCCTGGGATGAGC-3' (SEQ ID NO: 4) were used. As PCR conditions, after 2 minutes at 95°C, 40 cycles of 95°C/15 seconds, 54°C/5 seconds, and 72°C/30 seconds were repeated.

### • Establishment of CHO-tTA-TRE-E1B19K-TRE-E1B55K-IRES-E1A cells and HeLa-tTA-TRE-E1B19K-TRE-E1B55K-IRES-E1A cells (FIG. 2: TRE/IRES controlled type)

E1B19K-E1B55K/E1A control system cells were established by incorporating a control system in which helper genes E1B55K and E1A (an internal ribosome entry site (IRES) was arranged therebetween) were arranged downstream of the TRE into the E1B19K control system cells established above.

5 x 10⁶ 293T cells were seeded in a T75 flask on the day before transfection. After 24 hours of culture at 37°C and 5% CO₂, a transfection solution was added in which 500 µL of Opti-MEM medium containing three types of lentivirus packaging plasmids (pDNA# 1 to 3) and a plasmid (pDNA# 13) containing TRE-E1B55K-IRES-E1A as a GOI and 500 µL of Opti-MEM medium containing 10 µL of PEIpro (registered trademark) (PolyPlus-transfection SAS) were mixed. After 72 hours of culture, the supernatant was collected and filtered through a 0.45 µm filter to prepare a virus fluid.

**Table 5**

| Plasmid DNA used in establishment of CHO-tTA-TRE-E1B19K-TRE-E1B55K-IRES-E1A and HeLa-tTA-TRE-E1B19K-TRE-E1B55K-IRES-E1A expression cells | | | |
|---|---|---|---|
| pDNA# | Transfected pDNA | Length [bp] | Amount of transfected pDNA [µg] |
| 1 | pVSV-G | 5900 | 1.60 |
| 2 | pPACKH1-GAG | 11100 | 3.01 |
| 3 | pPACKH1-REV | 5500 | 2.98 |
| 13 | SYNp145_pLV-TRE-E1B55K-IRES-E1A-BlastR | 9008 | 2.44 |

CHO-tTA-TRE-E1B19K cells and HeLa-tTA-TRE-E1B19K cells were seeded at 5 × 10⁵ cells/well in a 6well plate on the day before transfection. After 24 hours of culture at 37°C and 5% CO₂, a 1/1000 volume of a polybrene solution (10 mg/mL) was mixed with the virus fluid and added to the culture. After 24 hours, the medium was replaced, and after another 24 hours, blasticidin was added to the medium to a concentration of 15 µg/mL. Thereafter, medium replacement and drug addition were repeated until all control cells to which the virus fluid was not added were killed by the drug.

In order to confirm the expression of GOI in the obtained cells, RNA extracted from the cells was reverse-transcribed into cDNA, and qPCR was performed. As primer sequences, 5'-tctgccacggaggtgttattac-3' (SEQ ID NO: 5) and 5'-cagccagtacctcttcgatcag-3' (SEQ ID NO: 6) were used. As PCR conditions, after 2 minutes at 95°C, 40 cycles of 95°C/15 seconds, 54°C/5 seconds, and 72°C/30 seconds were repeated.

### • Establishment of CHO-wt_E1 cells and HeLa-wt_E1 cells (FIG. 2: wild-type E1A/E1B)

As a control, cells in which wild-type E1A/E1B was incorporated (no expression control system) were established.

5 x 10⁶ 293T cells were seeded in a T75 flask on the day before transfection. After 24 hours of culture at 37°C and 5% CO₂, a transfection solution was added in which 500 µL of Opti-MEM medium containing three types of lentivirus packaging plasmids (pDNA# 1 to 3) shown in the table below and a plasmid (pDNA# 9) containing wt_E1 as a GOI and 500 µL of Opti-MEM medium containing 10 µL of PEIpro (registered trademark) (PolyPlus-transfection SAS) were mixed. After 72 hours of culture, the supernatant was collected and filtered through a 0.45 µm filter to prepare a virus fluid.

**Table 6**

| Plasmid DNA used in establishment of CHO-wt_E1 and HeLa-wt_E1 cells | | | |
|---|---|---|---|
| pDNA# | Transfected pDNA | Length [bp] | Amount of transfected pDNA [µg] |
| 1 | pVSV-G | 5900 | 1.59 |
| 2 | pPACKH1-GAG | 11100 | 2.99 |
| 3 | pPACKH1-REV | 5500 | 2.96 |
| 9 | SYNp116_pLV-E1 genome-PGK-PuroR | 9103 | 2.45 |

CHO cells and HeLa cells were seeded at 5 × 10⁵ cells/well in a 6well plate on the day before transfection. After 24 hours of culture at 37°C and 5% CO₂, a 1/1000 volume of a polybrene solution (10 mg/mL) was mixed with the virus fluid and added to the culture. After 24 hours, the medium was replaced, and after another 24 hours, puromycin was added to the medium to a concentration of 0.5 µg/mL. Thereafter, medium replacement and drug addition were repeated until all control cells to which the virus fluid was not added were killed by the drug.

In order to confirm the expression of GOI in the obtained cells, RNA extracted from the cells was reverse-transcribed into cDNA, and qPCR was performed. As primer sequences, 5'-tctgccacggaggtgttattac-3' (SEQ ID NO: 5) and 5'-cagccagtacctcttcgatcag-3' (SEQ ID NO: 6) were used. As PCR conditions, after 2 minutes at 95°C, 40 cycles of 95°C/15 seconds, 54°C/5 seconds, and 72°C/30 seconds were repeated.

The cells described above were used to evaluate the effects of the helper gene control system on Rep gene expression and viral particle production. An AAV vector was produced by introducing an all-in-one structure AAV vector plasmid (containing other helper genes E2A, VA and E4, Rep, Cap and the GFP gene as a GOI) into a producer cell.

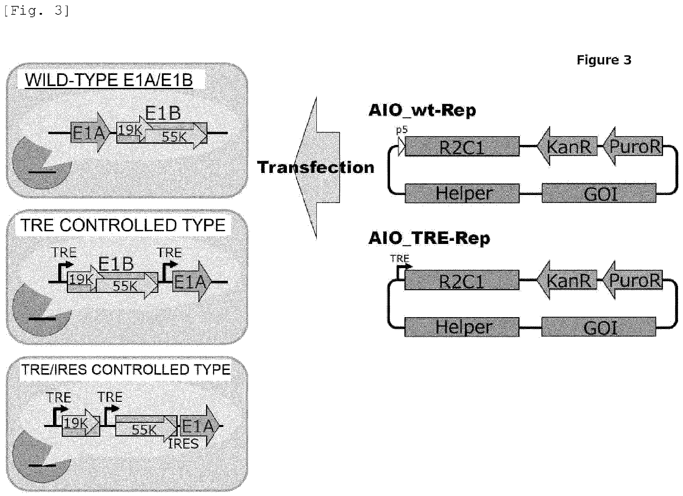

### • Production of rAAV (FIG. 3)

Five types of cells, 6.25 x 10⁵ 293 cells, CHO-tTA-TRE-E1B-TRE-E1A cells, HeLa-tTA-TRE-E1B-TRE-E1A cells, CHO-tTA-TRE-E1B19K-TRE-E1B55K-IRES-E1A cells, and HeLa-tTA-TRE-E1B19K-TRE-E1B55K-IRES-E1A cells were seeded in 6well plates on the day before transfection, respectively. Doxycycline was added to the medium at a final concentration of 3 µg/mL. After 22 hours of culture at 37°C and 5% CO₂, the medium was replaced with DMEM medium (2% FBS, 1% penicillin/streptomycin) for 293 cells and HeLa cells, and was replaced with 1.9 mL of Ham's F-12 medium (2% FBS, 1% penicillin/streptomycin) for CHO cells, and cultured at 37°C and 5% CO₂ for 2 hours. Doxycycline was added at a final concentration of 3 µg/mL (Dox addition conditions). Then a transfection solution was added, in which 50 µL of DMEM medium containing the plasmids shown in the table below and 50 µL of DMEM medium containing 1 time the amount of PEI pro of DNA were mixed.

**Table 7**

| Plasmid DNA used in evaluation of cells with E1 gene genome incorporated by lentivirus | | | |
|---|---|---|---|
| pDNA# | Transfected pDNA | Length [bp] | Amount of transfected pDNA [µg/well] |
| 14 | SYNp180-1_AIO_Helper-Ad5_R2C1_EGFP_km | 19464 | 1.13 |
| 15 | SYNp181_AIO_Helper-Ad5_TRE-R2C1_EGFP_km | 19719 | 1.15 |

### • Evaluation of rAAV

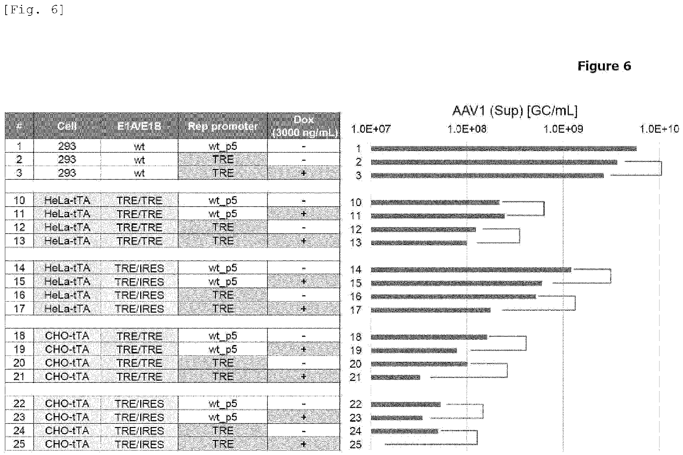

After 72 hours of culture at 37°C and 5% CO₂, the supernatant was collected, and the genome copy number of rAAV was quantified (FIG. 6). The supernatant was subjected to DNaseI treatment for 30 minutes, and reacted at 95°C for 10 minutes. The prepared sample was diluted 1000 times, and subjected to qPCR. Primer sequences of 5'-CATCAATGGGCGTGGATAGC-3' (SEQ ID NO: 7) and 5'-GGAGTTGTTACGACATTTTGGAAA-3' (SEQ ID NO: 8) were used targeting the CMV promoter. As reaction conditions, after 10 minutes at 95°C, 40 cycles of 95°C/15 seconds, 55°C/5 seconds, and 72°C/30 seconds were repeated.

### • Evaluation of gene expression level

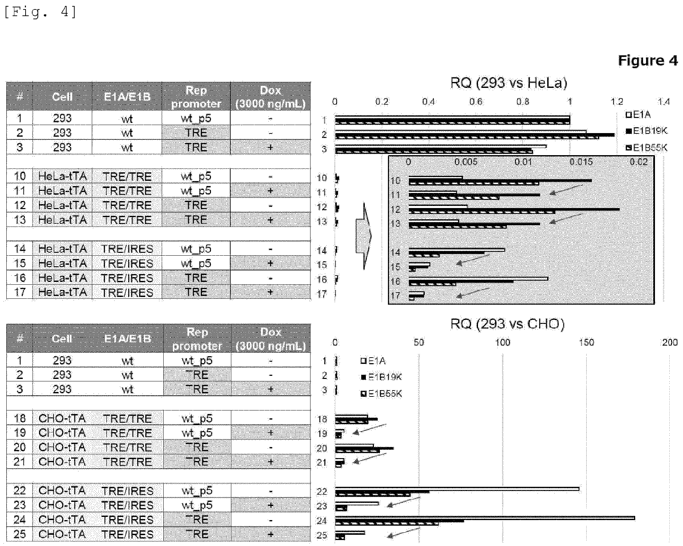

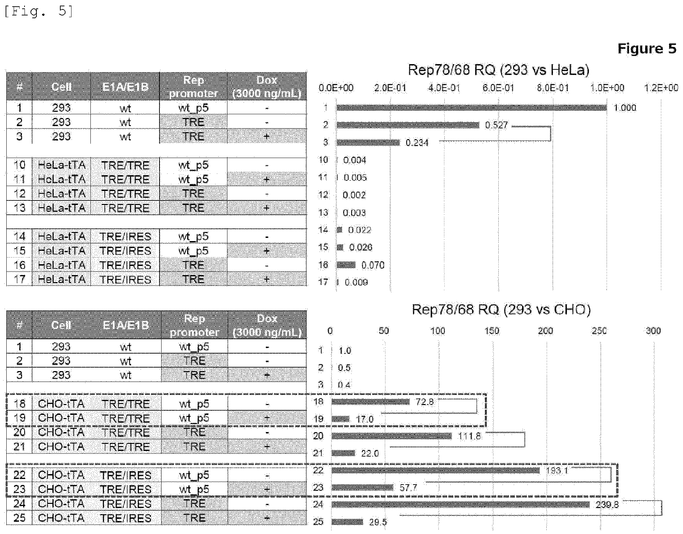

To collect the cells, 1/80 volume of 0.5M EDTA (pH8.0) was added to a 6well plate and allowed to stand at room temperature for 10 minutes. The detached cell suspension was collected and centrifuged at 1000×g for 5 minutes to completely remove the supernatant. RNA extracted from the cell pellet was reverse-transcribed into cDNA, and qPCR was performed. The primer sequences for GAPDH are 5'-CACCCACTCCTCCACCTTTG-3' (SEQ ID NO: 9) and 5'-GCCAAATTCGTTGTCATACCAGG-3' (SEQ ID NO: 10), the primer sequences for E1A are 5'-tctgccacggaggtgttattac-3' (SEQ ID NO: 5) and 5'-cagccagtacctcttcgatcag-3' (SEQ ID NO: 6), and the primer sequences for E1B19K are 5'-atggaggcttgggagtgtttg-3' (SEQ ID NO: 3) and 5'-ACTAACTTTGCCTGGGATGAGC-3' (SEQ ID NO: 4), the primer sequences for E1B55K are 5'-gaaggtgctgaggtacgatgag-3' (SEQ ID NO: 11) and 5'-ggtcacatccagcatcacagg-3' (SEQ ID NO: 12), and the primer sequences for Rep78/68 are 5'-TGACAGCTTTGTGAACTGGGTG-3' (SEQ ID NO: 13) and 5'-CGTGCATGTGGAAGTAGCTCTC-3' (SEQ ID NO: 14). As PCR conditions, after 2 minutes at 95°C, 40 cycles of 95°C/15 seconds, 60°C/5 seconds, and 72°C/30 seconds were repeated. GAPDH expression levels in 293 cells to which doxycycline was not added were standardized, and evaluation was performed using a relative value based on the gene expression levels in these cells (FIGS. 4 and 5).

It was confirmed that, in both the TRE controlled type and TRE/IRES controlled type control systems of the E1A/E1B gene, the expression of the Rep gene was suppressed and the production of AAV particles was reduced as the expression of the E1A/E1B gene was suppressed. Note that, in the measurement system used, the AAV expression level of about 1 × 10⁷ is close to a lower limit of the measurement (quantitative) limit, and may not be a strict quantitative result, and a quantitative correlation is not necessarily established between gene expression and AAV production that undergoes a multistage process thereafter.

Since helper genes such as E1A are associated not only with Rep but also with control of other helper genes and the like, direct Rep control and Rep control via helper genes can have different effects. Since helper genes such as E2A and E4 can also damage cells, helper gene control can achieve better control of damage to producer cells in that the expression of other helper genes in addition to Rep can also be controlled at the same time. Therefore, killing of producer cells can be reduced by Rep control via helper genes.

### (Example 2: Rep control by introduction of multiple plasmids with helper gene control system incorporated)

The E1A/E1B gene have been incorporated into 293 cells, whereas the genes are not incorporated into normal HeLa and CHO cells. Three types of plasmids (Rep/Cap plasmid, E2A/VA/E4 plasmid, and GOI/plasmid) are usually introduced to produce AAV vectors using 293 cells. In order to produce AAV vectors in HeLa cells and CHO cells, a total of two types of plasmids obtained by adding a plasmid containing E1A gene or E1B gene under the control of a control system to an all-in-one structure AAV vector plasmid containing these genes were introduced, and the expression of the Rep gene was evaluated.

Using cells in which only tTA was incorporated into the genome, the E1A/E1B gene under the control of a control system and an all-in-one structure AAV vector plasmid containing other AAV vector production genes (including E2A, VA and E4, Rep, Cap and the GFP gene as a GOI) were transiently introduced into producer cells, and the expression of the Rep gene was evaluated.

### • Production of rAAV

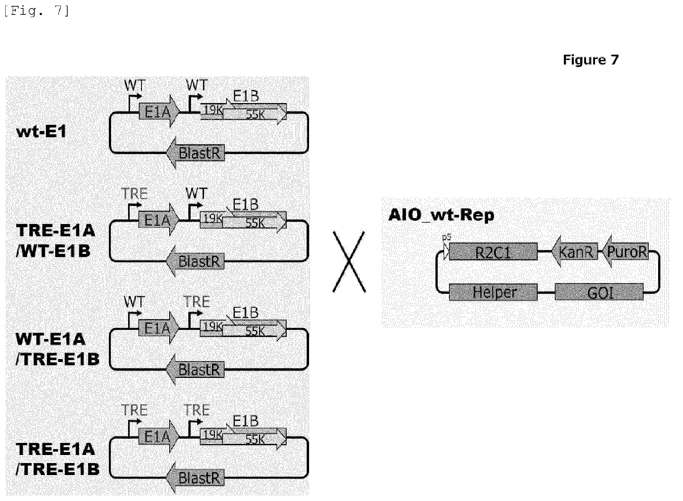

5 x 10⁵ cells/well of HeLa-tTA cells and CHO-tTA cells were each seeded in a 6well plate on the day before transfection. After 22 hours, the medium was replaced with 1.9 mL of DMEM medium or Ham's F-12 medium (2% FBS, 1% penicillin/streptomycin), and cultured at 37°C and 5%CO₂ for 2 hours. To the confluent culture, a transfection solution was added, in which 50 µL of a serum-free medium containing two types of plasmids (any one of pDNA# 14 and pDNA# 22 to 25) shown in the table below (FIG. 7) and 50 µL of a serum-free medium containing 1 time the amount of DNA of PEIpro (registered trademark) (PolyPlus-transfection SAS) were mixed. Doxycycline was added at a final concentration of 0.3 µg/mL. The cells were cultured at 37°C and 5% CO₂ for 72 hours.

**Table 8**

| Plasmid DNA used in evaluation of control for E1A and E1B genes | | | |
|---|---|---|---|
| pDNA# | Transfected pDNA | Length [bp] | Amount of transfected pDNA [µg/well] |
| 22 | SYNp171-1_E1 genome-BlastR | 7266 | 0.34 |
| 23 | SYNp171-2_TRE-E1A_wt-E1B_BlastR | 7286 | 0.34 |
| 24 | SYNp171-3_wt-E1A_TRE-E1B_BlastR | 7717 | 0.36 |
| 25 | SYNp171-4_TRE-E1A_TRE-E1B_BlastR | 7789 | 0.36 |
| 14 | SYNp180-1_AIO_Helper-Ad5_R2C1_EGFP_km | 19464 | 0.91 |

### • Evaluation of rAAV

The supernatant was subjected to DNaseI treatment for 30 minutes, and reacted at 95°C for 10 minutes. The prepared sample was diluted 1000 times, and subjected to qPCR. Primer sequences of 5'-CATCAATGGGCGTGGATAGC-3' (SEQ ID NO: 7) and 5'-GGAGTTGTTACGACATTTTGGAAA-3' (SEQ ID NO: 8) were used targeting the CMV promoter. As reaction conditions, after 10 minutes at 95°C, 40 cycles of 95°C/15 seconds, 55°C/5 seconds, and 72°C/30 seconds were repeated.

### • Evaluation of gene expression level

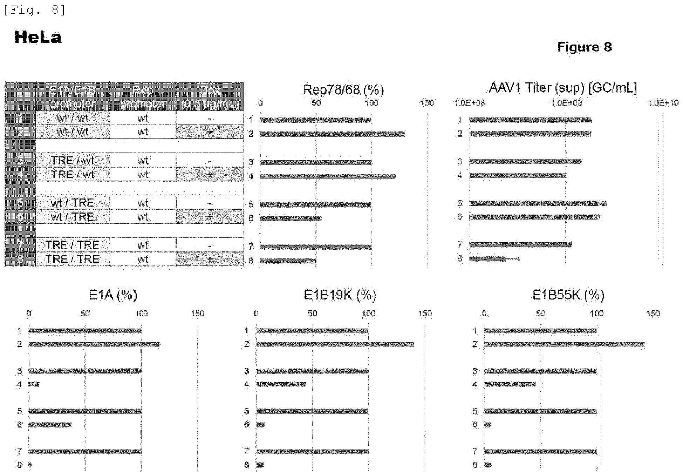

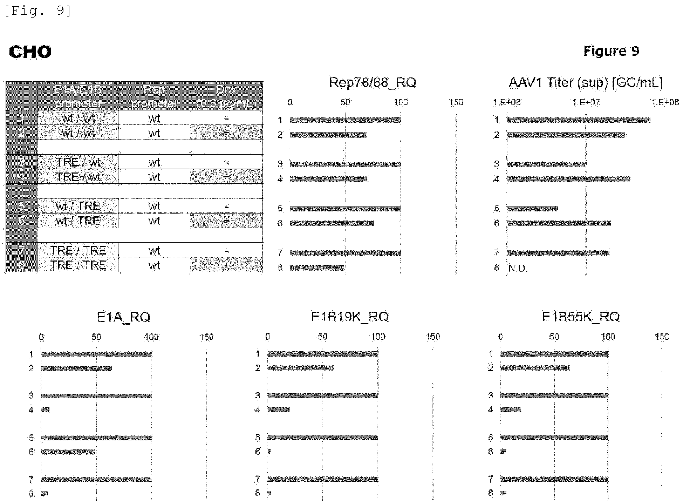

To collect the cells, 1/80 volume of 0.5M EDTA (pH8.0) was added to a 6well plate and allowed to stand at room temperature for 10 minutes. The detached cell suspension was collected and centrifuged at 1000×g for 5 minutes to completely remove the supernatant. RNA extracted from the cell pellet was reverse-transcribed into cDNA, and qPCR was performed. The primer sequences for GAPDH are 5'-CACCCACTCCTCCACCTTTG-3' (SEQ ID NO: 9) and 5'-GCCAAATTCGTTGTCATACCAGG-3' (SEQ ID NO: 10), the primer sequences for E1A are 5'-tctgccacggaggtgttattac-3' (SEQ ID NO: 5) and 5'-cagccagtacctcttcgatcag-3' (SEQ ID NO: 6), the primer sequences for E1B19K are 5'-atggaggcttgggagtgtttg-3' (SEQ ID NO: 3) and 5'-ACTAACTTTGCCTGGGATGAGC-3' (SEQ ID NO: 4), the primer sequences for E1B55K are 5'-gaaggtgctgaggtacgatgag-3' (SEQ ID NO: 11) and 5'-ggtcacatccagcatcacagg-3' (SEQ ID NO: 12), and the primer sequences for Rep78/68 are 5'-TGACAGCTTTGTGAACTGGGTG-3' (SEQ ID NO: 13) and 5'-CGTGCATGTGGAAGTAGCTCTC-3' (SEQ ID NO: 14). As PCR conditions, after 2 minutes at 95°C, 40 cycles of 95°C/15 seconds, 60°C/5 seconds, and 72°C/30 seconds were repeated. The expression level of E1A/E1B was standardized by the GAPDH expression level under the condition without addition of doxycycline in cells into which a plasmid (#22) having a wild-type promoter was introduced, and the expression level was evaluated by a relative value based on the case without addition of doxycycline (FIGS. 8 and 9).

It was confirmed that even when the E1B gene was controlled alone, the expression of the Rep gene was suppressed as the expression of the E1B gene was suppressed. The expression of the E1B gene can be effectively used particularly in controlling the expression of the Rep gene. Further, it was confirmed that suppressing the expression of both E1A and E1B genes is effective for suppressing the expression of the Rep gene.

### (Example 3: Rep control by introduction of all-in-one structure plasmid with control system for both helper genes E1A and E1B incorporated)

Using cells in which only tTA was incorporated into the genome, an all-in-one structure AAV vector plasmid containing the E1A and E1B genes under the control of a control system and other AAV vector production genes (including E2A, VA and E4, Rep, Cap and the GFP gene as a GOI) was transiently introduced into producer cells, and the effects on the expression of the Rep gene and viral particle production were evaluated.

### • Production of rAAV

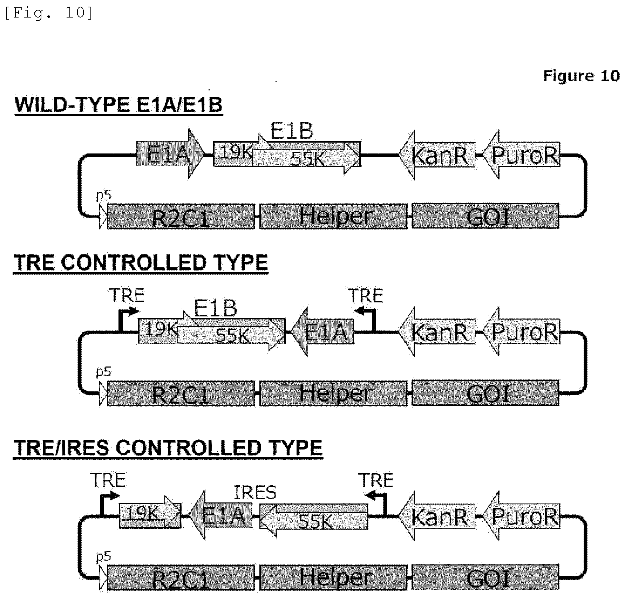

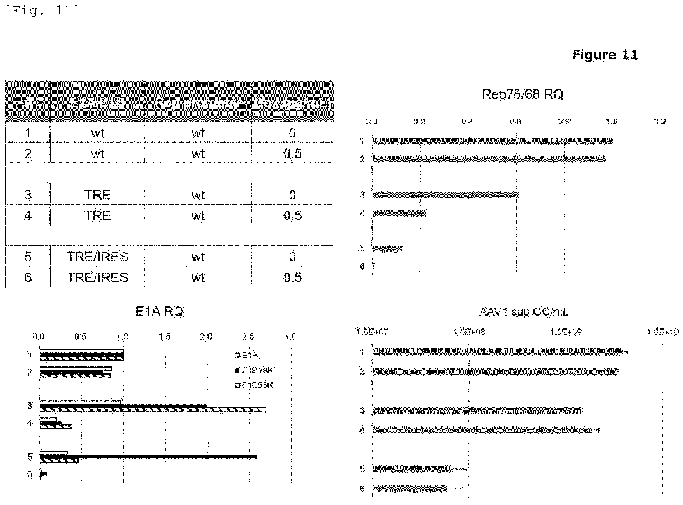

5 x 10⁵ cells/well of HeLa-tTA cells were seeded in a 6well plate on the day before transfection. After 22 hours, the medium was replaced with 1.9 mL of DMEM medium (2% FBS, 1% penicillin/streptomycin), and cultured at 37°C, 5% CO₂ for 2 hours. To the confluent culture, a transfection solution was added, in which 50 µL of a serum-free medium containing any one of the plasmids shown in the table below (FIG. 10) and 50 µL of a serum-free medium containing 1 time the amount of DNA of PEIpro (registered trademark) (PolyPlus-transfection SAS) were mixed. Doxycycline was added at a final concentration of 0.5 µg/mL. After 72 hours of culture at 37°C and 5% CO2, the supernatant was collected and the genomic copy number of rAAV was quantified (FIG. 11).

**Table 9**

| Plasmid DNA used in evaluation of cells with E1 gene introduced using single transfection | | | |
|---|---|---|---|
| pDNA# | Transfected pDNA | Length [bp] | Amount of transfected pDNA [µg/well] |
| 16 | SYNp177_AIO_Helper-Ad5_R2C1_EGFP_wtE1_km | 23500 | 1.10 |
| 17 | SYNp178_AIO_Helper-Ad5_R2C1_EGFP_TRE-E1_km | 23492 | 1.10 |
| 18 | SYNp179_AIO_Helper-Ad5_R2C1_EGFP_IRES-E1_km | 24306 | 1.14 |

### • Evaluation of rAAV

The supernatant was subjected to DNaseI treatment for 30 minutes, and reacted at 95°C for 10 minutes. The prepared sample was diluted 1000 times, and subjected to qPCR. Primer sequences of 5'-CATCAATGGGCGTGGATAGC-3' (SEQ ID NO: 7) and 5'-GGAGTTGTTACGACATTTTGGAAA-3' (SEQ ID NO: 8) were used targeting the CMV promoter. As reaction conditions, after 10 minutes at 95°C, 40 cycles of 95°C/15 seconds, 55°C/5 seconds, and 72°C/30 seconds were repeated.

### • Evaluation of gene expression level

To collect the cells, 1/80 volume of 0.5M EDTA (pH8.0) was added to a 6well plate and allowed to stand at room temperature for 10 minutes. The detached cell suspension was collected and centrifuged at 1000×g for 5 minutes to completely remove the supernatant. RNA extracted from the cell pellet was reverse-transcribed into cDNA, and qPCR was performed. The primer sequences for GAPDH are 5'-CACCCACTCCTCCACCTTTG-3' (SEQ ID NO: 9) and 5'-GCCAAATTCGTTGTCATACCAGG-3' (SEQ ID NO: 10), the primer sequences for E1A are 5'-tctgccacggaggtgttattac-3' (SEQ ID NO: 5) and 5'-cagccagtacctcttcgatcag-3' (SEQ ID NO: 6), and the primer sequences for E1B19K are 5'-atggaggcttgggagtgtttg-3' (SEQ ID NO: 3) and 5'-ACTAACTTTGCCTGGGATGAGC-3' (SEQ ID NO: 4), the primer sequences for E1B55K are 5'-gaaggtgctgaggtacgatgag-3' (SEQ ID NO: 11) and 5'-ggtcacatccagcatcacagg-3' (SEQ ID NO: 12), and the primer sequences for Rep78/68 are 5'-TGACAGCTTTGTGAACTGGGTG-3' (SEQ ID NO: 13) and 5'-CGTGCATGTGGAAGTAGCTCTC-3' (SEQ ID NO: 14). As PCR conditions, after 2 minutes at 95°C, 40 cycles of 95°C/15 seconds, 60°C/5 seconds, and 72°C/30 seconds were repeated. The expression level of E1A/E1B was standardized by the GAPDH expression level under the condition without addition of doxycycline in cells into which a plasmid (#16) having a wild type promoter was introduced, and the expression level was evaluated by a relative value based on the gene expression level of these cells (FIG. 11).

It was confirmed that even when an all-in-one structure plasmid including the E1A/E1B gene control system was used, the expression of the Rep gene was suppressed, and the production of AAV particles was reduced as the expression of the E1A/E1B gene was suppressed.

### (Example 4: Rep control by introduction of multiple plasmids with helper gene control system incorporated)

In order to produce an AAV vector in HeLa cells and CHO cells, a total of four types of plasmids obtained by adding a plasmid containing the E1A/E1B gene under the control of a control system to these three types of plasmids were introduced, and the effects on the expression of the Rep gene and viral particle production were evaluated.

Using cells in which only tTA was incorporated into the genome, the E1A/E1B gene under the control of a control system and three types of AAV vector plasmids containing other AAV vector production genes (including E2A, VA and E4, Rep, Cap and the GFP gene as a GOI) were transiently introduced into producer cells, and the effects on the expression of the Rep gene and viral particle production were evaluated.

### • Production of rAAV

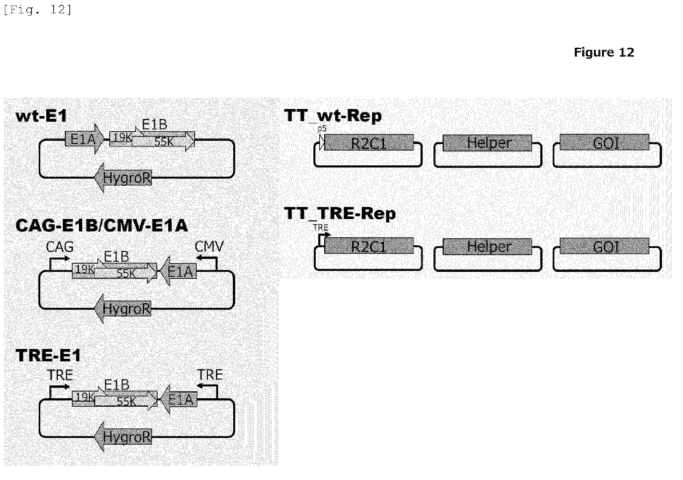

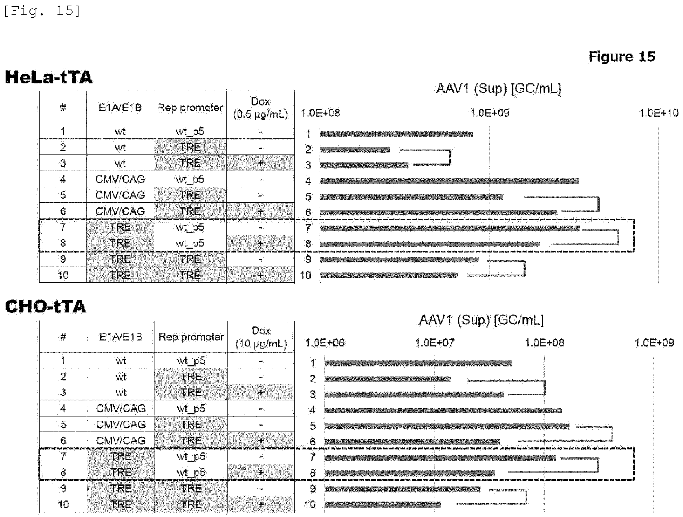

5 x 10⁵ cells/well of HeLa-tTA cells and CHO-tTA cells were each seeded in a 6well plate on the day before transfection. After 22 hours, the medium was replaced with 1.9 mL of DMEM medium or Ham's F-12 medium (2% FBS, 1% penicillin/streptomycin), and cultured at 37°C and 5%CO₂ for 2 hours. To the confluent culture, a transfection solution was added, in which 50 µL of a serum-free medium containing four types of plasmids (any one of pDNA# 4, pDNA# 5, pDNA# 6 or 7, and pDNA# 19 to 21) shown in the table below (FIG. 12) and 50 µL of a serum-free medium containing 1 time the amount of DNA of PEIpro (registered trademark) (PolyPlus-transfection SAS) were mixed. Doxycycline was added to HeLa-tTA cells at a final concentration of 0.5 µg/mL and to CHO-tTA cells at a final concentration of 10 µg/mL. After 72 hours of culture at 37°C and 5% CO₂, the supernatant was collected and the genomic copy number of rAAV was quantified (FIG. 15).

**Table 10**

| Plasmid DNA used in evaluation of cells with E1 gene | | | |
|---|---|---|---|
| pDNA# | Transfected pDNA | Length [bp] | Amount of transfected pDNA [µg/well] |
| 4 | pAAV-GFP | 5118 | 0.24 |
| 5 | SYNp34_pHelper | 11120 | 0.52 |
| 6 | SYNp21_pR2C1 | 6336 | 0.30 |
| 7 | SYNp176-2_TRE-R2C1 | 6599 | 0.31 |
| 19 | SYNp171_E1 genome-HygR | 7887 | 0.37 |
| 20 | SYNp172-3_CMV-E1A-CAG-E1B_HygroR | 9298 | 0.44 |
| 21 | SYNp172-5_TRE-E1A_TRE-E1B_HygroR | 8009 | 0.38 |

### • Evaluation of rAAV

The supernatant was subjected to DNaseI treatment for 30 minutes, and reacted at 95°C for 10 minutes. The prepared sample was diluted 1000 times, and subjected to qPCR. Primer sequences of 5'-CATCAATGGGCGTGGATAGC-3' (SEQ ID NO: 7) and 5'-GGAGTTGTTACGACATTTTGGAAA-3' (SEQ ID NO: 8) were used targeting the CMV promoter. As reaction conditions, after 10 minutes at 95°C, 40 cycles of 95°C/15 seconds, 55°C/5 seconds, and 72°C/30 seconds were repeated.

### • Evaluation of gene expression level

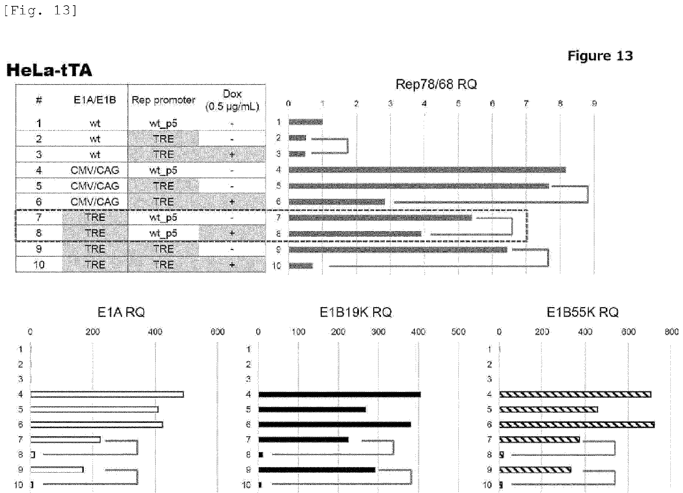

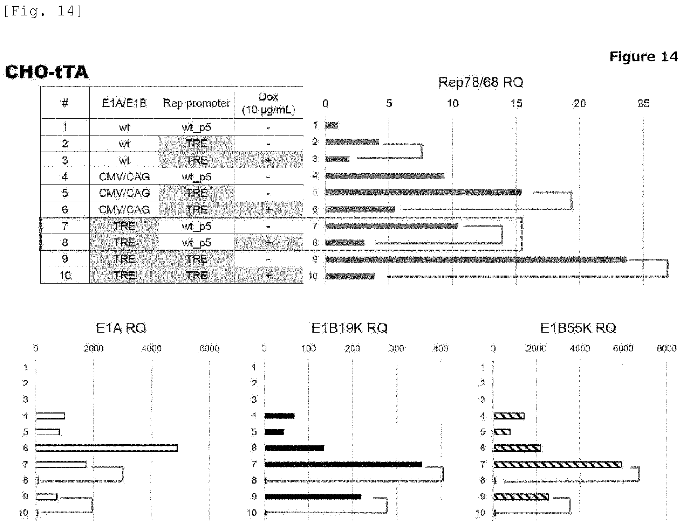

To collect the cells, 1/80 volume of 0.5M EDTA (pH8.0) was added to a 6well plate and allowed to stand at room temperature for 10 minutes. The detached cell suspension was collected and centrifuged at 1000×g for 5 minutes to completely remove the supernatant. RNA extracted from the cell pellet was reverse-transcribed into cDNA, and qPCR was performed. The primer sequences for GAPDH are 5'-CACCCACTCCTCCACCTTTG-3' (SEQ ID NO: 9) and 5'-GCCAAATTCGTTGTCATACCAGG-3' (SEQ ID NO: 10), the primer sequences for E1A are 5'-tctgccacggaggtgttattac-3' (SEQ ID NO: 5) and 5'-cagccagtacctcttcgatcag-3' (SEQ ID NO: 6), the primer sequences for E1B19K are 5'-atggaggcttgggagtgtttg-3' (SEQ ID NO: 3) and 5'-ACTAACTTTGCCTGGGATGAGC-3' (SEQ ID NO: 4), the primer sequences for E1B55K are 5'-gaaggtgctgaggtacgatgag-3' (SEQ ID NO: 11) and 5'-ggtcacatccagcatcacagg-3' (SEQ ID NO: 12), and the primer sequences for Rep78/68 are 5'-TGACAGCTTTGTGAACTGGGTG-3' (SEQ ID NO: 13) and 5'-CGTGCATGTGGAAGTAGCTCTC-3' (SEQ ID NO: 14). As PCR conditions, after 2 minutes at 95°C, 40 cycles of 95°C/15 seconds, 60°C/5 seconds, and 72°C/30 seconds were repeated. The expression level of E1A/E1B was standardized by the GAPDH expression level under the condition without addition of doxycycline in cells into which a plasmid (#19) having a wild-type promoter was introduced, and the expression level was evaluated by a relative value based on the gene expression level of these cells (FIG. 13, FIG. 14).

It was confirmed that even when four types of plasmids including both the gene control systems of E1A and E1B were used, the expression of the Rep gene was suppressed, and the production of AAV particles was reduced as the expression of the E1A/E1B gene was suppressed. Even when Rep expression is suppressed, Rep expression does not completely disappear, and a detailed mechanism regarding AAV production (such as an optimal expression level of each gene) has not been completely elucidated. Therefore, the amount of AAV production is expected to fluctuate to some extent, and a case where the amount of AAV production is larger when Dox is added than when Dox is not added is considered to be affected by such fluctuation.

Each plasmid used in the examples above was obtained or produced as follows.
• #1 pVSV-G
   Purchased from System Biosciences (LV100 A-1).
• #2 pPACKH1-GAG
   Purchased from System Biosciences (LV100 A-1).
• #3 pPACKH1-REV
   Purchased from System Biosciences (LV100 A-1).
• #4 pAAV-GFP
   Purchased from VectorBuilder (pAAV [Exp]-CMV>EGFP:WPRE).
• #5 SYNp34_pHelper

The pUC19 vector plasmid (Addgene: #500005) was treated with restriction enzymes (AatII, PciI). The following three fragments (VA fragment, E4 fragment and E2A fragment) were inserted thereinto using InFusion. The VA fragment was prepared using the genome of Ad5 (see ATCC VR-1516) as a template and the following primers: primer sequences 5'-ttagtaagcttccctcctgacgcggtaggaggaggggagggtgccctgcatg-3' (SEQ ID NO: 15), 5'-ccttttgctcacatgtcaattggtagatgtacctggacatccaggtgatgccggcg-3' (SEQ ID NO: 16). The E4 fragment was prepared using the genome of Ad5 as a template and the following primers: primer sequences 5'-tgtacaGGCGCGCCgaattcgttttagggcggagtaacttgtatgtgttgggaattgtag-3' (SEQ ID NO: 17), 5'-agggaagcttactaaacggtacacaggaaacaggagacacaactccaagtg-3' (SEQ ID NO: 18). The E2A fragment was prepared using the genome of Ad5 as a template and the following primers: primer sequences 5'-gaaaagtgccacctgacgtcgcggccgcGCGATCGCggtacccaactccatgctcaacagtccccaggtacagccc-3' (SEQ ID NO: 19), 5'-gaattcGGCGCGCCtgtacagcccgggcgaccgcaccctgtgacgaaagccgcccgcaag-3' (SEQ ID NO: 20).

### • #6 SYNp21_pR2C1

The pUC19 vector plasmid was treated with restriction enzymes (AatII, PciI). The following three fragments (p5 gene fragment, AAV2 Rep fragment and SV40 ori fragment) were inserted thereinto using InFusion. The p5 gene fragment was prepared using the following primers: primer sequences 5'-gaaaagtgccacctgacgtcgcggccgcggaggggtggagtcgtgacgtgaattacgtcatagggttagggaggtcctgtattag aggtcacgtgagtgttttgcgac-3' (SEQ ID NO: 21), 5'-gttcaaacctcccgcttcaaaatggagaccctgcgtgctcactcgggcttaaatacccagcgtgaccacatggtgtcgcaaaatgtc gcaaaacactca-3' (SEQ ID NO: 22). The AAV2 Rep fragment was prepared using pRC2-mi342 (Takara) as a template and the following primers: primer sequences 5'-gcgggaggtttgaacgcgcagccgccATGCCGGGGTTTTAC-3' (SEQ ID NO: 23), 5'-GATCGCAGCGCTatttaaatcatTTATTGTTCAAAGAT-3' (SEQ ID NO: 24). The SV40 ori fragment was prepared using the SV40ori gene sequence (TOYOBO) as a template and the following primers: primer sequences 5'-aatAGCGCTGCGATCGCggcctccaaaaaagc-3' (SEQ ID NO: 25), 5'-ccttttgctcacatgtcaattgatcccgcccctaact-3' (SEQ ID NO: 26). The resulting plasmid was treated with restriction enzymes (SwaI, AfeI). An AAV1Cap fragment was inserted thereinto using InFusion. The AAV1 Cap fragment was prepared using pRC1 (Takara) as a template and the following primers: primer sequences 5'-AACAATAAatgatttaaatcagg-3' (SEQ ID NO: 27), 5'-ggccGCGATCGCAGCGCTgtagccatggaaactagata-3' (SEQ ID NO: 28).

### • #7 SYNp176-2_TRE-R2C1

SYNp21_pR2C1 of plasmid #6 was treated with restriction enzymes (NotI, BamHI). A TRE fragment was inserted thereinto using InFusion. The TRE fragment was prepared using the TRE gene sequence (VectorBuilder) as a template and the following primers: primer sequences 5'-gtgccacctgacgtcGGCCGGCCgcggccgcggtaccgagctcgactttcac-3' (SEQ ID NO: 29), 5'-TGGTCCTCCTGGATCCGCTAGCcaattcggggccgcggaggc-3' (SEQ ID NO: 30). The resulting plasmid was treated with restriction enzymes (NheI, BamHI). The AAV1 Cap fragment was treated with restriction enzymes (NheI, BamHI) and ligated thereto. The AAV1 Cap fragment was prepared using SYNp21_pR2C1 of plasmid #6 as a template and using the following primers: primer sequences 5'-gggGctagcATGCCGGGGTTTTACGAGAT-3' (SEQ ID NO: 31), 5'-cccGGATCCACTGCTTCTCCGAGGTAATC-3' (SEQ ID NO: 32).

### • #8 SYNp72_pLV-CMV-tTA-PGK-HygR

pLV [Exp]-Puro-CMV>EGFP (VectorBuilder) was treated with restriction enzymes (NdeI, KpnI). The following four fragments (CMV promoter fragment, tTA fragment, mPGK promoter fragment and hygromycin resistant gene fragment) were inserted thereinto using InFusion. The CMV promoter fragment was prepared using pLV [Exp]-Puro-CMV>EGFP (VectorBuilder) as a template and the following primers: primer sequences 5'-ATCAAGTGTATCATATGCCAAG-3' (SEQ ID NO: 33), 5'-tctagacatggtggcATCTGACGGTTCACTA-3' (SEQ ID NO: 34). The tTA fragment was prepared using the tTA gene sequence (VectorBuilder) as a template and the following primers: primer sequences 5'-GATgccaccatgtctaga-3' (SEQ ID NO: 35), 5'-GCGGGAATTCCCGATctacccaccgtactc-3' (SEQ ID NO: 36). The mPGK promoter fragment was prepared using pLV [Exp]-Puro-CMV>EGFP (VectorBuilder) as a template and the following primers: primer sequences 5'-agATCGGGAATTCCCGCG-3' (SEQ ID NO: 37), 5'-ctttttcatGGTGGCCACGTGAGGTC-3' (SEQ ID NO: 38). The hygromycin resistant gene fragment was prepared using the hygromycin resistant gene (VectorBuilder) as a template and the following primers: primer sequences 5'-GCCACCatgaaaaagcctgaac-3' (SEQ ID NO: 39), 5'-TCATTGGTCTTAAAGGTACCctattcctttgccct-3' (SEQ ID NO: 40).

### • #9 SYNp116_pLV-E1 genome-PGK-PuroR

pLV [Exp]-Puro-CMV>EGFP (VectorBuilder) was treated with restriction enzymes (NheI, EcoRI). The following two fragments (cPPT/CTS fragment and TRE fragment) were inserted thereinto using InFusion. The cPPT/CTS fragment was prepared using pLV [Exp]-Puro-CMV>EGFP (VectorBuilder) as a template and the following primers: primer sequences 5'-CGACGGTATCGCTAGCTTTTAAAAGAAAAGG-3' (SEQ ID NO: 41), 5'-TACAAAGTTGGGATCCATCCGATAATCACTAGTA-3' (SEQ ID NO: 42). The TRE fragment was prepared using the TRE gene sequence (VectorBuilder) as a template and the following primers: primer sequences 5'-TGGATCCCAACTTTGTATAGAAAAG-3' (SEQ ID NO: 43), 5'-CTACCCGGTAGAATTCCTCGAGagcctgcttttttgta-3' (SEQ ID NO: 44). The resulting plasmid (pLV-TRE-Puro) was treated with restriction enzymes (BamHI, EcoRI). An E1A/E1B fragment was inserted thereinto using InFusion. The E1A/E1B fragment was prepared using the genome of Ad5 as a template and the following primers: primer sequences 5'-ATTATCGGATGGATCCtagtgtggcggaagtgtgatgttgcaagtgtggcggaac-3' (SEQ ID NO: 45), 5'-CTACCCGGTAGAATTCtcaatctgtatcttcatcgctagagccaaactcagcgcg-3' (SEQ ID NO: 46).

### • #10 SYNp141_pLV-TRE-E1B-PuroR

pLV-TRE-Puro was treated with restriction enzymes (XhoI, EcoRI). The E1B fragment was inserted thereinto using InFusion. The E1B fragment was prepared using the genome of Ad5 as a template and the following primers: primer sequences 5'-aaagcaggctCTCGAGatggaggcttgggagtgtttggaagatttttctgctgtgc-3' (SEQ ID NO: 47), 5'-CTACCCGGTAGAATTCtcaatctgtatcttcatcgctagagccaaactcagcgcgg-3' (SEQ ID NO: 48).

### • #11 SYNp142_pLV-TRE-E1B19K-PuroR

pLV-TRE-Puro was treated with restriction enzymes (XhoI, EcoRI). An E1B19K fragment was inserted thereinto using InFusion. The E1B19K fragment was prepared using the genome of Ad5 as a template and the following primers: primer sequences 5'-aaagcaggctCTCGAGatggaggcttgggagtgtttggaagatttttctgctgtgc-3' (SEQ ID NO: 47), 5'-CCTACCCGGTAGAATTCtcattcccgagggtccaggccggctctcgggttccatgg-3' (SEQ ID NO: 49).

### • #12 SYNp144_pLV-TRE-E1A-BlastR

pLV-TRE-Puro was treated with restriction enzymes (PmlI, PvuII). A Bsd fragment was inserted thereinto using InFusion. The Bsd fragment was prepared using the blasticidin resistance gene (VectorBuilder) as a template and the following primers: primer sequences 5'-GCCTTTCGACCTCACGTGgccaccatggccaagcctttg-3' (SEQ ID NO: 50), 5'-GGCTAAGATCTACAGCTGttagccctcccacacataac-3' (SEQ ID NO: 51). The resulting plasmid (pLV-TRE-Bsd) was treated with restriction enzymes (XhoI, EcoRI). The E1A fragment was inserted thereinto using InFusion. The E1A fragment was prepared using the genome of Ad5 as a template and the following primers: primer sequences 5'-aaagcaggctCTCGAGatgagacatattatctgccacggaggtgttattaccgaag-3' (SEQ ID NO: 52), 5'-CTACCCGGTAGAATTCttatggcctggggcgtttacagctcaagtccaaaggttgc-3' (SEQ ID NO: 53).

### • #13 SYNp145_pLV-TRE-E1B55K-IRES-E1A-BlastR

pLV-TRE-Bsd was treated with restriction enzymes (XhoI, EcoRI). An E1B55K fragment was inserted thereinto using InFusion. The E1B55K fragment was prepared using the genome of Ad5 as a template and the following primers: primer sequences 5'-aaagcaggctCTCGAGatggagcgaagaaacccatctgagcggggggtacctgctg-3' (SEQ ID NO: 54), 5'-CTACCCGGTAGAATTCGTCGACtcaatctgtatcttcatcgctagagccaaac-3' (SEQ ID NO: 55). The resulting plasmid was treated with restriction enzymes (XhoI, SalI). The E1A fragment was inserted thereinto using InFusion. The E1A fragment was prepared using the genome of Ad5 as a template and the following primers: primer sequences 5'-tacagattgaGTCGACatgagacatattatctgccacggaggtgttattac-3' (SEQ ID NO: 56), 5'-CTACCCGGTAGAATTCttatggcctggggcgtttacagctcaagtccaaaggttgc-3' (SEQ ID NO: 53). The resulting plasmid was treated with a restriction enzyme (SalI). An IRES fragment was inserted thereinto using InFusion. The IRES fragment was prepared using the IRES gene sequence (VectorBuilder) as a template and the following primers: primer sequences 5'-gcgatgaagatacagattgagcccctctccctcccccccc-3' (SEQ ID NO: 57), 5'-tggcagataatatgtctcatggttgtggccatattatcat-3' (SEQ ID NO: 58).

### • #14 SYNp180-1_AIO_Helper-Ad5_R2C1_EGFP_km

SYNp177_AIO_Helper-Ad5_R2C1_EGFP_wtE1_km of plasmid #16 was treated with restriction enzymes (FseI, SalI). A p5/AAV2 Rep fragment was inserted into the site from which the E1/p5/AAV2 Rep fragment had been removed using InFusion. The p5/AAV2 Rep fragment was prepared using SYNp21_pR2C1 of plasmid #6 as a template and the following primers: primer sequences 5'-TCACCATCTTGTCGACACAGTCGTTGAAGGG-3' (SEQ ID NO: 59), 5'-agcgcagcgagtcGGCCGGCCgcggccgcggaggggtggagtcg-3' (SEQ ID NO: 60).

### • #15 SYNp181_AIO_Helper-Ad5_TRE-R2C1_EGFP_km

SYNp177_AIO_Helper-Ad5_R2C1_EGFP_wtE1_km of plasmid #16 was treated with restriction enzymes (FseI, SalI) to remove the E1/p5/AAV2 Rep fragment. A TRE/AAV2 Rep fragment obtained by treating SYNp176-2_TRE-R2C1 of plasmid #7 with restriction enzymes (FseI, SalI) was ligated thereto.

### • #16 SYNp177_AIO_Helper-Ad5_R2C1_EGFP_wtE1_km

Prepared by gene synthesis and the OGAB method. The genes contained in this plasmid and their order are shown below: 5'ITR2 (VectorBuilder), CMV promoter (VectorBuilder), EGFP (VectorBuilder), WPRE (VectorBuilder), hGH polyA signal (TAKARA), 3'ITR2 (VectorBuilder), E2A (Ad5), E4 (Ad5), VA (Ad5), Cap (AAV1), Rep (AAV2), p5 (AAV2), E1A (Ad5), E1B (Ad5), pUC ori (pUC19), kanamycin resistance gene (VectorBuilder), HSV TK PolyA signal (TAKARA), puromycin resistance gene (VectorBuilder), mPGK promoter (VectorBuilder). Here, an exemplary procedure of the OGAB method is shown below. The introduced nucleic acid of interest is divided into fragments of about 1kb or less. The ends of each fragment are designed to be unique and complementary only to a specific fragment. Primers are designed for each of the fragments designed in this way, and each fragment is prepared by PCR. Each fragment is cloned into a vector plasmid, and E. coli transformation is performed using each vector plasmid to obtain a clone containing the correct sequence for each fragment. Plasmids are purified from these E. coli clones and the DNA concentration of the plasmid solution is measured. The respective plasmid solutions are fractionated and mixed so that the amount of plasmid of each fragment is equal. A restriction enzyme is added to the mixed liquid to generate DNA fragments each having ends that are unique and complementary only to a specific fragment. Thereafter, an OGAB accumulation vector cleaved with the same restriction enzyme is added to the solution in such an amount that the molar concentration is matched with that of each fragment, ligation is performed, and pairs of fragments having complementary ends (including fragments of the OGAB accumulation vector) are linked. This is added to a Bacillus subtilis competent cell and cultured, and then transformed colonies are obtained. A plasmid having the structure of interest is recovered from this colony.

### • #17 SYNp178_AIO_Helper-Ad5_R2C1_EGFP_TRE-E1_km

Prepared by gene synthesis and the OGAB method. The genes contained in this plasmid and their order are shown below: 5'ITR2 (VectorBuilder), CMV promoter (VectorBuilder), EGFP (VectorBuilder), WPRE (VectorBuilder), hGH polyA signal (TAKARA), 3'ITR2 (VectorBuilder), E2A (Ad5), E4 (Ad5), VA (Ad5), Cap (AAV1), Rep (AAV2), p5 (AAV2), TRE (VectorBuilder), E1A (Ad5), bGH PolyA signal (VectorBuilder), SV40 PolyA signal (VectorBuilder), E1B (Ad5), TRE (VectorBuilder), pUC ori (pUC19), kanamycin resistance gene (VectorBuilder), HSV TK PolyA signal (TAKARA), puromycin resistance gene (VectorBuilder), mPGK promoter (VectorBuilder).

### • #18 SYNp179_AIO_Helper-Ad5_R2C1_EGFP_IRES-E1_km

Prepared by gene synthesis and the OGAB method. The genes contained in this plasmid and their order are shown below: 5'ITR2 (VectorBuilder), CMV promoter (VectorBuilder), EGFP (VectorBuilder), WPRE (VectorBuilder), hGH polyA signal (TAKARA), 3'ITR2 (VectorBuilder), E2A (Ad5), E4 (Ad5), VA (Ad5), Cap (AAV1), Rep (AAV2), p5 (AAV2), TRE (VectorBuilder), E1B19K (Ad5), bGH PolyA signal (VectorBuilder), SV40 PolyA signal (VectorBuilder), E1A (Ad5), IRES (VectorBuilder), E1B55K (Ad5), TRE (VectorBuilder), pUC ori (pUC19), Kanamycin resistance gene (VectorBuilder), HSV TK PolyA signal (TAKARA), puromycin resistance gene (VectorBuilder), mPGK promoter (VectorBuilder).

### • #19 SYNp171_E1 genome-HygR

SYNp116_pLV-E1 genome-PGK-PuroR of plasmid #9 was treated with a restriction enzyme (EcoRI). A PIX fragment was inserted thereinto using InFusion. The PIX fragment was prepared using the genome of Ad5 as a template and the following primers: primer sequences 5'-gcgatgaagatacagattgaggtactgaaatgtgtgggcg-3' (SEQ ID NO: 61), 5'-CTCCCCTACCCGGTAGAATTCagacagcaagacacttgcttgatccaaatccaaacagagtc-3' (SEQ ID NO: 62). The resulting plasmid was treated with restriction enzymes (BamHI, EcoRI). This fragment and the following two fragments (mPGK promoter/hygromycin resistance gene/hGH polA signal fragment and ampicillin resistance gene/pUC ori fragment) were ligated using InFusion. The mPGK promoter/hygromycin resistance gene/hGH polA signal fragment was prepared using the mPGK-HygromycinR-hGH gene sequence (VectorBuilder) as a template and the following primers: primer sequences 5'-cttgctgtctGAATTctaggttctaccgggtaggggaggcgc-3' (SEQ ID NO: 63), 5'-gcggccgcagatctacgcgtgtcgacaaggacagggaagggagcag-3' (SEQ ID NO: 64). The ampicillin resistant gene/pUC ori fragment was prepared using pUC19 as a template and the following primers: primer sequences 5'-tagatctgcggccgcgagcaaaaggccagcaaaag-3' (SEQ ID NO: 65), 5'-cgccacactaGGATCCaagcttgcggccgcaggtggcacttttcgggga-3' (SEQ ID NO: 66).

### • #20 SYNp172-3_CMV-E1A_CAG-E1B_HygroR

The pUC19 vector plasmid was treated with restriction enzymes (AatII, PciI). The following four fragments (CMV promoter fragment, SV40 polyA signal fragment, bGH polyA signal fragment, and CAG promoter fragment) were inserted thereinto using InFusion. The CMV promoter fragment was prepared using the CMV promoter gene sequence (VectorBuilder) as a template and the following primers: primer sequences 5'-cgaaaagtgccacctgcggccgcaagctttctaggcgttacataacttacggtaa-3' (SEQ ID NO: 67), 5'-GAATTCCCTGCAGGGCTCTTCagctctgcttatatagacctc-3' (SEQ ID NO: 68). The SV40 polyA signal fragment was prepared using the SV40 polyA signal gene sequence (VectorBuilder) as a template and the following primers: primer sequences 5'-CCCTGCAGGGAATTCtaagatacattgatgag-3' (SEQ ID NO: 69), 5'-ggaggatccaacttgtttattgcagcttata-3' (SEQ ID NO: 70). The bGH polyA signal fragment was prepared using the bGH polyA signal gene sequence (VectorBuilder) as a template and the following primers: primer sequences 5'-caagttggatcctccccagcatgcctgctattc-3' (SEQ ID NO: 71), 5'-ttgGCTAGCtgtacactgtgccttctagttgcc-3' (SEQ ID NO: 72). The CAG promoter fragment was prepared using the CAG promoter gene sequence (VectorBuilder) as a template and the following primers: primer sequences 5'-caagttggatcctccccagcatgcctgctattc-3' (SEQ ID NO: 73), 5'-gctggccttttgctcgcggccgcagatctacgcgtCCTAGgctcgacattgattattgac-3' (SEQ ID NO: 74). The resulting plasmid was treated with a restriction enzyme (EcoRI). This fragment and the E1A fragment were ligated using InFusion. The E1A fragment was prepared using SYNp116_pLV-E1 genome-PGK-PuroR of plasmid #9 as a template and the following primers: primer sequences 5'-GCCCTGCAGGGaattcatgagacatattatc-3' (SEQ ID NO: 75), 5'-atgtatcttaGAATTttatggcctggggcgtttac-3' (SEQ ID NO: 76). The resulting plasmid was treated with a restriction enzyme (NheI). This fragment and the E1B fragment were ligated using InFusion. The E1B fragment was prepared using SYNp116_pLV-E1 genome-PGK-PuroR of plasmid #9 as a template and the following primers: primer sequences 5'-caaagaattgGCTAGCatggaggcttgggagtgtttg-3' (SEQ ID NO: 77), 5'-acagtgtacagctagtcaatctgtatcttcatcgc-3' (SEQ ID NO: 78). The resulting plasmid was treated with restriction enzymes (AvrII, MluI). This fragment and the mPGK promoter/hygromycin resistant gene/hGH polA signal fragment were ligated using InFusion. The mPGK promoter/hygromycin resistance gene/hGH polA signal fragment was prepared using SYNp171_E1 genome-HygR of plasmid #19 as a template and the following primers: primer sequences 5'-cttgctgtctGAATTctaggttctaccgggtaggg-3' (SEQ ID NO: 79), 5'-ccgcagatctacgcgtgtcgac-3' (SEQ ID NO: 80).

### • #21 SYNp172-5_TRE-E1A_TRE-E1B_HygroR

SYNp172-3_CMV-E1A_CAG-E1B_HygroR of plasmid #20 was treated with restriction enzymes (NheI, AvrII). A TRE fragment was inserted thereinto using InFusion. The TRE fragment was prepared using the TRE gene sequence (VectorBuilder) as a template and the following primers: primer sequences 5'-tcccaagcctccatGCTAGCcaattcggggccgcggaggc-3' (SEQ ID NO: 81), 5'-ccctacccggtagaacctaggcaactttgtatagaaaagttg-3' (SEQ ID NO: 82). The resulting plasmid was treated with restriction enzymes (EcoRI, MluI). This fragment and the following TRE fragment were ligated using InFusion. The TRE fragment was prepared using the TRE gene sequence (VectorBuilder) as a template and the following primers: primer sequences 5'-ctgtccttgtcgacacgcgtgagcaaaaggccagcaaaag-3' (SEQ ID NO: 83), 5'-gataatatgtctcatgaattCagcctgcttttttgtacaaac-3' (SEQ ID NO: 84).

### • #22 SYNp171-1_E1 genome-BlastR

SYNp171_E1 genome-HygR of plasmid #19 was treated with restriction enzymes (EcoRI, SalI). A mPGK promoter/Bsd/hGH polyA signal fragment was inserted thereinto using InFusion. The mPGK promoter/Bsd/hGH polyA signal fragment was prepared using the mPGK promoter-blasticidin resistance gene-hGH polyA signal (VectorBuilder) as a template and the following primers: primer sequences 5'-cttgctgtctGAATTctaggttctaccgggtagggg-3' (SEQ ID NO: 85), 5'-cgcggccgcagatctacgcgtgtcgac-3' (SEQ ID NO: 86).

### • #23 SYNp171-2_TRE-E1A_wt-E1B_BlastR

SYNp171-1_E1 genome-BlastR of plasmid #22 was treated with restriction enzymes (BamHI, XbaI). A TRE/E1A fragment was inserted thereinto using InFusion. The TRE/E1A fragment was prepared using SYNp172-5_TRE-E1A_TRE-E1B_HygroR of plasmid #21 as a template and the following primers: primer sequences 5'-ccgcaagcttGGATCCcaactttgtatagaaaagttg-3' (SEQ ID NO: 87), 5'-tattgcattctctagacacagg-3' (SEQ ID NO: 88).

### • #24 SYNp171-3_wt-E1A_TRE-E1B_BlastR

SYNp171-1_E1 genome-BlastR of plasmid #22 was treated with restriction enzymes (HpaI, PsiI). The following two fragments (E1A PolyA signal fragment and TRE/E1B fragment) were inserted thereinto using InFusion. The E1A PolyA signal fragment was prepared using SYNp171-1_E1 genome-BlastR of plasmid #22 as a template and the following primers: primer sequences 5'-attgcgtgtgtggttaacgcc-3' (SEQ ID NO: 89), 5'-ttctatacaaagttggaggtcagatgtaac-3' (SEQ ID NO: 90). The TRE/E1B fragment was prepared using SYNp172-5_TRE-E1A_TRE-E1B_HygroR of plasmid #21 as a template and the following primers: primer sequences 5'-CAACTTTGTATAGAAAAGTTG-3' (SEQ ID NO: 91), 5'-tccatttatcctttataaaactc-3' (SEQ ID NO: 92).

### • #25 SYNp171-4_TRE-E1A_TRE-E1B_BlastR

SYNp171-2_TRE-E1A_wt-E1B_BlastR of plasmid #23 was treated with restriction enzymes (XbaI, MfeI) to remove the E1B fragment. A TRE-E1B fragment obtained by treating SYNp171-3_wt-E1A_TRE-E1B_BlastR of plasmid #24 with restriction enzymes (XbaI, MfeI) was ligated thereto.

### (Example 5: Rep control by addition of helper gene E4orf6 control)

### • Production of HEK293 tTA cells

Similarly to CHO-tTA and HeLa-tTA, for testing by an expression control system (Tet-off system) by tTA-dox-TRE, HEK293 cells in which tTA was incorporated into the genome were established. For HEK293 cells, Single clone No. 32 (SC32), being a strain that was single cloned and used to evaluate the AAV production amount, was used.

5 x 10⁶ 293T cells were seeded in a T75 flask on the day before transfection. After 24 hours of culture at 37°C and 5% CO₂, a transfection solution was added in which 500 µL of Opti-MEM medium containing three types of lentivirus packaging plasmids (pDNA#1 to 3) shown in the table below and a plasmid (pDNA#8) containing tTA that is a gene of interest (GOI) and 500 µL of Opti-MEM medium containing 10 µL of PEIpro (registered trademark) (PolyPlus-transfection SAS) were mixed. After 72 hours of culture, the supernatant was collected and filtered through a 0.45 µm filter to prepare a virus fluid.

**Table 11**

| Plasmid DNA used in establishment of HEK293-tTA (SC32-tTA) cells | | | |
|---|---|---|---|
| pDN A# | Transfected pDNA | Length [bp] | Amount of transfected pDNA [µg] |
| 1 | pVSV-G | 5900 | 1.65 |
| 2 | pPACKH1-GAG | 11100 | 3.10 |
| 3 | pPACKH1-REV | 5500 | 3.08 |
| 8 | SYNp72_pLV-C MV-tTA-PGK-H ygR | 7760 | 2.17 |

HEK293 (SC32) cells were seeded at 5 × 10⁵ cells/well in a 6well plate on the day before transfection, respectively. After 24 hours of culture at 37°C and 5% CO₂, a 1/1000 volume of a polybrene solution (10 mg/mL) was mixed with the virus fluid and added to the culture. After 24 hours, the medium was replaced, and after another 24 hours, hygromycin was added to the medium to a concentration of 300 µg/mL. Thereafter, medium replacement and drug addition were repeated until all control cells to which the virus fluid was not added were killed by the drug.

In order to confirm the expression of GOI in the obtained cells, RNA extracted from the cells was reverse-transcribed into cDNA, and qPCR was performed. As primer sequences, 5'-CCTACGGCGCTCTGGATATG-3' (SEQ ID NO: 1) and 5'-TACCCACCGTACTCGTCAATTC-3' (SEQ ID NO: 2) were used. As PCR conditions, after 2 minutes at 95°C, 40 cycles of 95°C/15 seconds, 54°C/5 seconds, and 72°C/30 seconds were repeated.

### • Establishment of E1B promoter controlled strain of HEK293 (SC32 tTA)

To control the E1B gene in HEK293 cells, the promoter of the E1B gene from adenovirus incorporated into HEK293 cells was modified by genome editing. As HEK293 cells, SC32 tTA in which tTA was incorporated into the genome of Single Clone No. 32 (SC32) was used.

On the day of electroporation, SC32 tTA was detached by trypsinization. After centrifugation at 100×g for 10 minutes to remove the medium supernatant, the cells were suspended in Opti-MEM medium to prepare a cell suspension of 1×10⁷ cells/mL. 100 µL (1 x 10⁶) of the cell suspension was placed in a cuvette and two types of plasmids shown in Table 12 below (SEQ ID NOs: 26 and 27: SYNp203 and SYNp256) were added. At this time, the plasmid (SEQ ID NO: 27) of the donor DNA containing the E1B gene was used in a state of being treated with restriction enzymes (NotI and MluI). Using an electroporator NEPA21 (Nepa Gene Co., Ltd.), a bearing pulse (125 V, pulse width 7.5 ms, pulse interval 50 ms, twice, attenuation rate 10%, only in polarity + one direction) and a transfer pulse (20 V, pulse width 50 ms, pulse interval 50 ms, 5 times, attenuation rate 40%, polarity +- in two directions) were set to give a pulse. Thereafter, blasticidin was added to a concentration of 10 µg/mL, and culture was performed. Medium replacement and drug addition were repeated until the control cells to which no plasmid had been added were killed by the drug.

At the stage when the control cells were dead, the cells were detached by trypsinization, singulated using a cell sorter SH800 (Sony Corporation), and collected on a 384well plate. Culture in a blasticidin-containing medium was continued even after singulation of cells, and surviving cell clones were selected.

**Table 12**

| Plasmid DNA used in genome editing of E1B gene | | | |
|---|---|---|---|
| pDNA# | pDNA introduced by electroporation | Length [bp] | Amount of pDNA used in electroporation [µg] |
| 26 | SYNp203_n Cas9 (D10 A) -gE1A-g E1B | 10279 | 5.0 |
| 27 | SYNp_256_ wt-E1A_TR E-E1B_v3 | 10676 | 2.5 |

In order to confirm genome incorporation in the obtained cells, PCR was performed using DNA extracted from the cells as a template. As primer sequences, 5'-tctgaagactacagcgtcgc-3' (SEQ ID NO: 93) and 5'-ggtgtacaggatggaacatgaaggc-3' (SEQ ID NO: 94) were used. As PCR conditions, after 2 minutes of incubation at 94°C, 30 cycle of 98°C/10 seconds, 60°C/5 seconds, and 68°C/20 seconds were repeated, and clones in which amplification of specific bands could be confirmed by electrophoresis were selected.

A plasmid in which the promoter of helper gene E4orf6 was controlled was transfected into the cells obtained above, and the effects on the expression of the Rep gene and viral particle production were evaluated.

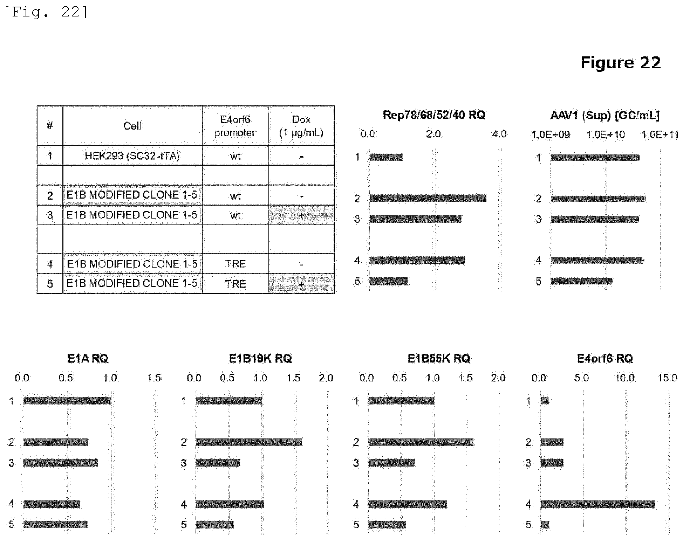

5 x 10⁵ cells/well of the cells and the parent strain (SC32-tTA) were seeded in a 6Well plate on the day before transfection. After 22 hours, the medium was replaced with 1.9 mL of DMEM medium (2% FBS, 1% penicillin/streptomycin), and cultured at 37°C, 5% CO₂ for 2 hours. Thereafter, a transfection solution was added, in which 50 µL of a serum-free medium containing the plasmids shown in the table below (Table 13) and 50 µL of a serum-free medium containing 1 time the amount of DNA of PEIpro (registered trademark) (PolyPlus-transfection SAS) were mixed. Doxycycline was added at a final concentration of 1.0 µg/mL, and cultured at 37°C, 5% CO₂ for 72 hours. The genomic copy number of rAAV was quantified (FIG. 22).

**Table 13**

| pDNA# | Transfected pDNA | Length [bp] | Amount of transfected pDNA [µg/well] |
|---|---|---|---|
| 4 | pAAV-GFP | 5118 | 0.24 |
| 6 | SYNp21_pR2C 1 | 6336 | 0.30 |
| 28 | SYNp517_pHe lper_Ad5_wt -E4orf6 | 9342 | 0.43 |
| 29 | SYNp518_pHe lper_Ad5_TR E-E4orf6 | 9478 | 0.44 |

### • Evaluation of gene expression level

To collect the cells, 1/80 volume of 0.5M EDTA (pH8.0) was added to a 6well plate and allowed to stand at room temperature for 10 minutes. The detached cell suspension was collected and centrifuged at 300×g for 5 minutes to completely remove the supernatant. RNA extracted from the cell pellet was reverse-transcribed into cDNA, and qPCR was performed. The primer sequences for GAPDH are 5'-CACCCACTCCTCCACCTTTG-3' (SEQ ID NO: 9) and 5'-GCCAAATTCGTTGTCATACCAGG-3' (SEQ ID NO: 10), the primer sequences for E1A are 5'-tctgccacggaggtgttattac-3' (SEQ ID NO: 5) and 5'-cagccagtacctcttcgatcag-3' (SEQ ID NO: 6), the primer sequences for E1B19K are 5'-atggaggcttgggagtgtttg-3' (SEQ ID NO: 3) and 5'-ACTAACTTTGCCTGGGATGAGC-3' (SEQ ID NO: 4), the primer sequences for E1B55K are 5'-gaaggtgctgaggtacgatgag-3' (SEQ ID NO: 11) and 5'-ggtcacatccagcatcacagg-3' (SEQ ID NO: 12), the primer sequences for E4orf6 are 5'-acctgcgcttgtggtatgatg-3' (SEQ ID NO: 95) and 5'-gtgcaaggcgctgtatccaaag-3' (SEQ ID NO: 96), and the primer sequences for Rep78/68/52/40 are 5'-atctccttcaatgcggcctc-3' (SEQ ID NO: 97) and 5'-agacggaagccgcatattgg-3' (SEQ ID NO: 98). As PCR conditions, after 2 minutes at 95°C, 40 cycles of 95°C/15 seconds, 60°C/5 seconds, and 72°C/30 seconds were repeated. The expression level was standardized by the GAPDH expression level under the condition without addition of doxycycline in the cells into which the plasmid (#28) containing the E4 gene with an uncontrolled type (sequence derived from the wild-type E4 gene) promoter attached to the parent strain SC32 tTA was introduced, and the expression level was evaluated by a relative value based on the gene expression level of these cell (FIG. 22).

In the cells in which the expression of E1B was suppressed, the suppression of the expression of the Rep gene and the decrease in the production amount of AAV particles could be confirmed by the suppression of E1B alone. Furthermore, it was confirmed that in a case where the expression of both E1B and E4orf6 was suppressed, the suppression of the expression of the Rep gene and the decrease in the production amount of AAV particles were improved.

Each plasmid used in the examples above was obtained or produced as follows.

### • #26 SYNp203_nCas9(D10A)-gE1A-gE1B

Mammalian CRISPR Vector (Dual gRNA) was purchased from VectorBuilder. At this time, 5'-atgagacatattatctgcca-3' (SEQ ID NO: 99) targeting E1A was inserted into gRNA#1, and 5'-gccaaactcagcgcgggtgc-3' (SEQ ID NO: 100) targeting E1B was inserted into gRNA#2. The Cas9 expression plasmid into which these two gRNAs were inserted was treated with restriction enzymes (AgeI and BglII) to change it to an nCas9 expression plasmid. The following two fragments (nCas9 (D10A) and Cas9) were inserted thereinto using InFusion. The nCas9 (D10A) fragment was prepared without a template using the following primers: primer sequences 5'-ttcaggttggaccggtgccaccatgccgaagaaaaagcgcaaggtcgaagcgtccgacaaga-3' (SEQ ID NO: 101), 5'-cccacagagttggtgccgatggccaggccgatgctgtacttcttgtcggacgcttcgacc-3' (SEQ ID NO: 102). The Cas9 fragment was prepared using the Mammalian CRISPR Vector (Dual gRNA) as a template and the following primers: primer sequences 5'-gccatcggcaccaactctgtggg-3' (SEQ ID NO: 103), 5'-tcgttgctgaagatctcttgcag-3' (SEQ ID NO: 104).

### • #27 SYNp256_wt-E1A_TRE-E1B_v3

SYNp171-4_TRE-E1A_TRE-E1B_BlastR of plasmid #25 was treated with restriction enzymes (NotI and NheI) to remove the TRE-E1A-TRE fragment. The following four fragments (5' homology region fragment, E1A promoter fragment, nCas9-unrecognized E1A fragment, and TRE fragment) were inserted thereinto using the overlap extension PCR method and InFusion. The 5' homology region fragment was amplified using HEK293 genome as a template and the following primers: primer sequence 5'-aggaacatccccttccctcggatg-3' (SEQ ID NO: 105), primer sequence 5'-aaaggtgaggcggctccggagaa-3' (SEQ ID NO: 106). Subsequently, it was prepared using the amplified fragment as a template and the following primers: primer sequence 5'-cggcgcgccgcggccgcaggaacatccccttccctcggatg-3' (SEQ ID NO: 107), primer sequence 5'-cgccacactaggatcaagtcatcaacttgttatcctggtttacag-3' (SEQ ID NO: 108). The E1A promoter fragment was prepared using SYNp171-3_wt-E1A_TRE-E1B_BlastR of plasmid #24 as a template and the following primers: primer sequence 5'-gacttgatcctagtgtggcggaagtgtgatg-3' (SEQ ID NO: 109), primer sequence 5'-tcacgccgccgtgacagatgatgtgccgcattttcagtcccggtgtcggag-3' (SEQ ID NO: 110). The nCas9-unrecognized E1A fragment was prepared using SYNp171-3_wt-E1A_TRE-E1B_BlastR of plasmid #24 as a template and the following primers: primer sequence 5'-tcatctgtcacggcggcgtgatcacagaggaaatggccgccagtcttttggaccagctg-3' (SEQ ID NO: 111), primer sequence 5'-gcaaacaaaggcgttaaccacacacgcaatcacaggtttacacc-3' (SEQ ID NO: 112). The TRE fragment was prepared using SYNp171-3_wt-E1A_TRE-E1B_BlastR of plasmid #24 as a template and the following primers: primer sequence 5'-attgcgtgtgtggttaacgcctttgtttgctgaatgagttgatgtaagtttaataaacaactttgtatagaaaagttgggtaccgagc-3' (SEQ ID NO: 113), primer sequence 5'-tcccaagcctccatgctagccaattcgggg-3' (SEQ ID NO: 114). Next, the wild-type E1B fragment was removed by treatment with restriction enzymes (EcoNI and AgeI). The following two fragments (nCas9-unrecognized E1B fragment and E1B 3'UTR fragment) were inserted thereinto using the overlap extension PCR method and InFusion. The nCas9-unrecognized E1B fragment was prepared using SYNp171-3_wt-E1A_TRE-E1B_BlastR of plasmid #24 as a template and the following primers: primer sequence 5'-ccttgggtatcctgtctgagggtaactcca-3' (SEQ ID NO: 115), primer sequence 5'-gtcggagctgccaaactcggctctggtacaggccagcaccaagtgatcgggcc-3' (SEQ ID NO: 116). The E1B 3'UTR fragment was prepared using SYNp171-3_wt-E1A_TREE1B_BlastR of plasmid #24 as a template and the following primers: primer sequence 5'-gagtttggcagctccgacgaggacaccgattgaggtactgaaatgtgtgggcg-3' (SEQ ID NO: 117), primer sequence 5'-gccggttggcgcctaccggtggatgtggaatgtgt-3' (SEQ ID NO: 118). Next, it was treated with restriction enzymes (SalI and MluI). A 3' homology region fragment was inserted thereinto using InFusion. The 3' homology region fragment was amplified using the HEK293 genome as a template and the following primers: primer sequence 5'-gataaatatgtggccggggg-3' (SEQ ID NO: 119), primer sequence 5'-tgaccactgaagcacagcatcacag-3' (SEQ ID NO: 120). Subsequently, it was prepared using the amplified fragment as a template and the following primers: primer sequence 5'-ccctgtccttgtcgaccaaagatgtcagaacaagactcccc-3' (SEQ ID NO: 121), primer sequence 5'-ccttttgctcacgcgttgaccactgaagcacagcatcacag-3' (SEQ ID NO: 122).

### • #28 SYNp517_pHelper_Ad5_wt-E4orf6

The pHelper of SYNp34 in plasmid #5 was treated with restriction enzymes (ApaI and XbaI). Each fragment of the E2A fragment, the E4orf6 fragment, and the VA fragment was inserted thereinto using InFusion. The E2A fragment was prepared using SYNp34_pHelper of plasmid #5 as a template and the following primers: primer sequence 5'-tcagcagcagccgcgggcccttg-3' (SEQ ID NO: 123), primer sequence 5'-gcccgggcgaccgcaccctg-3' (SEQ ID NO: 124). The E4orf6 fragment was prepared using pGETS161-23-006 of plasmid #30 as a template and the following primers: primer sequence 5'-agggtgcggtcgcccgggcgttttagggcggagtaacttgtatgtgttgg-3' (SEQ ID NO: 125), primer sequence 5'-ggttaactccccagcatgcctgctattctc-3' (SEQ ID NO: 126). The VA fragment was prepared using SYNp34_pHelper of plasmid #5 as a template and the following primers: primer sequence 5'-gcaggcatgctggggagttaacccctcctgacgcggtaggaggag-3' (SEQ ID NO: 127), primer sequence 5'-tcgttgacgctctagaccgtgcaaaaggagagcctg-3' (SEQ ID NO: 128).

### • #29 SYNp518_pHelper_Ad5_TRE-E4orf6

The pHelper of SYNp34 in plasmid #5 was treated with restriction enzymes (ApaI and XbaI). The E2A fragment, the TRE fragment, and the E4orf6-polyA-VA fragment were inserted thereinto using InFusion. The E2A fragment was prepared using SYNp34_pHelper of plasmid #5 as a template and the following primers: primer sequence 5'-tcagcagcagccgcgggcccttg-3' (SEQ ID NO: 123), primer sequence 5'-gcccgggcgaccgcaccctg-3' (SEQ ID NO: 124). The TRE fragment was prepared using the TRE promoter sequence (VectorBuilder) as a template and the following primers: primer sequence 5'-agggtgcggtcgcccgggccaactttgtatagaaaagttgggtaccgag-3' (SEQ ID NO: 129), primer sequence 5'-ggaacgccggacgtagtcatcaattcggggccgcggaggc-3' (SEQ ID NO: 130). The E4orf6-polyA-VA fragment was prepared using SYNp517_pHelper_Ad5_wt-E4orf6 of plasmid #28 as a template and the following primers: primer sequence 5'-atgactacgtccggcgttccatttgg-3' (SEQ ID NO: 131), primer sequence 5'-tcgttgacgctctagaccgtgcaaaaggagagcctg-3' (SEQ ID NO: 128).

### • #30 pGETS161-23-006

The species Plasmid#14 of WO 2023/214578 A1 was used. Prepared by gene synthesis and the OGAB method. The genes contained in this plasmid and their order are shown below: VA (Ad5), E2A promoter (Ad5), E2A (Ad5) (ΔSfiI), SV40 PolyA signal (VectorBuilder), E4 promoter (Ad5), E4orf6 (Ad5), bGH polyA signal (VectorBuilder), p40 promoter (AAV1), Cap (AAV1), hGH polyA signal (Takara Bio Inc.), p5 promoter (AAV2), Rep2 (AAV2), and HSV TK polyA signal (Takara Bio Inc.).

### (Example 6: Rep control by other helper gene control)

As in the case of E1A/E1B described above, an expression control system is constructed in which the wild type promoter upstream of the other helper gene (E2A) alone is substituted with a Tet-off-controlled TRE promoter. Using such an expression control system, the effect of expression of other helper genes on Rep expression can be confirmed. The effect on Rep expression can also be confirmed for an expression control system in a case where control of the expression of E1A/E1B and control of the expression of other helper genes are combined.

### (Example 7: Construction of plasmids with various expression control systems incorporated)

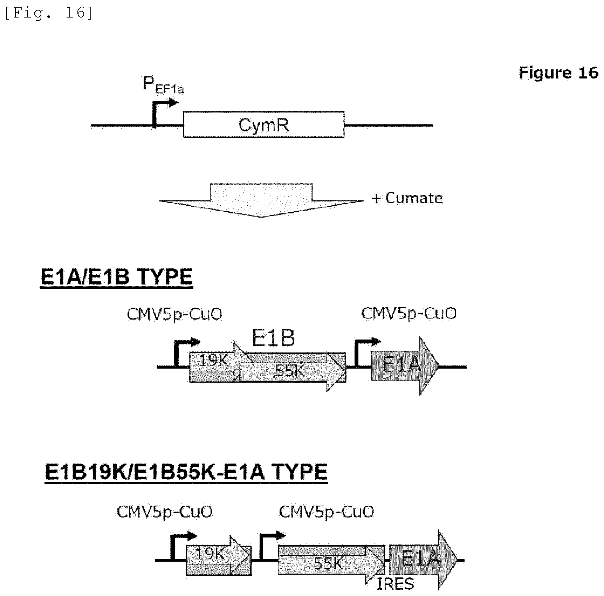

In addition to the exemplary helper gene expression control system described above, helper gene expression control systems having various structures can be used to control the expression of Rep. Other exemplary structures of plasmids for introducing a helper gene expression control system into a cell are shown in FIGS. 16 to 21.

FIG. 16 shows an example of a plasmid structure in which an expression control system of a Cumate-responsive promoter for E1B and E1A is incorporated.

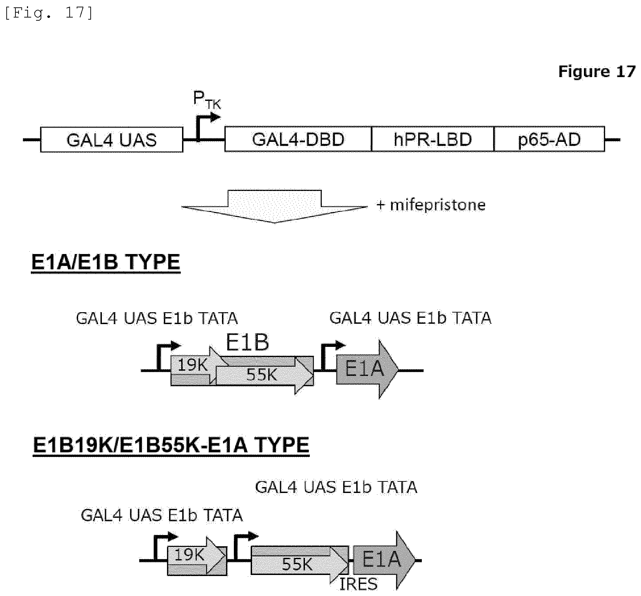

FIG. 17 shows an example of a plasmid structure in which an expression control system of a mifepristone responsive promoter for E1B and E1A is incorporated.

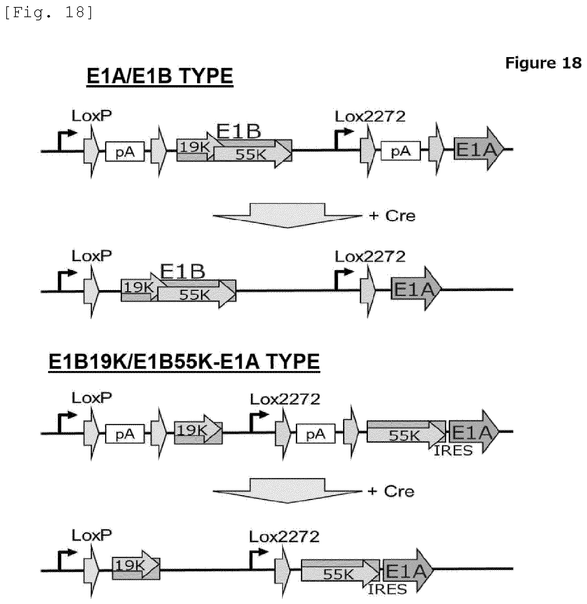

FIG. 18 shows an example of a plasmid structure in which a Cre protein expression control system for E1B and E1A is incorporated. The poly A regions of E1B and E1A are removed when the switch is activated.

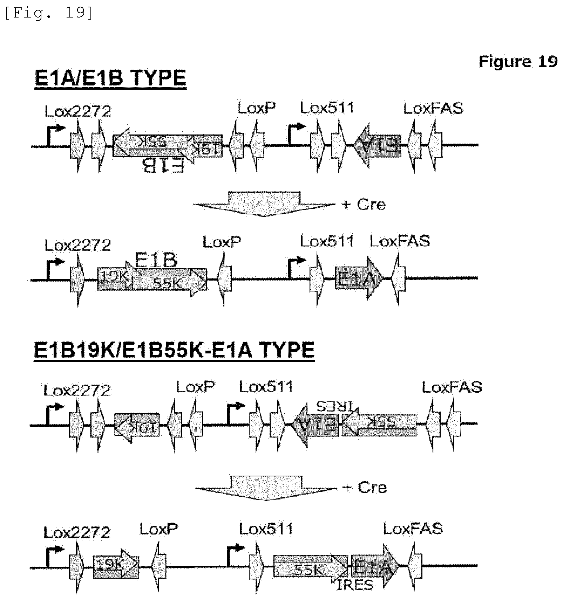

FIG. 19 shows an example of a plasmid structure in which the Cre protein expression control system for E1B and E1A is incorporated. When the switch is activated, the orientations of E1B and E1A are reversed, allowing them to function normally.

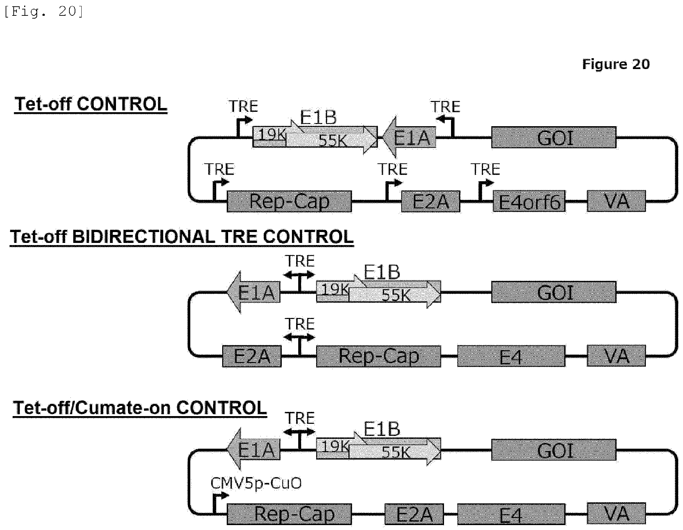

FIG. 20 shows an example of a plasmid structure in which an expression control system for other helper genes (E2A, E4, VA) in addition to E1B and E1A is incorporated.

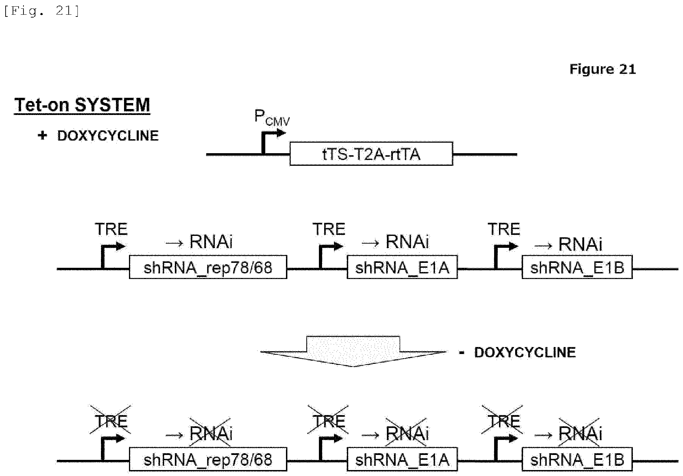

FIG. 21 shows an example of a plasmid structure in which an E1A/E1B expression control system via the shRNA expression control by the Tet-on system and an expression control system for the Rep gene are incorporated. When doxycycline is removed, the expression of shRNA against E1A/E1B and Rep genes (resulting in RNAi) is suppressed, and the expression of E1A/E1B and Rep genes is enhanced.

### (Note)

As described above, although the present disclosure has been illustrated using preferred embodiments of the present disclosure, it is understood that the scope of the present disclosure should be interpreted only by the scope of the claims. It is understood that the patents, patent applications, and literature cited herein should be incorporated by reference to the present description in the same manner as their content were specifically described herein. This application claims priority to Japanese Patent Application No. 2022-210794 filed on December 27, 2022 to the Japanese Patent Office, the entire contents of which are incorporated herein by reference.

### Industrial Applicability

The present disclosure provides a new method for controlling the expression of a Rep gene, thereby making it possible to efficiently provide a virus-derived construct.

### Sequence Listing Free Text

- SEQ ID NO: 1: CCTACGGCGCTCTGGATATG, Primer 1 for tTA
- SEQ ID NO: 2: TACCCACCGTACTCGTCAATTC, Primer 2 for tTA
- SEQ ID NO: 3: ATGGAGGCTTGGGAGTGTTTG, Primer 1 for E1B19K
- SEQ ID NO: 4: ACTAACTTTGCCTGGGATGAGC, Primer 2 for E1B19K
- SEQ ID NO: 5: TCTGCCACGGAGGTGTTATTAC, Primer 1 for E1A
- SEQ ID NO: 6: CAGCCAGTACCTCTTCGATCAG, Primer 2 for E1A confirmation
- SEQ ID NO: 7: CATCAATGGGCGTGGATAGC, Primer 1 for CMV promoter
- SEQ ID NO: 8: GGAGTTGTTACGACATTTTGGAAA, Primer 2 for CMV promoter
- SEQ ID NO: 9: CACCCACTCCTCCACCTTTG, Primer 1 for GAPDH
- SEQ ID NO: 10: GCCAAATTCGTTGTCATACCAGG, Primer 2 for GAPDH
- SEQ ID NO: 11: GAAGGTGCTGAGGTACGATGAG, Primer 1 for E1B55K
- SEQ ID NO: 12: GGTCACATCCAGCATCACAGG, Primer 2 for E1B55K
- SEQ ID NO: 13: TGACAGCTTTGTGAACTGGGTG, Primer 1 for Rep78/68
- SEQ ID NO: 14: CGTGCATGTGGAAGTAGCTCTC, Primer 2 for Rep78/68
- SEQ ID NO: 15: TTAGTAAGCTTCCCTCCTGACGCGGTAGGAGGAGGGGAGGGTGCCCTGCATG, Primer 1 for VA fragment
- SEQ ID NO: 16: CCTTTTGCTCACATGTCAATTGGTAGATGTACCTGGACATCCAGGTGATGCCGGC G, Primer 2 for VA fragment
- SEQ ID NO: 17: TGTACAGGCGCGCCGAATTCGTTTTAGGGCGGAGTAACTTGTATGTGTTGGGAAT TGTAG, Primer 1 for E4 fragment
- SEQ ID NO: 18: AGGGAAGCTTACTAAACGGTACACAGGAAACAGGAGACACAACTCCAAGTG, Primer 2 for E4 fragment
- SEQ ID NO: 19: GAAAAGTGCCACCTGACGTCGCGGCCGCGCGATCGCGGTACCCAACTCCATGCT CAACAGTCCCCAGGTACAGCCC, Primer 1 for E2A fragment
- SEQ ID NO: 20: GAATTCGGCGCGCCTGTACAGCCCGGGCGACCGCACCCTGTGACGAAAGCCGC CCGCAAG, Primer 2 for E2A fragment
- SEQ ID NO: 21: 
gAAAAGTGCCACCTGACGTCGCGGCCGCGGAGGGGTGGAGTCGTGACGTGAAT

TACGTCATAGGGTTAGGGAGGTCCTGTATTAGAGGTCACGTGAGTGTTTTGCGAC
 , Primer 1 for p5 gene fragment
- SEQ ID NO: 22: GTTCAAACCTCCCGCTTCAAAATGGAGACCCTGCGTGCTCACTCGGGCTTAAAT ACCCAGCGTGACCACATGGTGTCGCAAAATGTCGCAAAACACTCA, Primer 2 for p5 gene fragment
- SEQ ID NO: 23: GCGGGAGGTTTGAACGCGCAGCCGCCATGCCGGGGTTTTAC, Primer 1 for AAV2 Rep fragment
- SEQ ID NO: 24: GATCGCAGCGCTATTTAAATCATTTATTGTTCAAAGAT, Primer 2 for AAV2 Rep fragment
- SEQ ID NO: 25: AATAGCGCTGCGATCGCGGCCTCCAAAAAAGC, Primer 1 for SV40 ori fragment
- SEQ ID NO: 26: CCTTTTGCTCACATGTCAATTGATCCCGCCCCTAACT, Primer 2 for SV40 ori fragment
- SEQ ID NO: 27: AACAATAAATGATTTAAATCAGG, Primer 1 for AAV1 Cap fragment
- SEQ ID NO: 28: GGCCGCGATCGCAGCGCTGTAGCCATGGAAACTAGATA, Primer 2 for AAV1 Cap fragment
- SEQ ID NO: 29: GTGCCACCTGACGTCGGCCGGCCGCGGCCGCGGTACCGAGCTCGACTTTCAC, Primer 1 for TRE fragment
- SEQ ID NO: 30: TGGTCCTCCTGGATCCGCTAGCCAATTCGGGGCCGCGGAGGC, Primer 2 for TRE fragment
- SEQ ID NO: 31: GGGGCTAGCATGCCGGGGTTTTACGAGAT, Primer 1 for AAV1 Cap fragment
- SEQ ID NO: 32: CCCGGATCCACTGCTTCTCCGAGGTAATC, Primer 2 for AAV1 Cap fragment
- SEQ ID NO: 33: ATCAAGTGTATCATATGCCAAG, Primer 1 for CMV promoter fragment
- SEQ ID NO: 34: TCTAGACATGGTGGCATCTGACGGTTCACT, Primer 2 for CMV promoter fragment
- SEQ ID NO: 35: GATGCCACCATGTCTAGA, Primer 1 for tTA fragment
- SEQ ID NO: 36: GCGGGAATTCCCGATCTACCCACCGTACTC, Primer 2 for tTA fragment
- SEQ ID NO: 37: AGATCGGGAATTCCCGCG, Primer 1 for mPGK promoter fragment
- SEQ ID NO: 38: CTTTTTCATGGTGGCCACGTGAGGTC, Primer 2 for mPGK promoter fragment
- SEQ ID NO: 39: GCCACCATGAAAAAGCCTGAAC, Primer 1 for hygromycin resistant gene fragment
- SEQ ID NO: 40: TCATTGGTCTTAAAGGTACCCTATTCCTTTGCCCT, Primer 2 for hygromycin resistant gene fragment
- SEQ ID NO: 41: CGACGGTATCGCTAGCTTTTAAAAGAAAAGG, Primer 1 for cPPT/CTS fragment
- SEQ ID NO: 42: TACAAAGTTGGGATCCATCCGATAATCACTAGTA, Primer 2 for cPPT/CTS fragment
- SEQ ID NO: 43: TGGATCCCAACTTTGTATAGAAAAG, Primer 1 for TRE fragment
- SEQ ID NO: 44: CTACCCGGTAGAATTCCTCGAGAGCCTGCTTTTTTGTA, Primer 2 for TRE fragment
- SEQ ID NO: 45: ATTATCGGATGGATCCTAGTGTGGCGGAAGTGTGATGTTGCAAGTGTGGCGGAA C, Primer 1 for E1A/E1B fragment
- SEQ ID NO: 46: CTACCCGGTAGAATTCTCAATCTGTATCTTCATCGCTAGAGCCAAACTCAGCGCG, Primer 2 for E1A/E1B fragment
- SEQ ID NO: 47: AAAGCAGGCTCTCGAGATGGAGGCTTGGGAGTGTTTGGAAGATTTTTCTGCTGT GC, Primer 1 for E1B fragment/
   Primer 1 for E1B19K fragment
- SEQ ID NO: 48: CTACCCGGTAGAATTCTCAATCTGTATCTTCATCGCTAGAGCCAAACTCAGCGCG G, Primer 2 for E1B fragment
- SEQ ID NO: 49: CCTACCCGGTAGAATTCTCATTCCCGAGGGTCCAGGCCGGCTCTCGGGTTCCATG G, Primer 2 for E1B 19K fragment
- SEQ ID NO: 50: GCCTTTCGACCTCACGTGGCCACCATGGCCAAGCCTTTG, Primer 1 for Bsd fragment
- SEQ ID NO: 51: GGCTAAGATCTACAGCTGTTAGCCCTCCCACACATAAC, Primer 2 for Bsd fragment
- SEQ ID NO: 52: AAAGCAGGCTCTCGAGATGAGACATATTATCTGCCACGGAGGTGTTATTACCGAA G, Primer 1 for E1A fragment
- SEQ ID NO: 53: CTACCCGGTAGAATTCTTATGGCCTGGGGCGTTTACAGCTCAAGTCCAAAGGTTG C, Primer 2 for E1A fragment
- SEQ ID NO: 54: AAAGCAGGCTCTCGAGATGGAGCGAAGAAACCCATCTGAGCGGGGGGTACCTG CTG, Primer 1 for E1B55K fragment
- SEQ ID NO: 55: CTACCCGGTAGAATTCGTCGACTCAATCTGTATCTTCATCGCTAGAGCCAAAC, Primer 2 for E1B55K fragment
- SEQ ID NO: 56: TACAGATTGAGTCGACATGAGACATATTATCTGCCACGGAGGTGTTATTAC, Primer 1 for E1A fragment
- SEQ ID NO: 57: GCGATGAAGATACAGATTGAGCCCCTCTCCCTCCCCCCCC, Primer 1 for IRES fragment
- SEQ ID NO: 58: TGGCAGATAATATGTCTCATGGTTGTGGCCATATTATCAT, Primer 2 for IRES fragment
- SEQ ID NO: 59: TCACCATCTTGTCGACACAGTCGTTGAAGGG, Primer 1 for p5/AAV2 Rep fragment
- SEQ ID NO: 60: AGCGCAGCGAGTCGGCCGGCCGCGGCCGCGGAGGGGTGGAGTCG, Primer 2 for p5/AAV2 Rep fragment
- SEQ ID NO: 61: GCGATGAAGATACAGATTGAGGTACTGAAATGTGTGGGCG, Primer 1 for PIX fragment
- SEQ ID NO: 62: CTCCCCTACCCGGTAGAATTCAGACAGCAAGACACTTGCTTGATCCAAATCCAA ACAGAGTC, Primer 2 for PIX fragment
- SEQ ID NO: 63: CTTGCTGTCTGAATTCTAGGTTCTACCGGGTAGGGGAGGCGC, Primer 1 for mPGK promoter/hygromycin resistant gene/hGH polA signal fragment
- SEQ ID NO: 64: GCGGCCGCAGATCTACGCGTGTCGACAAGGACAGGGAAGGGAGCAG, Primer 2 for mPGK promoter/hygromycin resistant gene/hGH polA signal fragment
- SEQ ID NO: 65: TAGATCTGCGGCCGCGAGCAAAAGGCCAGCAAAAG, Primer 1 for ampicillin resistant gene/pUC ori fragment
- SEQ ID NO: 66: CGCCACACTAGGATCCAAGCTTGCGGCCGCAGGTGGCACTTTTCGGGGA, Primer 2 for ampicillin resistance gene/pUC ori fragment
- SEQ ID NO: 67: CGAAAAGTGCCACCTGCGGCCGCAAGCTTTCTAGGCGTTACATAACTTACGGTA A, Primer 1 for CMV promoter fragment
- SEQ ID NO: 68: GAATTCCCTGCAGGGCTCTTCAGCTCTGCTTATATAGACCTC, Primer 2 for CMV promoter fragment
- SEQ ID NO: 69: CCCTGCAGGGAATTCTAAGATACATTGATGAG, Primer 1 for SV40 polyA signal fragment
- SEQ ID NO: 70: GGAGGATCCAACTTGTTTATTGCAGCTTATA, Primer 2 for SV40 polyA signal fragment
- SEQ ID NO: 71: CAAGTTGGATCCTCCCCAGCATGCCTGCTATTC, Primer 1 for bGH polyA signal fragment
- SEQ ID NO: 72: TTGGCTAGCTGTACACTGTGCCTTCTAGTTGCC, Primer 2 for bGH polyA signal fragment
- SEQ ID NO: 73: CAAGTTGGATCCTCCCCAGCATGCCTGCTATTC, Primer 1 for CAG promoter fragment
- SEQ ID NO: 74: GCTGGCCTTTTGCTCGCGGCCGCAGATCTACGCGTCCTAGGCTCGACATTGATTA TTGAC, Primer 2 for CAG promoter fragment
- SEQ ID NO: 75: GCCCTGCAGGGAATTCATGAGACATATTATC, Primer 1 for E1A fragment
- SEQ ID NO: 76: ATGTATCTTAGAATTTTATGGCCTGGGGCGTTTAC, Primer 2 for E1A fragment
- SEQ ID NO: 77: CAAAGAATTGGCTAGCATGGAGGCTTGGGAGTGTTTG, Primer 1 for E1B fragment
- SEQ ID NO: 78: ACAGTGTACAGCTAGTCAATCTGTATCTTCATCGC, Primer 2 for E1B fragment
- SEQ ID NO: 79: CTTGCTGTCTGAATTCTAGGTTCTACCGGGTAGGG, Primer 1 for mPGK promoter/hygromycin resistant gene/hGH polA signal fragment
- SEQ ID NO: 80: CCGCAGATCTACGCGTGTCGAC, Primer 2 for mPGK promoter/hygromycin resistant gene/hGH polA signal fragment
- SEQ ID NO: 81: TCCCAAGCCTCCATGCTAGCCAATTCGGGGCCGCGGAGGC, Primer 1 for TRE fragment
- SEQ ID NO: 82: CCCTACCCGGTAGAACCTAGGCAACTTTGTATAGAAAAGTTG, Primer 2 for TRE fragment
- SEQ ID NO: 83: CTGTCCTTGTCGACACGCGTGAGCAAAAGGCCAGCAAAAG, Primer 1 for TRE fragment
- SEQ ID NO: 84: GATAATATGTCTCATGAATTCAGCCTGCTTTTTTGTACAAAC, Primer 2 for TRE fragment
- SEQ ID NO: 85: CTTGCTGTCTGAATTCTAGGTTCTACCGGGTAGGGG, Primer 1 for mPGK promoter/Bsd/hGH polyA signal fragment
- SEQ ID NO: 86: CGCGGCCGCAGATCTACGCGTGTCGAC, Primer 2 for mPGK promoter/Bsd/hGH polyA signal fragment
- SEQ ID NO: 87: CCGCAAGCTTGGATCCCAACTTTGTATAGAAAAGTTG, Primer 1 for TRE/E1A fragment
- SEQ ID NO: 88: TATTGCATTCTCTAGACACAGG, Primer 2 for TRE/E1A fragment
- SEQ ID NO: 89: ATTGCGTGTGTGGTTAACGCC, Primer 1 for E1A PolyA signal fragment
- SEQ ID NO: 90: TTCTATACAAAGTTGGAGGTCAGATGTAA, Primer 2 for E1A PolyA signal fragment
- SEQ ID NO: 91: CAACTTTGTATAGAAAAGTTG, Primer 1 for TRE/E1B fragment
- SEQ ID NO: 92: TCCATTTATCCTTTATAAAACTC, Primer 2 for TRE/E1B fragment

SEQ ID NO: 93: tctgaagactacagcgtcgc, Primer 1 for E1B modification confirmation
SEQ ID NO: 94: ggtgtacaggatggaacatgaaggc, Primer 2 for E1B modification confirmation
SEQ ID NO: 95: acctgcgcttgtggtatgatg, Primer 1 for E4orf6
SEQ ID NO: 96: gtgcaaggcgctgtatccaaag, Primer 2 for E4orf6
SEQ ID NO: 97: atctccttcaatgcggcctc, Primer 1 for Rep78/68/52/40
SEQ ID NO: 98: agacggaagccgcatattgg, Primer 2 for Rep78/68/52/40
SEQ ID NO: 99: atgagacatattatctgcca, gRNA targeting E1A
SEQ ID NO: 100: gccaaactcagcgcgggtgc, gRNA targeting E1B
SEQ ID NO: 101: ttcaggttggaccggtgccaccatgccgaagaaaaagcgcaaggtcgaagcgtccgacaaga, Primer 1 for nCas9 (D10A)
SEQ ID NO: 102: cccacagagttggtgccgatggccaggccgatgctgtacttcttgtcggacgcttcgacc, Primer 2 for nCas9 (D10A)
SEQ ID NO: 103: gccatcggcaccaactctgtggg, Primer 1 for Cas9
SEQ ID NO: 104: tcgttgctgaagatctcttgcag, Primer 2 for Cas9
SEQ ID NO: 105: aggaacatccccttccctcggatg, Primer 1 for 5' homology region amplification
SEQ ID NO: 106: aaaggtgaggcggctccggagaa, Primer 2 for 5' homology region amplification
SEQ ID NO: 107: cggcgcgccgcggccgcaggaacatccccttccctcggatg, Primer 1 for 5' homology region fragment
SEQ ID NO: 108: cgccacactaggatcaagtcatcaacttgttatcctggtttacag, Primer 2 for 5' homology region fragment
SEQ ID NO: 109: gacttgatcctagtgtggcggaagtgtgatg, Primer 1 for E1A promoter fragment
SEQ ID NO: 110: tcacgccgccgtgacagatgatgtgccgcattttcagtcccggtgtcggag, Primer 2 for E1A promoter fragment
SEQ ID NO: 111: tcatctgtcacggcggcgtgatcacagaggaaatggccgccagtcttttggaccagctg, Primer 1 for nCas9-unrecognized E1A fragment
SEQ ID NO: 112: gcaaacaaaggcgttaaccacacacgcaatcacaggtttacacc, Primer 2 for nCas9-unrecognized E1A fragment
SEQ ID NO: 113: attgcgtgtgtggttaacgcctttgtttgctgaatgagttgatgtaagtttaataaacaactttgtatagaaaagttgggtaccgagc, Primer 1 for TRE fragment
SEQ ID NO: 114: tcccaagcctccatgctagccaattcgggg, Primer 2 for TRE fragment
SEQ ID NO: 115: ccttgggtatcctgtctgagggtaactcca, Primer 1 for nCas9-unrecognized E1B fragment
SEQ ID NO: 116: gtcggagctgccaaactcggctctggtacaggccagcaccaagtgatcgggcc, Primer 2 for nCas9-unrecognized E1B fragment
SEQ ID NO: 117: gagtttggcagctccgacgaggacaccgattgaggtactgaaatgtgtgggcg, Primer 1 for E1B 3'UTR fragment
SEQ ID NO: 118: gccggttggcgcctaccggtggatgtggaatgtgt, Primer 2 for E1B 3'UTR fragment
SEQ ID NO: 119: gataaatatgtggccggggg, Primer 1 for 3' homology region amplification
SEQ ID NO: 120: tgaccactgaagcacagcatcacag, Primer 2 for 3' homology region amplification
SEQ ID NO: 121: ccctgtccttgtcgaccaaagatgtcagaacaagactcccc, Primer 1 for 3' homology region fragment
SEQ ID NO: 122: ccttttgctcacgcgttgaccactgaagcacagcatcacag, Primer 2 for 3' homology region fragment
SEQ ID NO: 123: tcagcagcagccgcgggcccttg, Primer 1 for E2A fragment
SEQ ID NO: 124: gcccgggcgaccgcaccctg, Primer 2 for E2A fragment
SEQ ID NO: 125: agggtgcggtcgcccgggcgttttagggcggagtaacttgtatgtgttgg, Primer 1 for E4orf6 fragment
SEQ ID NO: 126: ggttaactccccagcatgcctgctattctc, Primer 2 for E4orf6 fragment
SEQ ID NO: 127: gcaggcatgctggggagttaacccctcctgacgcggtaggaggag, Primer 1 for VA fragment
SEQ ID NO: 128: tcgttgacgctctagaccgtgcaaaaggagagcctg, Primer 2 for VA fragment
SEQ ID NO: 129: agggtgcggtcgcccgggccaactttgtatagaaaagttgggtaccgag, Primer 1 for TRE fragment
SEQ ID NO: 130: ggaacgccggacgtagtcatcaattcggggccgcggaggc, Primer 2 for TRE fragment
SEQ ID NO: 131: atgactacgtccggcgttccatttgg, Primer for E4orf6-polyA-VA fragment

## Claims

1. A method for controlling expression of a Rep gene, comprising a step of controlling expression of a helper gene selected from E1B, E2, E4 and VA.

2. A method for controlling expression of a Rep gene, comprising a step of controlling expression of an E1B helper gene.

3. A method for controlling expression of a Rep gene, comprising a step of controlling expression of two or more helper genes related to production of an adeno-associated virus (AAV) -derived construct.

4. The method according to claim 3, wherein the helper gene includes a helper gene selected from E1A, E1B, E2, E4 and VA.

5. The method according to claim 3, wherein the helper gene includes a helper gene selected from E1A and E1B and a helper gene selected from E2, E4 and VA.

6. The method according to any one of claims 1 to 3, wherein a decrease in expression of the helper gene results in a decrease in expression of the Rep gene.

7. The method according to any one of claims 1 to 3, wherein the step of controlling expression of the helper gene uses an expression control system having at least one of the following functions of controlling activity of a promoter operably linked to the helper gene, degrading nucleic acids transcribed from the helper gene, making the helper gene transcriptionally inactive, and degrading a protein encoded by the helper gene.

8. The method according to claim 7, wherein the expression control system is activated by addition or removal of a drug.

9. The method according to any one of claims 1 to 3, wherein the expression control system includes a stimuli-responsive promoter operably linked to the helper gene, a Cre protein-responsive nucleic acid region that enables removal or inversion of at least a part of a nucleic acid region of the helper gene or a promoter operably linked thereto, a CRISPR-Cas9 system, or an shRNA for a nucleic acid transcribed from the helper gene.

10. The method according to any one of claims 1 to 3, further comprising a step of triggering an expression control system for the Rep other than the helper gene.

11. The method according to any one of claims 1 to 3, wherein expression of the Rep gene is controlled in a cell containing the helper gene and the Rep gene.

12. The method according to any one of claim 1 to 3, wherein expression of the Rep gene is controlled in a producer cell containing the helper gene and the Rep gene that produces an AAV-derived construct.

13. The method according to claim 12, wherein production of the AAV-derived construct is controlled by controlling expression of the helper gene.

14. The method according to claim 12, wherein the producer cell contains an inverted terminal repeat (ITR), a gene of interest (GOI), and a Cap gene.

15. The method according to claim 14, wherein the producer cell contains the helper gene, ITR, GOI, Cap gene, and Rep gene in a chromosome.

16. The method according to claim 14, wherein the producer cell contains the helper gene, ITR, GOI, Cap gene, and Rep gene at one locus on a chromosome.

17. A combination for controlling expression of a Rep gene, comprising: a nucleic acid containing a Rep gene;
a nucleic acid containing a helper gene selected from E1B, E2, E4 and VA; and
a control system for the helper gene.

18. A combination for controlling expression of a Rep gene, comprising: a nucleic acid containing a Rep gene;
a nucleic acid containing two or more helper genes required for production of an adeno-associated virus (AAV) -derived construct; and
a control system for the helper gene.

19. The combination according to claim 18, wherein the helper gene includes a helper gene selected from E1A, E1B, E2, E4 and VA.

20. The combination according to claim 18, wherein the helper gene includes a helper gene selected from E1A and E1B and a helper gene selected from E2, E4 and VA.

21. The combination according to claim 17 or 18, wherein the nucleic acid containing the Rep gene and the nucleic acid containing the helper gene form separate molecules or separate double-stranded complexes.

22. The combination according to claim 17 or 18, wherein the nucleic acid containing the Rep gene and the nucleic acid containing the helper gene form the same molecule or the same double-stranded complex.

23. The combination according to claim 17 or 18, wherein the control system includes a combination of a drug and the drug-responsive promoter operably linked to the helper gene, a combination of a Cre protein and the Cre protein-responsive nucleic acid region that enables removal of a poly A region linked to the helper gene, or an shRNA for a nucleic acid transcribed from the helper gene.

24. The combination according to claim 17 or 18, wherein the nucleic acid containing the Rep gene and/or the nucleic acid containing the helper gene is a plasmid.

25. The combination according to claim 17 or 18, wherein the nucleic acid containing the Rep gene and/or the nucleic acid containing the helper gene forms a complex with a cationic substance.

26. The combination according to claim 17 or 18, comprising a cell that contains a nucleic acid containing the Rep gene and a nucleic acid containing the helper gene.

27. The combination according to claim 26, wherein the cell is a producer cell that produces an AAV-derived construct.

28. The combination according to claim 27, wherein the producer cell contains an inverted terminal repeat (ITR), a gene of interest (GOI), and a Cap gene.

29. The combination according to claim 28, wherein the producer cell contains the helper gene, ITR, GOI, Cap gene, and Rep gene in a chromosome.

30. The combination according to claim 28, wherein the producer cell contains the helper gene, ITR, GOI, Cap gene, and Rep gene at one locus on a chromosome.

31. A method of producing an adeno-associated virus (AAV) -derived construct using the combination according to claim 17 or 18, the method comprising:
a step of preparing a producer cell that contains a nucleic acid containing the Rep gene and a nucleic acid containing the helper gene; and
a step of controlling expression of the Rep gene using a control system for the helper gene.

32. The method according to claim 31, wherein the producer cell contains the helper gene and the Rep gene in a chromosome.

33. An AAV-derived construct produced by the method according to claim
